# EUROPEAN PATENT APPLICATION

(11) **EP 2 573 107 A1**
(43) Date of publication of application: **27.03.2013**
(21) Application number: 11182235.9
(22) Date of filing: 21.09.2011
(51) Int. Cl.: C07K 14/47, C12N 15/62

(54) **A recombinant fusion protein**

(71) Applicant: Norwegian Institute of Public Health, 0403 Oslo (NO)
(72) Inventor: Holm-Hansen, Carol Joanne Church, 0403 Oslo (NO); Ihle, Øistein, 0403 Oslo (NO); Michaelsen, Terje Einar, 0403 Oslo (NO)
(74) Representative: Arends, William Gerrit

(57) **Abstract**

A recombinant fusion protein comprising at least one antigenic region comprising an amino acid sequence with at least 80% sequence identity to any of SEQ. ID NOS. 1 to 3, 8, 4, 7, 6 or 5, or an antigenic fragment thereof. The recombinant fusion protein also comprises an anchoring region comprising a sequence of at least 25 amino acids.

## Description

### Field of the Invention

The present invention relates to a recombinant fusion protein and also to a nucleic acid molecule encoding the fusion protein and to a vector comprising the nucleic acid molecule. The present invention also relates to a kit for the detection of active tuberculosis in a patient. The invention, further, relates to a method of detecting active tuberculosis in a patient and to the use of a peptide as a biological marker for the presence of active tuberculosis in a patient.

### Background of the Invention

Tuberculosis (hereinafter "TB") is a serious infectious disease caused by certain strains of mycobacteria, principally *Mycobacterium tuberculosis.* It is estimated that one third of the world's population is infected with *M. tuberculosis.* However, about 90% of individuals infected with *M. tuberculosis* have a so-called "latent" infection (referred to as "Class 2" TB under the clinical classification system for TB) in which the bacteria lie inactive within macrophages in the individual. No symptoms of the infection are presented in an individual with a latent TB infection although a positive reaction is observed if a tuberculin skin test is carried out. Moreover, an individual with a latent TB infection cannot transmit TB to others. Nevertheless, 10% of individuals infected with *M. tuberculosis* develop "active" TB within their lifetime which involves symptoms such as cough, chest pains and hemoptysis. Active TB (referred to "Class 3" TB under the clinical classification system) will typically result in the death of the patient in around 50% of cases, if untreated, and it is individuals with active TB who are able to transmit the infection to others.

The prevalence of TB varies significantly from country to country. Broadly speaking, TB infection rates in developed countries are relatively low (e.g. in the United Kingdom, the national average was 15 cases per 100,000 in 2007) whereas rates of infection in developing countries are much higher (e.g. 1200 cases per 100,000 people in Swaziland in 2007). Detection and treatment of TB is essential to control the pandemic. Individuals with the active, untreated TB disease can infect 10-15 other people per year, according to some estimates. One of the difficulties in control of TB infection in developing countries is that there is often not ready access to a healthcare infrastructure which can reliably diagnose active TB infection in individuals quickly enough to prevent infected individuals from spreading TB more widely.

There is a range of different tests that can be carried out to detect active TB in a patient. A common test is a microbiological study in which a biological sample (e.g. sputum or pus) is obtained from a patient and investigated for evidence of *M. tuberculosis.* Typically, a sputum specimen is stained and examined microscopically for the presence of bacteria and thereafter subjected to culture. Sputum smear microscopy is not sensitive and culture, although reliable, takes six to eight weeks to complete and requires laboratory facilities that are not available in remote parts of developing countries.

Another common technique for the detection of TB is a tuberculin skin test. In brief, this test involves injecting tuberculin (a glycerol extract of *M. tuberculosis*) intradermally and then observing the patient 48 to 72 hours later. An individual who has been exposed to *M. tuberculosis* should display an immune response in the skin containing the tuberculin. The problem with the tuberculin skin test is that an individual with latent TB will also display a positive reaction to the skin test so the test does not enable individuals infected with active TB to be distinguished from those with a latent TB infection. Since a large proportion of individuals will test positive to this test, it is not feasible to isolate individuals on the basis of a positive result from the test.

There are also various other tests for TB infection which are known in the art. However, these other tests are generally characterised by yielding many false-negative results and not complying with WHO requirements. WHO has undertaken a laboratory-based evaluation of 19 commercially available rapid diagnostic tests for TB infection. The findings were published as part of a "Diagnostic Evaluation Series" in 2008. The performance, reproducibility and operational characteristics of the 19 commercially available tests was evaluated using 355 well-characterised archived serum samples from eight geographically diverse collection sites. Sera from TB-negative patients were used as a control population. A number of the tests analysed had a high specificity however, all of the tests with a high specificity (>95%) also had very low sensitivity (0.97-21%). In addition test performance was poor in patients with sputum smear-negative TB. Importantly, none of the tests analysed performed well enough to replace microscopy. Combining smear microscopy with the most rapid tests improved overall diagnostic sensitivity but yielded an unacceptable overall false positive rate of 42%. WHO concluded, based on this evaluation, that a simple to use, accurate, inexpensive and point of case diagnostic test for active TB is urgently needed.

Accordingly, the present invention seeks to alleviate one or more of the above problems. More specifically, the present invention seeks, in certain embodiments, to provide products and methods for the detection of active TB in individuals. The products and methods of the present invention arise from the surprising finding that individuals with active TB have a different repertoire of antibodies from individuals with latent TB. Individuals with active TB have antibodies specific for certain antigens of *M. tuberculosis* against which individuals with latent TB do not have antibodies. The products and methods of the invention are not intended to replace the microbiological tests for active TB described above. Instead, the products and methods of the present invention enable a means of rapidly detecting active TB in patients (or at least indicating a high likelihood of active TB in patients) using a technique which can distinguish between cases of active TB and latent TB and which, moreover, has a low rate of false-negative results. Since it is only individuals with active TB who are able to transmit the infection, the products and methods of the present invention enable infectious individuals to be identified and referred for confirmatory testing and treatment, thereby preventing the spread of TB infection within a population.

### Summary of the Invention

According to a first aspect of the present invention, there is provided a recombinant fusion protein comprising:
(a) at least one antigenic region comprising an amino acid sequence with at least 80% sequence identity to any of SEQ. ID NOS. 1 to 8 or an antigenic fragment thereof; and
(b) at least one anchoring region comprising a sequence of at least 25 amino acids.

Conveniently, the at least one antigenic region comprises a plurality of different antigenic regions having a different sequence selected from the following (a) to (h):
(a) a sequence with at least 80% sequence identity to SEQ. ID NO. 1 or an antigenic fragment thereof;
(b) a sequence with at least 80% sequence identity to SEQ. ID NO. 2 or an antigenic fragment thereof;
(c) a sequence with at least 80% sequence identity to SEQ. ID NO. 3 or an antigenic fragment thereof;
(d) a sequence with at least 80% sequence identity to SEQ. ID NO. 4 or an antigenic fragment thereof;
(e) a sequence with at least 80% sequence identity to SEQ. ID NO. 5 or an antigenic fragment thereof;
(f) a sequence with at least 80% sequence identity to SEQ. ID NO. 6 or an antigenic fragment thereof;
(g) a sequence with at least 80% sequence identity to SEQ. ID NO. 7 or an antigenic fragment thereof; and
(h) a sequence with at least 80% sequence identity to SEQ. ID NO. 8 or an antigenic fragment thereof.

Preferably, at least one antigenic region is N-terminal to the at least one anchoring region.

Advantageously, at least one antigenic region is C-terminal to the at least one anchoring region.

More preferably, the at least one antigenic region having a sequence selected from (a), (b), (d) and (g) is C-terminal to the at least one anchoring region. Preferably, the at least one antigenic region having a sequence selected from (a), (b), (c), (e), (f) and (h) is N-terminal to the at least one anchoring region.

Preferably, the fusion protein comprises a plurality of anchoring regions.

Conveniently, the at least one anchoring region comprises at least constant domains 2 and 3 and a hinge region of an immunoglobulin Fc region, preferably a murine IgG2a Fc region or a corresponding rabbit, rat or sheep Fc region. Preferably, the at least one anchoring region comprises an entire IgG molecule, more preferably a murine IgG2a antibody. Alternatively, the at least one anchoring region comprises a histidine (His) tag or a Glutathione S-transferase (GST) tag. Conveniently, the at least one anchoring region comprises a plurality of anchoring regions.

Advantageously, the fusion protein is glycosylated. Suitably, the fusion protein has a prokaryotic glycosylation pattern. Alternatively, the fusion protein has a eukaryotic glycosylation pattern.

Preferably, the glycosylation is selected from the group comprising N-linked glycosylation, O-linked glycosylation, P-linked glycosylation and C-linked glycosylation.

According to a second aspect of the present invention, there is provided a nucleic acid molecule comprising a nucleotide sequence encoding a recombinant fusion protein according to the first aspect of the invention or the antigenic region according to the tenth aspect of the invention.

According to a third aspect of the present invention, there is provided a vector comprising a nucleic acid molecule according to the second aspect of the invention or the antigenic region according to the tenth aspect of the invention.

According to a fourth aspect of the present invention, there is provided a host cell comprising a nucleic acid molecule according to the second aspect of the invention or a vector according to the third aspect of the invention and capable of expressing a recombinant fusion protein according to the first aspect of the invention or the antigenic region according to the tenth aspect of the invention.

According to a fifth aspect of the present invention, there is provided a kit for the detection of active TB in a patient comprising at least two different peptides selected from the following (a) to (h):
(a) a sequence with at least 80% sequence identity to SEQ. ID NO. 1 or an antigenic fragment thereof;
(b) a sequence with at least 80% sequence identity to SEQ. ID NO. 2 or an antigenic fragment thereof;
(c) a sequence with at least 80% sequence identity to SEQ. ID NO. 3 or an antigenic fragment thereof;
(d) a sequence with at least 80% sequence identity to SEQ. ID NO. 4 or an antigenic fragment thereof;
(e) a sequence with at least 80% sequence identity to SEQ. ID NO. 5 or an antigenic fragment thereof;
(f) a sequence with at least 80% sequence identity to SEQ. ID NO. 6 or an antigenic fragment thereof;
(g) a sequence with at least 80% sequence identity to SEQ. ID NO. 7 or an antigenic fragment thereof; and
(h) a sequence with at least 80% sequence identity to SEQ. ID NO. 8 or an antigenic fragment thereof.

Conveniently, the kit comprises at least two recombinant fusion proteins according to the first aspect of the invention, wherein one of the at least two different peptides is the antigenic region in one of the at least two recombinant fusion proteins and the other of the at least two different peptides is the antigenic region in the other of the at least two recombinant fusion proteins.

According to a sixth aspect of the present invention, there is provided a kit for the detection of active TB in a patient comprising:
(a) a solid phase; and
(b) at least one peptide bound to the solid phase wherein the peptide comprises an amino acid sequence with at least 80% sequence identity to a sequence selected from any of SEQ. ID NOS. 1 to 8 or an antigenic fragment thereof.

Preferably, the solid phase comprises a substrate. Alternatively, the solid phase comprises a particle.

Advantageously, the kit comprises a plurality of particles each comprising at least one of the peptides bound thereto.

Preferably, the or each particle further comprises a detectable label.

Conveniently, the at least one peptide is a plurality of sets of different peptides and the peptide or peptides in each set comprises a different amino acid sequence selected from the following (a) to (h):
(a) a sequence with at least 80% sequence identity to SEQ. ID NO. 1 or an antigenic fragment thereof;
(b) a sequence with at least 80% sequence identity to SEQ. ID NO. 2 or an antigenic fragment thereof;
(c) a sequence with at least 80% sequence identity to SEQ. ID NO. 3 or an antigenic fragment thereof;
(d) a sequence with at least 80% sequence identity to SEQ. ID NO. 4 or an antigenic fragment thereof;
(e) a sequence with at least 80% sequence identity to SEQ. ID NO. 5 or an antigenic fragment thereof;
(f) a sequence with at least 80% sequence identity to SEQ. ID NO. 6 or an antigenic fragment thereof;
(g) a sequence with at least 80% sequence identity to SEQ. ID NO. 7 or an antigenic fragment thereof; and
(h) a sequence with at least 80% sequence identity to SEQ. ID NO. 8 or an antigenic fragment thereof.
wherein each particle has bound to it at least one of the peptides from one of the sets of peptides but does not have bound to it any peptides from any of the other sets of peptides.

Preferably, the solid phase comprises a strip on which a fluid can pass by capillary action.

Advantageously, the kit further comprises a secondary antibody capable of binding human IgG.

Conveniently, the secondary antibody is bound to a detectable label or has a detectable label attachable to it

According to a seventh aspect of the present invention, there is provided a method of detecting active TB in a patient comprising the steps of:
(a) providing at least one peptide comprising an amino acid sequence with at least 80% sequence identity to a sequence selected from SEQ. ID NOS. 1 to 8 or an antigenic fragment thereof;
(b) contacting the at least one peptide with a biological sample obtained from the patient; and
(c) detecting the binding of the at least one peptide with an antibody in the biological sample wherein the presence of an antibody binding to the peptide is indicative of active TB in the patient.

Conveniently, the at least one peptide is a plurality of peptides, each comprising a different sequence selected from the following (a) to (h):
(a) a sequence with at least 80% sequence identity to SEQ. ID NO. 1 or an antigenic fragment thereof;
(b) a sequence with at least 80% sequence identity to SEQ. ID NO. 2 or an antigenic fragment thereof;
(c) a sequence with at least 80% sequence identity to SEQ. ID NO. 3 or an antigenic fragment thereof;
(d) a sequence with at least 80% sequence identity to SEQ. ID NO. 4 or an antigenic fragment thereof;
(e) a sequence with at least 80% sequence identity to SEQ. ID NO. 5 or an antigenic fragment thereof;
(f) a sequence with at least 80% sequence identity to SEQ. ID NO. 6 or an antigenic fragment thereof;
(g) a sequence with at least 80% sequence identity to SEQ. ID NO. 7 or an antigenic fragment thereof; and
(h) a sequence with at least 80% sequence identity to SEQ. ID NO. 8 or an antigenic fragment thereof.
and wherein, in step (c), the presence of at least one antibody binding to at least one peptide is indicative of active TB in the patient.

Preferably, the at least one peptide is part of a fusion protein in accordance with the first aspect of the invention.

According to an eighth aspect of the present invention, there is provided the use of a peptide as a biological marker for the presence of active TB in a patient, when the peptide comprises an amino acid sequence with at least 80% sequence identity to a sequence selected from any of SEQ. ID NOS. 1 to 8 or an antigenic fragment thereof. According to a ninth aspect of the present invention, there is provided a method of treating active TB in a patient comprising detecting active TB in a patient in accordance with the seventh aspect of the present invention and administering to the patient a pharmaceutically effective amount of an active ingredient capable of treating TB. Preferably, the active ingredient comprises an antibiotic.

According to a tenth aspect of the present invention, there is provided an antigenic region comprising an amino acid sequence with at least 80% sequence identity to any of SEQ ID NOS. 1 to 8, or an antigenic fragment thereof, wherein the antigenic region is glycosylated. Suitably, the fusion protein has a prokaryotic glycosylation pattern. Alternatively, the fusion protein has a eukaryotic glycosylation pattern. Preferably, the glycosylation is selected from the group comprising N-linked glycosylation, O-linked glycosylation, P-linked glycosylation and C-linked glycosylation.

The term "active TB" as used herein refers to a tuberculosis infection (whether pulmonary or otherwise) that is clinically active. In particular "active TB" is a tuberculosis infection that is within Class 3 of the World Health Organisation's official classification system.

The term "amino acid" as used herein refers to naturally occurring and synthetic amino acids, as well as amino acid analogues and amino acid mimetics that have a function that is similar to the naturally occurring amino acids. Naturally occurring amino acids are those encoded by the genetic code, as well as those modified after translation in cells (e.g. hydroxyproline, gamma-carboxyglutamate, and O-phosphoserine). The phrase "amino acid analogue" refers to compounds that have the same basic chemical structure (an alpha carbon bound to a hydrogen, a carboxy group, an amino group, and an R group) as a naturally occurring amino acid but have a modified R group or modified backbones (e.g. homoserine, norleucine, methionine sulfoxide, methionine methyl sulphonium). The phrase "amino acid mimetic" refers to chemical compounds that have different structures.

The term "antigenic fragment" as used herein in relation to an amino acid sequence refers to a polypeptide having a contiguous series of amino acid residues present in the amino acid sequence, which polypeptide comprises an epitope that can be bound by an antibody. As such, antigenic fragments may comprise at least 6, 8, 10, 12 or 15 amino acid residues.

The term "nucleotide" as used herein refers to naturally occurring nucleotides and synthetic nucleotide analogues that are recognised by cellular enzymes.

The terms "polynucleotides", and "nucleic acid molecules" are used interchangeably herein to refer to a polymer of multiple nucleotides. The nucleic acid molecules may comprise naturally occurring nucleic acids or may comprise artificial nucleic acids such as peptide nucleic acids, morpholin and locked nucleic acids as well as glycol nucleic acids and threose nucleic acids.

The terms "polypeptide", "peptide" and "protein" are used interchangeably herein to refer to a polymer of amino acid residues. The terms apply to amino acid polymers in which one or more amino acid residues is a modified residue, or a non-naturally occurring residue, such as an artificial chemical mimetic of a corresponding naturally occurring amino acid, as well as to naturally occurring amino acid polymers.

The term "recombinant" as used herein refers to a nucleic acid molecule or a polypeptide which is located in a non-naturally occurring context and which has been produced by artificial intervention. For example, a first polypeptide isolated from other polypeptides or linked by a peptide bond to a second polypeptide sequence having a different amino acid sequence from any polypeptide with which the first polypeptide is associated in nature is a recombinant polypeptide.

The term "glycosylation" as used herein refers to a co-translation or post-translation modification whereby glycans are attached to proteins or other molecules. Proteins produced and secreted by eukaryotic cells have a different pattern of glycosylation than proteins produced and secreted by prokaryotic cells. There are several different types of glycans that can be attached, namely N-linked glycans, O-linked glycans, Phospho-linked glycans or C-linked glycans.

The term "prokaryotic glycosylation pattern" as used herein, refers to the glycans that are attached to a protein when the protein is produced and secreted by a prokaryotic cell.

The term "eukaryotic glycosylation pattern" as used herein, refers to the glycans that are attached to a protein when the protein is produced and secreted by a eukaryotic cell.

In this specification, the percentage "identity" between two sequences is determined using the BLASTP algorithm version 2.2.2 (Altschul, Stephen F., Thomas L. Madden, Alejandro A. Schäffer, Jinghui Zhang, Zheng Zhang, Webb Miller, and David J. Lipman (1997), "Gapped BLAST and PSI-BLAST: a new generation of protein database search programs", Nucleic Acids Res. 25:3389-3402) using default parameters. In particular, the BLAST algorithm can be accessed on the internet using the URL http://www.ncbi.nlm.nih.gov/blast/.

### Brief Description of the Figures

Figure 1 is a schematic diagram of a fusion protein in accordance with one embodiment of the present invention.
Figure 2 is a schematic diagram of a fusion protein in accordance with another embodiment of the present invention.
Figure 3 is a schematic diagram of a fusion protein in accordance with a further embodiment of the present invention.
Figure 4 is a schematic diagram of a fusion protein in accordance with a further embodiment of the present invention.
Figure 5 is a schematic diagram of the components of an ELISA protocol in accordance with one embodiment of the present invention. Figure 5(b) shows a kit in accordance with one embodiment of the invention and Figure 5(a) shows a component thereof. Figures 5(c) to 5(e) show the use of the kit.
Figure 6 is a schematic diagram of a laminar flow immunoassay in accordance with another embodiment of the present invention. Figure 6(a) is a plan of the laminar flow immunoassay of Figure 6(b) is a side elevation thereof.
Figure 7 is a schematic diagram of the components of the labelled particle assay in accordance with a further embodiment of the present invention. Figure 7(a) shows a kit in accordance with this embodiment of the invention. Figures 7(a) shows a kit in accordance with this embodiment of the invention. Figures 7(b) and 7(c) show the components of the embodiment in use.

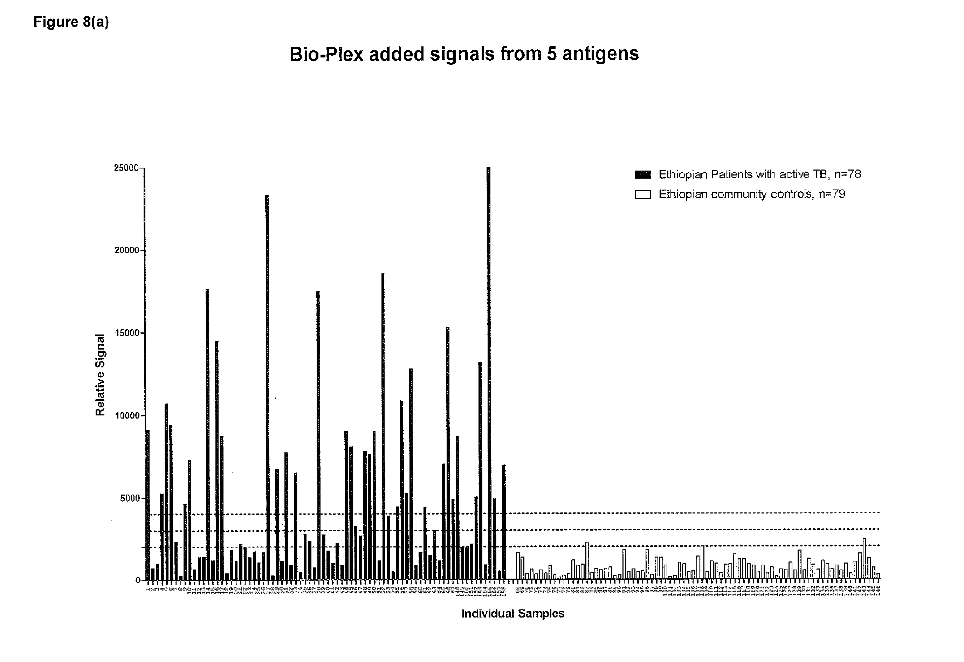

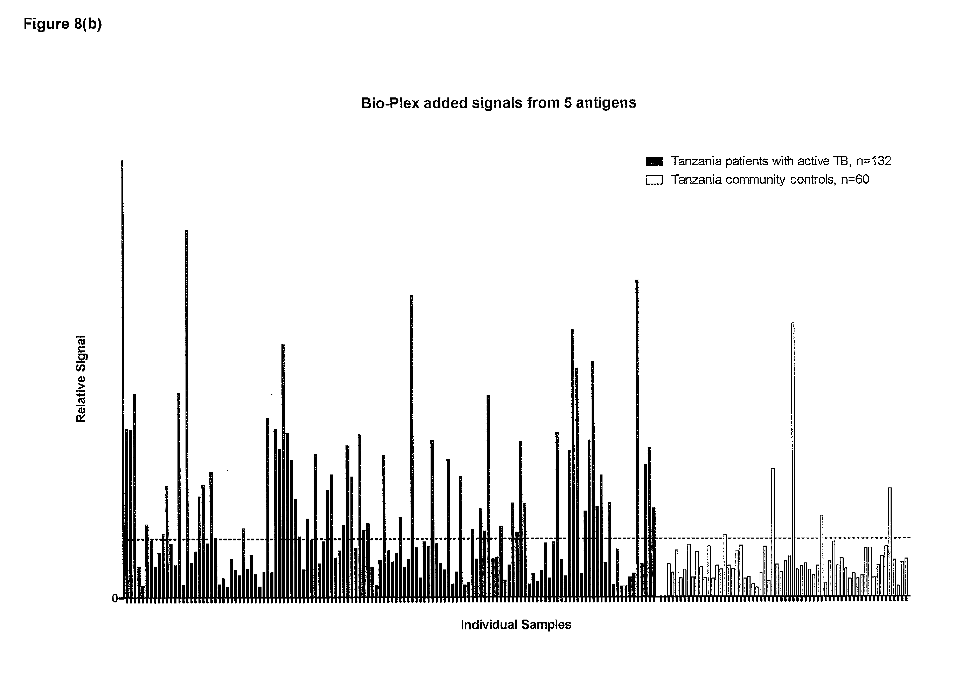

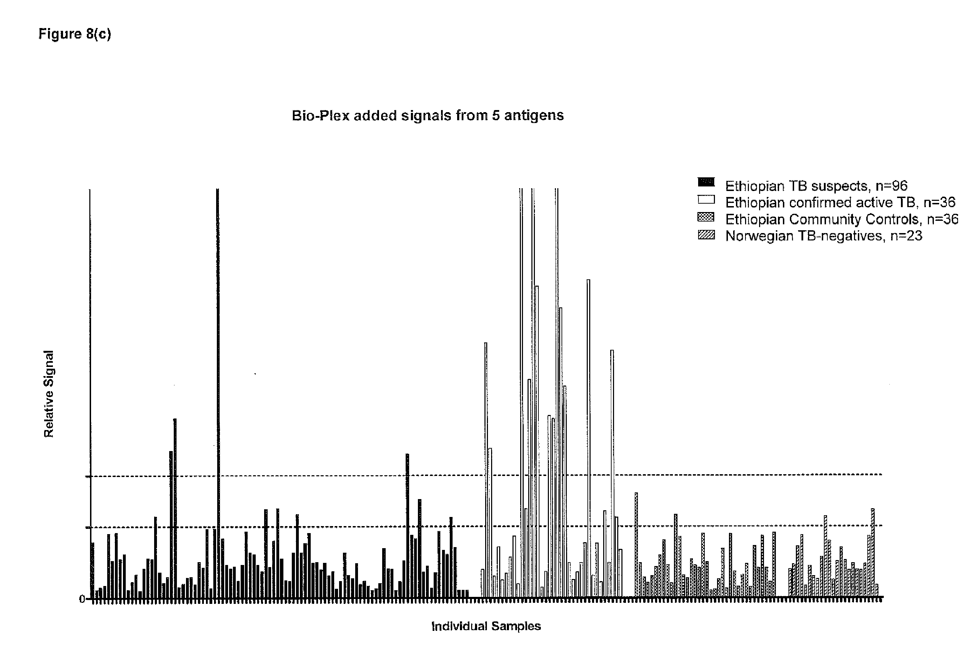

Figure 8 is a graphical representation of the Bio-Plex Assay results. Figure 8(a) illustrates the number of patients testing positive for active TB in a TB-confirmed Ethiopian test group and in a healthy HIV-negative Ethiopian community control group. Figure 8(b) illustrates the number of patients testing positive for active TB in a TB-confirmed Tanzanian test group and in a healthy HIV-negative Tanzanian community control group. Figure 8(c) illustrates the number of individuals testing positive for active TB among Ethiopian patients suspected of having TB (but confirmed non-TB with conventional tests), in a TB-confirmed Ethiopian test group, in a healthy HIV-negative Ethiopian community control group, and among Norwegian TB-negative controls.
Figure 9 is a graphical representation of the Rapid Test TB "prototype" ELISA results illustrating the number of patients testing positive for active TB in a TB-confirmed Ethiopian test group and in a healthy HIV-negative Ethiopian community control group.
Figure 10 is a graphical representation of the Bio-Plex Assay results, illustrating the number of patients testing positive for active TB using Rv0934 fusion protein in a TB-confirmed Ethiopian test group and in a healthy HIV-negative Ethiopian community control group.
Figure 11 is a graphical representation of the Bio-Plex Assay results illustrating the number of patients testing positive for active TB using Rv1759 fusion protein in a TB-confirmed Ethiopian test group and in a healthy HIV-negative Ethiopian community control group.
Figure 12 is a graphical representation of the Bio-Plex Assay results, illustrating the number of patients testing positive for active TB using Rv1886 fusion protein in a TB-confirmed Ethiopian test group and in a healthy HIV-negative Ethiopian community control group.
Figure 13 is a graphical representation of the Bio-Plex Assay results, illustrating the number of patients testing positive for active TB using Rv3874 fusion protein in a TB-confirmed Ethiopian test group and in a healthy HIV-negative Ethiopian community control group.
Figure 14 is a graphical representation of the Bio-Plex Assay results, illustrating the number of patients testing positive for active TB using Rv3881 c fusion protein in a TB-confirmed Ethiopian test group and in a healthy HIV-negative Ethiopian community control group.

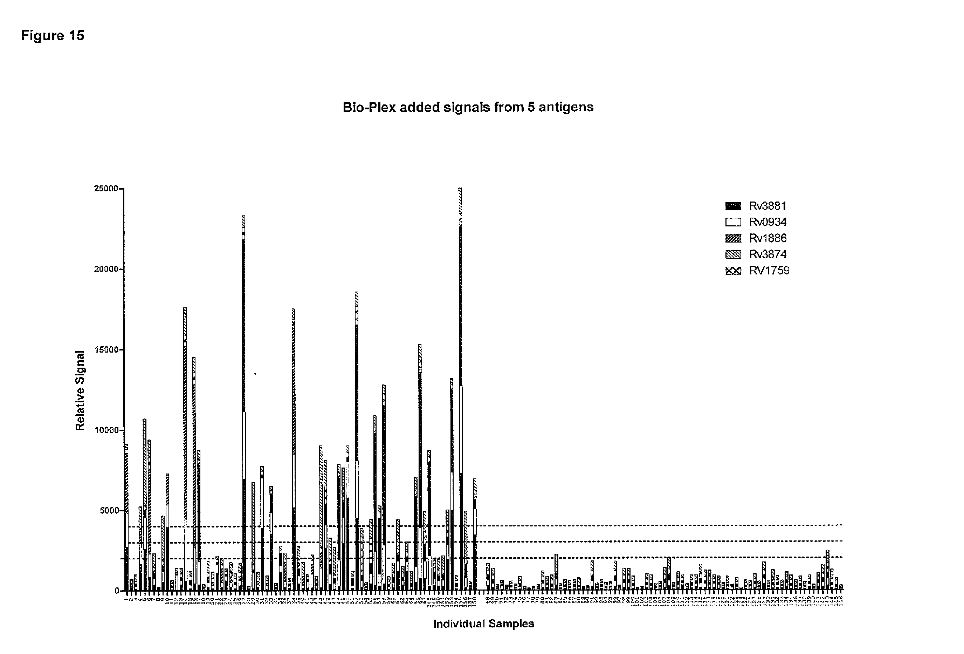

Figure 15 is a graphical representation of the Bio-Plex Assay results, as illustrated in Figure 8(a), showing the signal contribution for each fusion protein used in the test.
Figure 16 illustrates the eight optimised DNA sequences for each antigenic region, containing the coding sequence of the antigen (no underline), the flanking sequences (underlined) and the restriction enzyme sites (in bold).

### Detailed Description of the Invention

The present invention is based on the finding that individuals with an active TB infection have circulating antibodies which are specific for certain protein antigens of the *M. tuberculosis* organism hereinafter referred to as "target antigens". The target antigens are all proteins encoded by genes within the *M. tuberculosis* genome. Importantly, individuals with latent TB do not, generally, have circulating antibodies against these antigenic proteins. The target antigens of the invention and the names of the genes encoding them are set out in Table 1.

**Table 1 : The target antigens of the invention**

| **Gene** | **C-terminal** | **N-terminal** | **Preferred Orientation** |
|---|---|---|---|
| Rv3881c | **Yes** | **Yes** | **C-terminal** |
| Rv0934 | **Yes** | **Yes** | **N-terminal** |
| Rv1886c | **No** | **Yes** | |
| Rv1759c | **Yes** | **No** | |
| Rv1980c | **No** | **Yes** | |
| Rv1860 | **No** | **Yes** | |
| Rv3874 | **Yes** | **No** | |
| Rv3875 | **No** | **Yes** | |

The preferred orientation of the antigenic regions in relation to the anchoring region of the fusion protein is presented in Table 2.

**Table 2: Preferred orientation of the antigenic regions in relation to anchoring region**

| **Gene** | **Alternative names for Gene** | **Protein sequence (SEQ. ID NO.)** | **Optimized DNA coding sequence (SEQ. ID NO.)** |
|---|---|---|---|
| Rv3881c | - | 1 | 9 |
| Rv0934 | PSTS1; PBP-1 | 2 | 11 |
| Rv1886c | fbpβ | 3 | 13 |
| Rv3875 | | 8 | 23 |
| Rv1759c | - | 4 | 15 |
| Rv3874 | | 7 | 21 |
| Rv1860 | | 6 | 19 |
| Rv1980c | MPT64 | 5 | 17 |

The invention provides, in one aspect, a recombinant fusion protein comprising an antigenic region and an anchoring region. Since the fusion protein is recombinant, the anchoring region is of a sequence that is not found adjacent to the antigenic region in nature. The antigenic region comprises a target antigen, this is to say an amino acid sequence encoded by Rv3881c, Rv0934, Rv1886c, Rv1759c, Rv1980c, Rv1860, Rv3874 and Rv3875. However, the antigenic region need not contain an entire target antigen. Instead, the antigenic region may comprise a shorter amino acid sequence, which is still antigenic.

Furthermore, the amino acid sequence of the antigenic region may be different from the target antigen sequence that is encoded by the corresponding *M. tuberculoses* gene, provided that it has at least 80% sequence identity to the naturally occurring target antigen sequence and is bound by antibodies against the naturally occurring target. In other embodiments, the amino acid sequence of the antigenic region has at least 90%, 95% or 99% sequence identity to the target region. It is also preferred that any addition or substitution of the amino acid sequence set out in SEQ. ID NOS. 1 to 8 results in the conservation of the properties of the original amino acid side chain. That is to say the substitution or modification is "conservative".

Conservative substitution tables providing functionally similar amino acids are well known in the art. Examples of properties of amino acid side chains are hydrophobic amino acids (A, I, L, M, F, P, W, Y, V), hydrophilic amino acids (R, D, N, C, E, Q, G, H, K, S, T), and side chains having the following functional groups or characteristics in common: an aliphatic side-chain (G, A, V, L, I, P); a hydroxyl group containing side chain (S, T, Y); a sulphur atom containing side-chain (C, M); a carboxylic acid and amide containing side-chain (D, N, E, Q); a base containing side-chain (R, K, H); and an aromatic containing side-chain (H, F, Y, W). In addition, the following eight groups each contain amino acids that are conservative substitutions for one another (see e.g. Creighton, Proteins (1984):
1) Alanine (A), Glycine (G);
2) Aspartic acid (D), Glutamic acid (E);
3) Aspargine (N), Glutamine (Q);
4) Arginine (R), Lysine (K);
5) Isoleucine (I), Leucine (L), Methionine (M), Valine (V);
6) Phenylalanine (F), Tyrosine (Y), Tryptophan (W);
7) Serine (S), Threonine (T); and
8) Cysteine (C), Methionine (M).

In some embodiments, the antigenic region comprises non-naturally occurring amino acids but is still bound by antibodies against one of the target antigens.

The anchoring region comprises a sequence of at least 25 amino acids in length and is adjacent to the antigenic region. In alternative embodiments the anchoring region is larger than 25 amino acids and may, for example, comprise at least 30, 40, 50, 60, 70, 80, 90, 100 or 150 amino acids. In preferred embodiments, the anchoring region comprises at least a part of the Fc region of an immunoglobulin molecule, in particular constant domains 2 and 3 and the hinge region thereof in the case of an Fc region from an IgG, IgA or IgD antibody or constant domains 2 to 4 and the hinge region thereof in the case of an Fc region from an IgE or IgM antibody. Such Fc regions have the ability to bind Protein A and Protein G which enables straightforward purification of the fusion protein. The Fc region can come from any species with mouse, rat and rabbit being preferred. A particularly preferred anchoring region is the murine IgG2a Fc region as there are polyclonal antibodies available which are exceptionally selective for this Fc region which enables a high level of sensitivity in the assays described below. In an alternative embodiment the anchoring region is an entire IgG molecule, preferably a murine IgG2a antibody. An alternative anchoring region is a His-tag or glutathione S-transferase. The purpose of the anchoring region is to increase the solubility of the fusion protein and to increase the stability of the fusion protein. In some embodiments, the anchoring region also enables binding of the fusion protein to another component (e.g. a substrate or an antibody) whilst allowing the antigenic region to be displayed, without blocking binding of an antibody to the antigenic region.

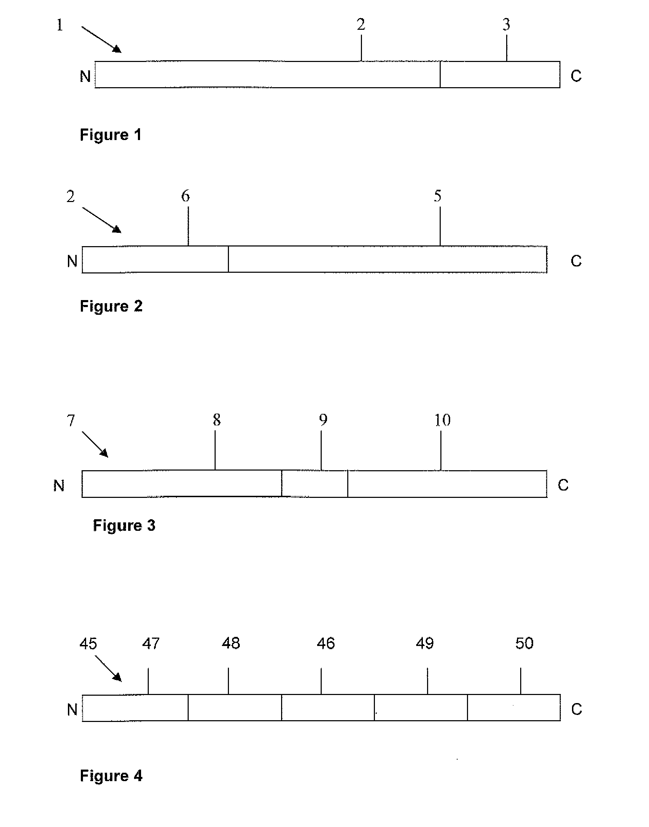

Referring, now, to Figure 1, a recombinant fusion protein 1, in accordance with one embodiment of the present invention is shown. The recombinant fusion protein comprises an antigenic region 2 which is at the N-terminal end of the fusion protein with an anchoring region 3 C-terminal thereof. Referring to Figure 2, an alternative embodiment of the present invention is shown in which a fusion protein 4 comprises an antigenic region 5 which is at the C-terminal end of the fusion protein with an anchoring region 6 being N-terminal of the antigenic region 5.

In still further embodiments of the present invention, there are provided fusion proteins having a plurality of antigenic regions. For example, in one embodiment, shown in Figure 3, a fusion protein 7 comprises a first antigenic region 8, at its N-terminus. At the C-terminal end of the first antigenic region 8, there is provided an anchoring region 9. At the C-terminal end of the anchoring region 9, there is provided a second antigenic region 10. Thus, in this embodiment, the fusion protein 7 comprises an anchoring region 9 which is flanked by first and second antigenic regions 8, 10. Referring to Figure 4, an alternative embodiment of the present invention is shown in which, a fusion protein 45, comprises, at its N-terminus a first antigenic region 47, and a second antigenic region 48, and at its C-terminus, a third antigenic region 49, and a fourth antigenic region 50. An anchoring region 46 is flanked by the first and second antigenic regions 47 and 48 and the third and fourth antigenic regions 49 and 50.

In another aspect of the present invention, there are provided nucleic acid molecules which encode a fusion protein of the present invention. Owing to the degeneracy of the nucleotide code, a wide range of different sequences may encode the same amino acid sequence. The nucleic acid molecule may be of any nucleotide sequence and may even comprise nucleic acid analogues, provided that the nucleic acid molecule encodes the fusion protein. Nevertheless, it is preferred that the nucleic acid molecule is of a sequence which is optimised for expression in a particular host cell (e.g. mammalian cells). Nucleotide sequences optimised for expression in human cells and encoding target antigens of the invention are listed in Table 1.

In some embodiments, the nucleic acid molecule encodes additional sequences, aside from those which specifically encode the fusion protein. For example, in some embodiments, the nucleic acid molecule also encodes a 5' terminal signal peptide for excretion of the fusion protein by a host cell in which it is expressed. For example if the host cell is a mammalian host cell then it is preferred that the signal peptide be a mammalian leader sequence. An exemplary signal peptide is Human Spd195, having the sequence of SEQ ID NO. 25. A further exemplary signal peptide is a signal peptide having the sequence of SEQ ID NO. 26. The signal peptide may be one which directs the fusion protein to one of several internal cell compartments, such as the nucleus, golgi apparatus or the mitochondria. In a preferred embodiment the signal peptide is one which directs the fusion protein to be secreted out of the cell entirely. Other additional sequences that the nucleic acid molecule may comprise include regulatory regions such as enhancers.

In another aspect of the present invention, there is provided a vector comprising a nucleic acid molecule according to the present invention. In particular embodiments, the vector may, in addition to the sequence of the nucleic acid molecule, comprise components such as an origin of replication, genetic markers, antibiotic resistance genes, COS sites, cloning sites and targeting sequences. Exemplary vectors include cosmids, and viral vectors such as bacteriophage.

In a further aspect of the present invention, there is provided a host cell comprising a vector of the present invention or a nucleic acid molecule of the present invention recombinantly introduced into the genome of the host cell. In preferred embodiments, the host cell is a mammalian cell such as a 293E cell. The host cell is capable of expressing the nucleotide sequence encoding the fusion protein.

In order to synthesize a fusion protein in accordance with the present invention, a nucleic acid molecule of the present invention is first synthesised using standard microbiological techniques. The nucleic acid molecule is then incorporated into an expression vector, comprising the necessary elements for sustaining and reproducing the vector in a host cell and also elements, such as an antibiotic resistance gene, for identifying transfected cells. A suitable expression vector is the pFRIDA vector. The pFRIDA vector may be a pFRIDA-IgG2a-N having a sequence of SEQ ID NO. 27. Alternatively, the pFRIDA vector may be a pFRIDA-IgG2a-C having a sequence of SEQ ID NO. 28. A host cell is then transfected with the expression vector using techniques known in the art such as the calcium phosphate technique, electroporation, Polyethylenimine (PEI) transfection or by using a cationic lipid. Preferred host cells are mammalian cells, in particular 293E cells. After confirming that the host cell has been transfected with the vector of the invention, the cell is cultured and the nucleic acid molecule encoding the fusion protein is expressed.

In some embodiments, the nucleic acid molecule also encodes a secretory signal peptide and the anchoring region is an Fc region from an IgG molecule. In these embodiments, the fusion protein is translated containing the secretory signal peptide which leads to the protein being exuded from the cell (and the signal peptide cleaved off) by virtue of the host cell's expression systems. In particular, the Fc region enables the fusion protein to exit the plasma membrane of the cell by improving the solubility and stability of the antigenic region. In these embodiments, the fusion protein can then be recovered from the extracellular culture medium in a relatively straightforward manner by, for example, purification on a column (e.g. a protein A column if the anchoring region is an Fc region).

However, in other embodiments, where the secretory signal peptide is absent and/or the anchoring region does not comprise an Fc region, the expressed fusion protein remains intracellular and is recovered by lysing the host cell and purifying the fusion protein, again, by passing the protein through a column containing binding elements (e.g. antibodies) capable of binding the anchoring region.

Once a fusion protein of the invention has been expressed and purified, it is, in certain embodiments of the invention, incorporated into a kit for the detection of active TB in a patient.

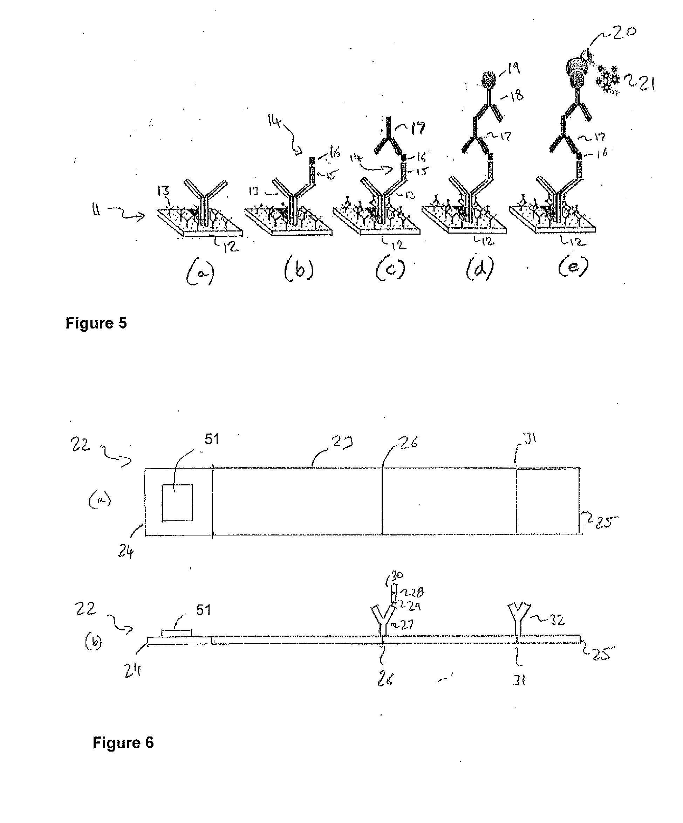

Referring to Figures 5(a) to 5(e), one embodiment of a kit is shown schematically. A kit 11 (of which only a portion is shown in Figure 5) comprises an ELISA plate 12 (a section of which is shown in Figures 5(a) to 4(e)). Immobilised on the surface of the ELISA plate 12 are a plurality of goat anti-murine IgG2a antibodies 13 via covalent bonds. Each of the anti-murine IgG2a antibodies 13 is specific for the murine IgG2a hinge/Fc region which forms the anchoring region of the fusion protein in this embodiment.

Figure 5(b) disclose a section of the prepared kit 11 in which a fusion protein 14 has been added to the surface of the ELISA plate. The fusion protein 14 comprises an anchoring region 15 bound to the variable domain of an anti-murine IgG2a and an antigenic region 16 which is displayed. The kit 11 also comprises a supply of a secondary antibody (18) (not shown in Figure 5(b)) which is an anti-human IgG antibody conjugated to a reporter enzyme 19. The kit 11 is also provided with a supply of a substrate of the enzyme which undergoes a colour change on conversion by the reporter enzyme. The kit 11 of which a section is shown in Figure 5(b) is in the form in which it is distributed and sold.

In use of the kit 11 for detection of active TB, an individual presents to a clinic for testing. The individual has symptoms which suggest possible infection with TB. Alternatively, the individual may be tested because he or she has had contact with another individual who has been infected with TB or may present as part of a general screening programme. A biological sample is then taken from the individual. In this embodiment, the biological sample is a blood sample but in other embodiments, a sample of bodily fluid such as saliva is obtained instead. Serum, if necessary, is obtained from the blood sample by centrifugation. Whole blood samples are suitable for use in rapid point of care assays. The biological sample is then prepared (e.g. by addition of a protease inhibitor). The prepared sample is then deposited on the ELISA plate 12.

If the individual has active TB then the individual's blood/serum contains an active TB-specific antibody 17 which binds the antigenic region 16 of the fusion protein 14 as is shown in Figure 5(c).

Referring to Figure 5(d), the next stage in the use of the kit is shown. The secondary antibody 18 to which is conjugated the reporter enzyme 19 is then added to the ELISA plate 12. The secondary antibody 18 binds to the Fc region of the active TB-specific antibody 17. Subsequently, the plate 12 is washed in order to remove unbound antibody and the enzyme substrate 20 is deposited on the plate 12, as is shown in Figure 5(e). The substrate 20 is converted by the reporter enzyme in order to produce a detectable product 21. Thus the presence of the detectable product 21 is indicative of the presence of the active TB-specific antibody 17 in the biological sample of the individual, which is, in turn, indicative of the individual having active TB.

In the case of an individual without active TB, the individual's biological sample does not contain antibodies capable of binding the antigenic region 16. Thus, no antibody binds to the fusion protein 14 that is deposited on the ELISA plate 12. The secondary antibody 18 is washed from the plate before addition of the enzyme substrate 20 and, in the absence of the reporter enzyme 19, none of the detectable product 21 is produced. Thus for a patient who does not have active TB, none of the detectable product 21 is produced.

The kit 11 thus permits rapid detection of active TB in an individual, or at least a high likelihood of active TB in an individual. An individual identified in this way will be referred for confirmatory testing at a clinic or hospital with laboratory facilities. An individual with positive confirmatory test results will be treated with appropriate antibiotics such as isoniazid or rifampicin and thus the spread of infection will be reduced. The kit 11 has a low level of false-negative results. To exclude false-positive results, and ensure correct diagnosis, individuals testing positive in an assay based on the kit 11 will be re-tested with conventional techniques such as sputum smear microscopy and mycobacterial culture.

It is to be understood that many variations in the kit 11 described in this embodiment may be made. For example, in some embodiments, the anti-immune IgG2a antibodies 13 are not provided and, instead, the fusion protein 14 is directly bound to the plate 12, for example by a covalent bond. In these embodiments, it is not essential that the anchoring region be provided since, in some embodiments, the antigenic region 16 is directly bound to the plate 12 (e.g. by a covalent bond). In still further embodiments, the antigenic region 16 is attached to the plate 12 by virtue of a binding pair other than an antigen-antibody. For example, in one embodiment, the plate 12 is coated with biotin and the antigenic region 16 is bound to streptavidin or avidin in place of the anchoring region. Thus on depositing the antigenic region 16 on the plate, the streptavidin or avidin binds to the biotin and the antigenic region 16 is immobilised on the plate 12.

Referring to Figures 6(a) and 6(b), a kit 22 for the detection of active TB in a patient in accordance with another embodiment of the present invention is shown. The kit 22 comprises a nitrocellulose strip 23 having first and second ends 24, 25. The strip is such that fluid may pass along it via capillary action. Adjacent to the first end 24 of the strip 23 is provided a sample receiving zone 51 comprising an absorbent pad. Approximately half way between the first and second ends 24 and 25 of the strip 23 is provided a detection zone 26. The detection zone 26 comprises a plurality of goat anti-murine IgG2a antibodies immobilised in a line which is perpendicular to the longitudinal axis of the strip 23. The goat antibodies 27 are immobilised on the strip 23 in the same manner as described in relation to the embodiment shown in Figure 6. Bound to the variable region of the goat antibodies 27 is provided a fusion protein 28 which consists of an anchoring region 29 which is bound to the goat antibody 27 and an antigenic region 30 which comprises one of the target antigens as described above.

Adjacent to the second end 25 of the strip 23 is provided a control zone 31, which comprises a plurality of anti-human IgG antibodies 32 immobilised covalently on the strip 23 in a line perpendicular to the longitudinal axis of the strip 23.

Also provided with the kit 22 is a supply of a secondary antibody (not shown) which is also an anti-human IgG antibody and which is conjugated to a detectable label such as a latex particle or a gold particle.

In use of the kit as is shown in Figure 6, a biological sample is obtained from an individual, as described in the previous embodiments. If the sample has been correctly obtained then it contains antibodies from the individual and, if the individual has active TB, these antibodies include active TB-specific antibodies. The biological sample is added to phosphate buffered saline (PBS) or Chaser Buffer. The sample may be treated with a number of compounds, for example, a protease inhibitor can be added to avoid degradation of antibodies, detergents can be added to minimize unspecific binding in the test, blocking agents can be added such as BSA (bovine serum albumine) and skimmed milk to enhance the specific binding in the test or thiocyanate salts can be added in order to abolish low avidity antibody-antigen interactions, eliminating background signal in the test.

The sample is deposited on the sample receiving zone 51. The sample soaks through the absorbent pad and, by capillary action, passes from the first end 24 to the second end 25 of the strip 23. If the individual has active TB then, when the biological sample reaches the detection zone 26, active TB-specific antibodies in the biological sample bind to the antigenic region 30 of the fusion protein 28 and are immobilised there. The remainder of the biological sample, including other antibodies in the sample that are not specific for TB, passes to the second end 25 of the kit 22. The non-TB-specific antibodies in the sample bind to the anti-human IgG antibodies 32 in the control zone 31 and are immobilised there.

Subsequently, a buffer containing the secondary antibody (not shown) is added to the sample receiving zone 51. Upon it being deposited at the sample receiving zone 51, the secondary antibody soaks through the absorbent pad and passes along the strip 23 from the first end 24 to the second end 25, by capillary action. When the secondary antibody reaches the detection zone 26, it binds to the active TB-specific antibody from the individual and the concentration of the detectable label is such that a visible line appears at the detection zone 26. The appearance of this visible line at the detection zone 26 is thus indicative of the presence of antibodies capable of binding a target antigen in the individual and thus is indicative of the individual having active TB.

An excess of secondary antibody is provided on the sample receiving zone 51 so some of the secondary antibody passes through the detection zone 26 and binds to the non-TB-specific antibodies that are immobilised at the control zone 31. The concentration of the detectable label at the control zone 31 is thus indicative that the assay has completed because the biological sample has passed through the detection zone 26 and the outcome of the assay should be visible at the detection zone 26. Indeed, the relative intensities of the lines at the detection zone 26 and the control zone 31 can be compared to determine whether a visible line is present at the detection zone 26. This helps to avoid false negative results which could occur if, for example, there is insufficient liquid in the biological sample for any of the biological sample to reach the detection zone 26 or if the biological sample has been incorrectly obtained and does not contain any antibodies from the individual.

In cases where the individual does not have active TB, the biological sample does not have active TB-specific antibodies capable of binding a target antigen. After depositing of the biological sample on the sample receiving zone 51, the sample passes along the strip 23, as described above, but passes through the detection zone 26 since there are no antibodies in the biological sample capable of binding the antigenic region 30 of the fusion protein 28. Thus the sample passes all the way to the second end 25 of the strip 23. The non-TB-specific antibodies in the sample bind to the anti-human IgG antibodies 32 in the control zone 31 and are immobilised there. When the secondary antibody is deposited on the sample receiving zone 51, this too passes along the strip 23 from the first end 24 to the second end 25, as described above but passes through the detection zone 26 because there is no active TB-specific antibody immobilised at the detection zone 26 for the secondary antibody to bind to. However, the secondary antibody does bind to the non-TB-specific antibodies that are immobilised at the control zone 31 and thus the secondary antibody is immobilised at the control zone 31 and the concentration of the detectable label at the control zone 31 is visible to the naked eye. The presence of a visible line at the control zone 31 is indicative that the assay preformed using the kit 22 has run its course and the absence of a visible line at the detection zone 26 is indicative that the individual does not have active TB.

It is to be understood that in other embodiments of the present invention, the goat antibody 27 is not provided. Instead, in these embodiments, the fusion protein 28 is immobilised directly onto the surface of the strip 23, for example by covalent binding. Indeed, in some of these embodiments, it is not even necessary for a fusion protein to be provided since the antigenic region 16 is covalently bound directly to the surface of the strip 23. Furthermore, the provision of the control zone 31 is not essential to the invention since the assay can be run without confirmation that it has been completed.

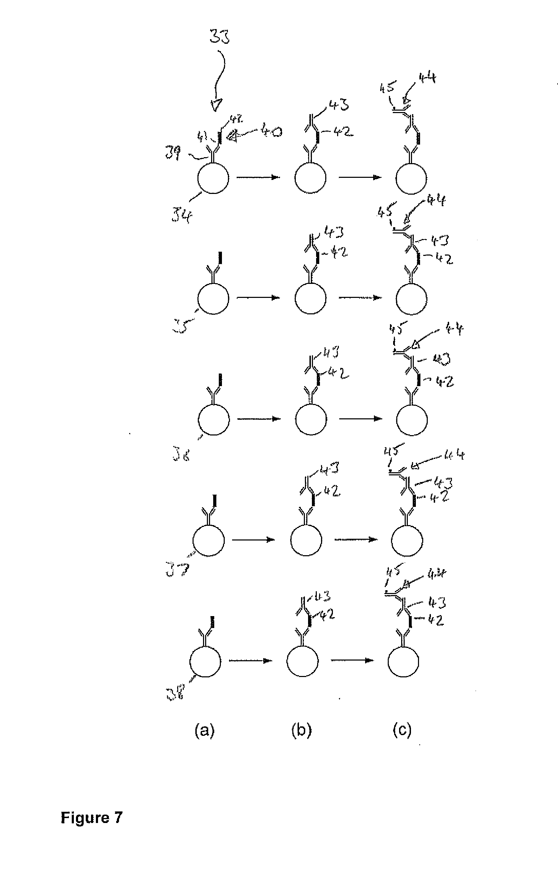

Referring to Figure 7, another embodiment of a kit for the detection of active TB in an individual is shown in schematic form. As shown in Figure 7(a), the kit 33 comprises five sets of polymer particles 34 to 38 (for convenience, each set of particles is shown as a single particle in Figure 7). Each set of particles 34 to 38 is marked with a different label, such as a series of dyes selected from the Alexa Fluor family of fluorescent dyes produced by Molecular Probes. Referring now to the particle 34 of the first set, the particle 34 has bound to its surface a plurality of goat anti-murine IgG2a antibodies 39, for example by protein G affinity coupling. The variable region of the goat antibody 39 is bound to a fusion protein 40 which comprises an anchoring region 41 which is bound to the goat antibody 39 and an antigenic region 42 which has the sequence of SEQ. ID NO. 1.

Each of the second to fifth sets of particles 35 to 38 also has displayed on it a goat antibody and a fusion protein. However, for each set of particles 34 to 38 the fusion protein 40 is different in that the antigenic region is of a different sequence. More specifically, while the antigenic region 42 of the first set of particles 34 comprises the sequence of SEQ. ID NO. 1, the second antigenic region 42 of the fusion protein 40 on the second set of particles 35 comprises the sequence of SEQ. ID NO. 2; the third set of particles 36 has an antigenic region 42 comprising the sequence of SEQ. ID NO. 3 and so forth up the fifth set of particles 38 which display an antigenic region 42 comprising the sequence of SEQ. ID NO. 5. Thus the kit 33 comprises a mixture of these five sets of particles 34 to 38. The kit also comprises a supply of a secondary antibody 44 which is an anti-human IgG antibody carrying a detectable label 45 such as a fluorescent or luminescent dye. In some alternative embodiments, the secondary antibody does not carry the detectable label, itself. Instead, the detectable label is attachable to the secondary antibody, for example by a binding pair such as biotin and streptavidin or avidin.

In use, a biological sample is obtained from an individual and is processed in the manner described for the previous embodiments. The processed sample is then mixed with the five sets of particles 34 to 38. If the individual has active TB and thus has active TB-specific antibodies in the biological sample which bind to the target antigens then the individual's antibodies 43 bind to the respective antigenic regions 42 displayed on the corresponding first to fifth sets of particles 34 to 38, as shown in Figure 7(b).

After mixing of the biological sample with the set of particles, the secondary antibody 44 carrying the detectable label 45 is mixed with the particles. The secondary antibody 44 binds the Fc region of all of the antibodies in the biological sample, irrespective of the binding specificity of the human antibodies, as is shown in Figure 7(c). The mixture of particles 34 to 38 is then analysed using a flow cytometer. More specifically, the flow cytometer examines each particle and detects the presence or absence of the detectable label 45 in association with each particle, as well as the identity of the dye which marks the particle, itself. Since each set of particles 34 to 38 displays a different antigenic region 42, the output from the flow cytometer is therefore indicative of whether the biological sample contained antibodies capable of binding each of the antigenic regions 42. Thus this embodiment allows a multiplex detection for antibodies specific for five different antigenic regions 42 representative of the different target antigens.

If, however, the individual from whom the biological sample is taken does not have active TB then the biological sample does not contain any active TB-specific antibodies. Accordingly none of the individual's antibodies in the biological sample bind to the antigenic regions 42 displayed by the five sets of particles 34 to 38. Thus when the secondary antibody 45 is added, although it binds to the human antibodies 44 in the mixture, it is not associated with the particles 34 to 38, themselves, since there is no link between the secondary antibody 44 and the particles 34 to 38. When the particles 34 to 38 are analysed in the flow cytometer, there is no association between the detectable marker 45 and each particle 34 to 38. The absence of such a signal is therefore indicative of the absence of active TB in the individual.

It is also to be understood that in some circumstances an individual from whom the biological sample is taken has antibodies that bind some of the target antigens, but not all of the target antigens. For example, in one embodiment, the individual has antibodies capable of binding epitopes in SEQ. ID NOS. 1 to 3 but not epitopes in SEQ. ID NOS. 4 or 5. In these circumstances, the secondary antibody becomes associated with only the first to third sets of particles 34 to 36 but does not become associated with the fourth and fifth sets of particles 37, 38 since there is no active TB-specific antibody to bind the secondary antibody 44 to the antigenic region 42 of the fourth and fifth sets of particles 37, 38. In these circumstances, the flow cytometer detects the detectable label 45 only in association with the first to third sets of beads 34 to 36 and generates a signal indicative of this. This information then enables clinicians to make a decision as to the diagnosis of the individual. For example, in one embodiment the detection in a biological sample from an individual of active TB-specific antibodies against at least one of the target antigens (i.e. SEQ. ID NOS. 1 to 8) is indicative of the individual having active TB.

It is to be appreciated that in some embodiments the goat antibody 39 is not provided and instead the anchoring regions 41 are bound directly to the surface of their respective particles 34 to 38. Alternatively, the anchoring regions 41 are omitted and the antigenic regions 42 are bound directly to the surface of their respective particles 34 to 38. The anchoring regions 41 or antigenic regions 42 may be bound to the surface of their respective particles 34 to 38 either by a covalent bond or by another binding pair such as biotin and streptavidin or avidin.

It is also to be understood that the present invention is not limited to the provision of kits comprising five different sets of particles. It is to be understood that in a further embodiment eight different sets of particles are provided, in which for each set of particles the fusion protein is different in that the antigenic region is of a different sequence, namely SEQ. ID NO. 1 to SEQ ID NO.8, respectively. In some embodiments 1, 2, 3, 4, 6, or 7 different sets of particles are provided instead.

### Examples

### Materials and Methods

### Cloning and production of the TB fusion protein

### Synthesis of TB antigen

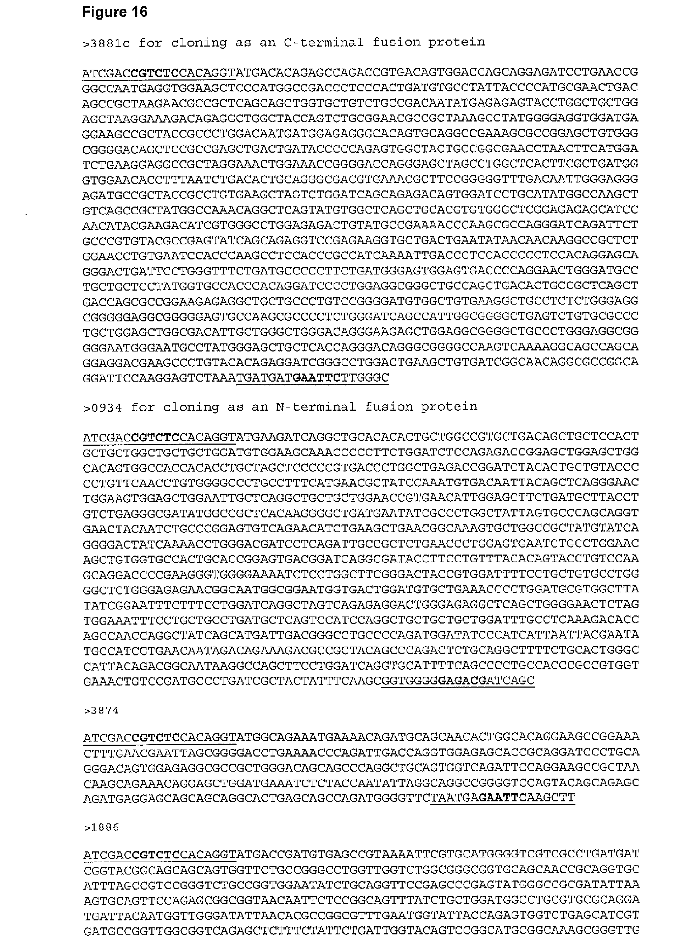

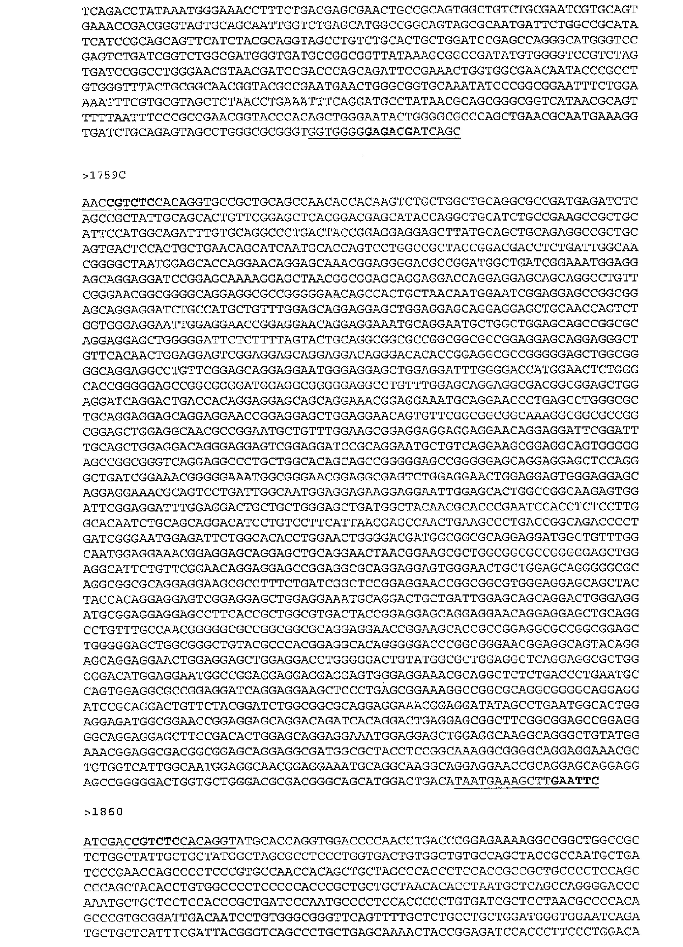

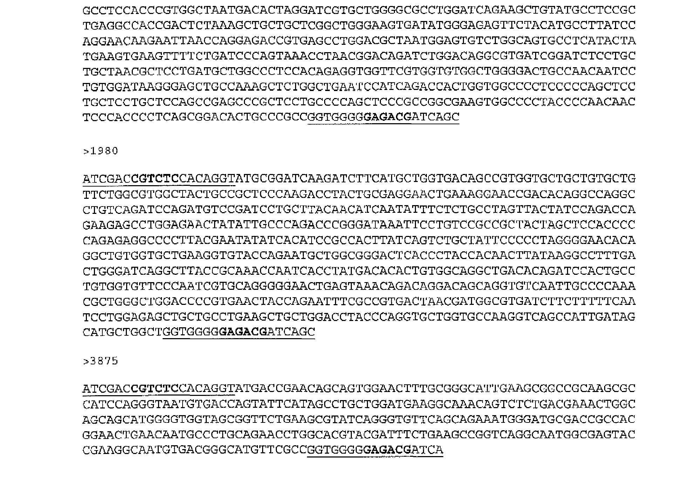

The sequence encoding the gene of the TB antigen to be produced as a fusion protein was retrieved from publicly available databases. Flanking sequences containing endonuclease restriction sites were added to the gene sequence, to make the gene compatible with expression in the pFRIDA vector. The flanking sequences containing the restriction enzyme sites added to each gene sequence are shown in Figure 16. The genes were synthesised by GenScript (USA), optimizing the DNA codons for expression in human cells. The synthesised genes where received as an insert in a pUC57 vector construct.

### Cloning of TB antigen

2 µg of the pUC57 vector containing the synthesized gene encoding the TB antigen was digested with restriction enzymes, *Esp*3I (www.fermentas.com) and *Eco*RI (www.fermentas.com) in a reaction volume of 60 µl for C-terminal cloning. For N-terminal cloning, only the *Esp*3I enzyme was added to the reaction. The digestion reaction was incubated for 60 minutes at 37°C. The resulting DNA fragments were separated by agarose gel electrophoresis, and the DNA fragment corresponding to the gene encoding the TB-antigen was isolated by excision. The excised gel fragment was treated according to the protocol of the Qiagen (www.qiagen.com) MinElute Gel Extraction Kit, and the DNA was recovered.

The recovered DNA encoding the TB antigen was added to the corresponding pFRIDA vector for either N-terminal or C-terminal expression as a fusion protein. The pFRIDA vector had been previously digested with either *Esp*3I, or *Esp*3I and *Eco*RI, and then purified by agarose gel electrophoresis. A molar ratio of 3:1 of the insert:vector was mixed, and 0.5 µl T4-DNA ligase (www.neb.com) was added in a total reaction of 25 µl. The ligation reaction was incubated at 20-24°C for 1-2 hours.

### Transformation of E.coli

30 µl of competent XL-10 Gold *E.coli* cells where added 10 µl of the ligation reaction and treated as described by the manufacturer (www.agilent.com). The resulting transformation mix was spread on solid agar plates containing 100µg/ml ampicillin. The agar plates were incubated 12-16 hours at 37°C.

### Bacteria colony isolation, growth and DNA plasmid isolation

From the incubated agar plates, 5-10 single colonies where isolated and grown individually in 2 ml of liquid 2xYT medium (www.sigma-adrich.com) containing 100µg/ml ampicillin (www.sigma-adrich.com). The colonies where incubated for 6-8 hours with shaking (300rpm) at 37°C. Each of the colony suspensions were then treated with the QIAprep Spin Miniprep Kit, according to the manufacturer's (www.qiagen.com) instructions. This resulted in the recovery of about 10µg of plasmid DNA.

### Verification of DNA plasmid

To verify that the gene encoding the TB antigen had been ligated successfully into the pFRIDA vector, the isolated plasmids where digested with restriction enzymes Nhel and *Eco*RI, and the resulting pattern of DNA fragments analyzed on an agarose gel. In some cases standard DNA sequencing was performed.

### PEI Transfection of 293E cells

Plasmids verified to contain the gene encoding the TB antigen were used in a PEI transfection procedure.

Approximately 2 million 293E cells were suspended in 10 ml of DMEM (www.invitrogen.com) with glutamine and 10% fetal calf serum (FCS) was added to a 15 ml bottle (Nunc, Denmark) and incubated overnight in a CO₂-incubator.

150 µl RPMI medium (www.invitrogen.com) was then added to 3 µg plasmid DNA and heated to 80°C for 5 minutes, and then cooled to 0-4°C. 25 µl of PEI solution (www.polysciences.com) was then added and the reaction mix was incubated at room temperature for 8-12 minutes. 1350 µl of DMEM (www.invitrogen.com) with glutamine and 10% FCS (www.invitrogen.com), was then added.

The cell growth medium in the 15 ml Nunc (www.nunc.com) bottle was removed, and the transformation reaction (about 1.5 ml) was added to the bottle, ensuring the liquid covered all the cells in the bottle. The bottle was then incubated for 2 hours in the CO₂- incubator, before adding 10 ml of DMEM with glutamine and 10% FCS.

### Isolation of TB fusion antigen containing growth media

The growth media contained approximately 1-5 µg/ml of the TB antigen murine IgG2a fusion protein after 2-5 days growth of the transfected 293E cells. 10 ml of growth media was removed after 4-5 days, and replaced with 10 ml of fresh media for a possible second harvest of TB fusion protein in 5-10 days. The collected growth media was centrifuged at 2-4000 x g to remove cell debris, and added a preservative like sodium azide before being stored at 4°C.

### Bio-Plex Procedure

### Labelling of beads with antibody

Bio-Rad MagPlex beads (www.bio-rad.com) representing different regions, were labelled with Sigma-Aldrich product M4434 (goat-anti murine IgG2a antibody; www.sigma-adrich.com) using the Bio-Rad bead labelling kit (www.bio-rad.com) as instructed by the manufacturer. Approximately, 5x10⁶ beads were labelled, using 50µg M4434 for each reaction.

### Labelling of beads with TB-Antigen

Approximately 1-2x10⁶ beads of a specific region were added to 1 ml of a specific TB fusion protein containing growth media, and incubated for 1 hour at room temperature. For fusion proteins produced in low quantities, the beads were repeatedly incubated with more growth media to saturate the beads. The beads were then washed with 1 ml PBS (www.sigma-adrich.com) 3 times and then resuspended in 150 µl PBS per 1x10⁶ TB-antigen loaded beads.

### Preparation of Sample

2 µl of each serum sample to be tested was added to 20 µl PBS or Chaser Buffer.

### Incubation of TB-antigen labelled beads with human sera

Beads representing different regions with corresponding TB-antigens attached, were mixed in equal amounts and washed once in PBS. The bead mix was resuspended in 150 µl PBS per 1x10⁶ TB-antigen loaded beads. Approximately 100µl of this mix (1x10⁶) beads was used per 100 samples to be tested, and when testing 5 different antigens. For each 100 samples, 100 µl of the bead mix was added 5.5 ml of PBS with 0.05% Tween 20, 1 % skimmed milk, 1% BSA and 60mM NH₄SCN.

2 µl of each serum sample to be tested was added to 20 µl PBS or Chaser Buffer. The 20 µl PBS containing the 2 µl of serum was then added 50 µl of the bead mix, and the 70 µl reaction mixture was incubated at room temperature, or at 37°C, with shaking in a Bio-Plex incubation tray for 1 hour. The incubation tray was washed by the Bio-Plex magnetic washer, and the washed beads were resuspended in 50 µl of a 5.5 ml PBS solution containing 0.05% Tween 20 and 5.5 µl of anti-human IgG antibody (Sigma-Aldrich product P9170).

Secondary PE-labelled antibody was added and incubated at room temperature, or at 37°C, with shaking in a Bio-Plex incubation tray for 1 hour. The incubation tray was washed using Bio-Plex magnetic washer, and the washed beads were resuspended in 100 µl of PBS and briefly shaken to resuspend the beads.

### Detection of signal in Bio-Plex

The Bio-Plex machine was used as recommended by the manufacturer. The recorded results where exported to Microsoft Excel, and graphically represented by use of Prism/Graphpad.

### Sandwich ELISA Procedure

### Coating of ELISA wells with antibody

10 µg of Sigma-Aldrich product P9170 was added to 10 ml of PBS. 100 µl was added to each well of a 96 well Maxisorb ELISA plate (www.nunc.com). The Maxisorb plate was incubated at 4°C for 3-4 days or at room temperature for 16-24h.

### Coating ELISA wells with TB-antigen

The antibody-coated Maxisorb ELISA plates were washed three times with PBS containing 0.05% Tween 20. 100µl of growth medium containing TB-fusion protein was added to each well. The 100µl of growth medium could contain one or a mix of several antigens. The wells were incubated at 37°C for 1 hour. If the concentration of the TB-antigens in the growth medium was low, the step was repeated.

### Incubation of TB-antigen labelled ELISA wells with human sera

Excess fusion protein was removed by washing the wells three times with PBS containing 0.05% Tween 20. 2 µl of each serum sample to be tested was added 98 µl PBS with 0.05% Tween 20, 1 % skimmed milk, 1% BSA and 60mM NH₄SCN and added to a well. The sample solutions were incubated in a Maxisorb plate for 1 hour at 37°C. Excess serum proteins were removed by washing the wells three times with PBS containing 0.05% Tween 20. 2 µl of Sigma-Aldrich product A8542 was then added to 10ml of PBS containing 0.05% Tween 20, and 100 µl of this solution was added to each well. The Maxisorb plate was then incubated for 1 hour at 37°C.

### Detection of signal in ELISA

Excess secondary antibody was removed by washing the wells three times with PBS containing 0.05% Tween 20. The wells were added 100µl of a buffer containing alkali phosphatase, resulting in a yellow colour appearing in positive wells. The plate was allowed to develop for 10-40 minutes, and the results were recorded by an automatic ELISA plate reader. The recorded results where exported to Microsoft Excel, and graphically represented by use of Prism/Graphpad.

### Example 1: Detection of active TB using the Bio-Plex procedure

### Test Subjects

The Bio-Plex procedure was carried out on the following test groups:
(i) 78 Ethiopian patients with confirmed active TB and 79 healthy HIV-negative Ethiopian community controls. The community controls were people living in the same neighbourhood as the patients.
(ii) 132 patients from Tanzania with confirmed active TB and 60 healthy HIV-negative Tanzanian community controls. The community controls were people living in the same neighbourhood as the patients.
(iii) 36 Ethiopian patients with confirmed TB, 96 Ethiopian patients with suspected TB, i.e. patients with TB-like symptoms but where no disease was detected using conventional tests, 36 Ethiopian community controls and 23 Norwegian TB-negative persons.

### Fusion proteins

Five fusion proteins were used in the Bio-Plex procedure. The antigenic regions of the five fusion proteins are Rv3881c, Rv0934, Rv1886, Rv3875 and Rv1860. The anchoring region of each fusion protein is a murine IgG2a Fc fragment.

### Results

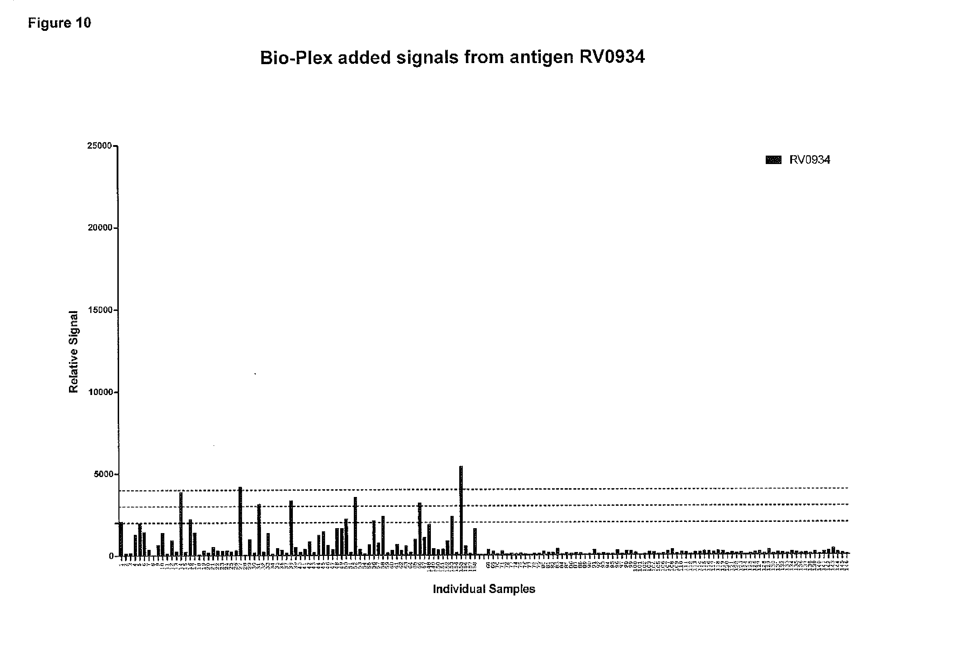

Referring to Figure 8 (a) and (b), the Bio-Plex test successfully distinguished (50-60%) between patients with active TB and community control patients, i.e. those living in the same neighbourhood as the patients with active TB. A sample was defined as positive if the signal generated was at, or above, the predetermined cut off level, i.e. the lowest line in Figure 8(a) to (c). Figure 8(a) illustrates the combined signal for all fusion proteins (Rv3881c, Rv0934, Rv1886, Rv3875 and Rv1860) used in the ELISA test. Figures 10 to 14, each illustrate the signal obtained from a single fusion protein, i.e., the signal contribution each fusion protein made to the total signal reported in Figure 8(a).

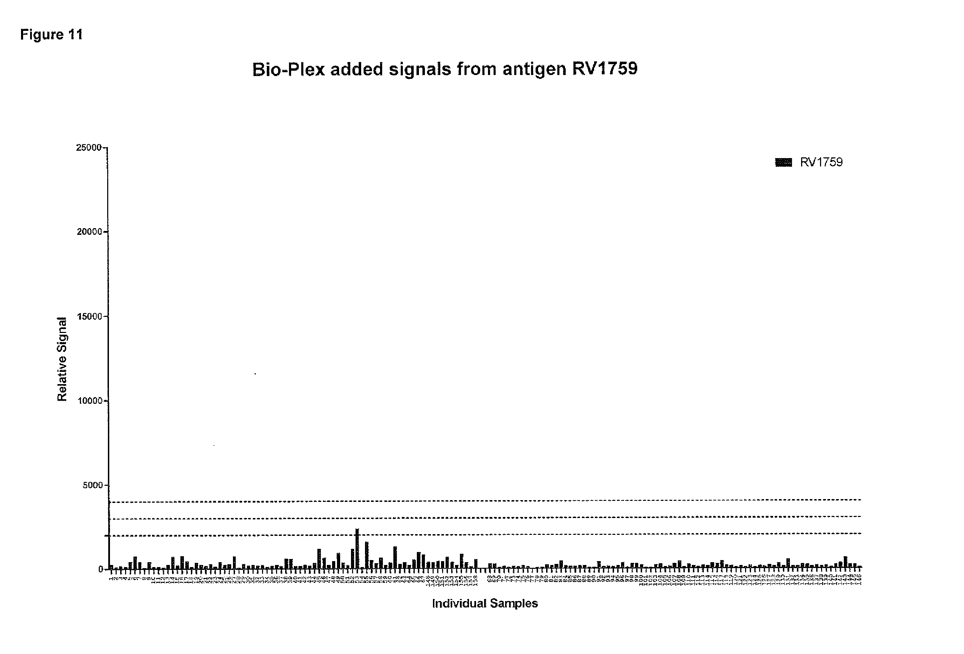

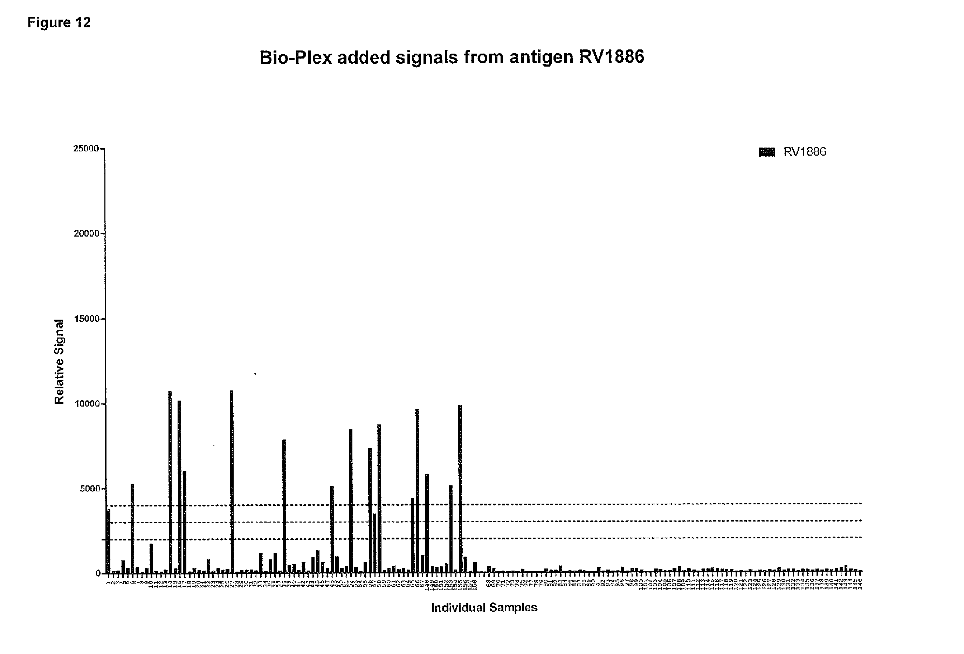

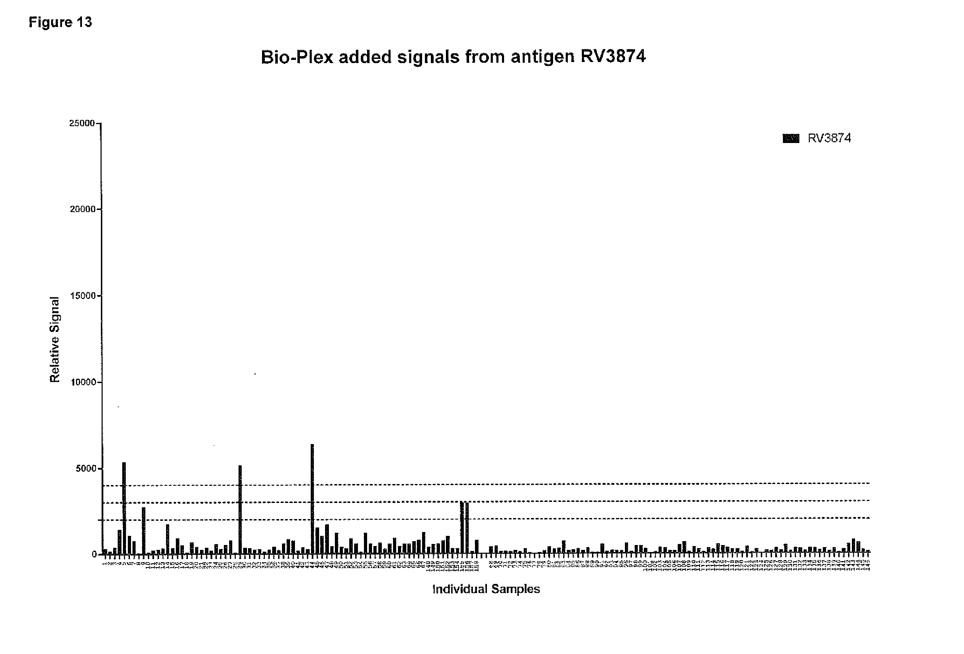

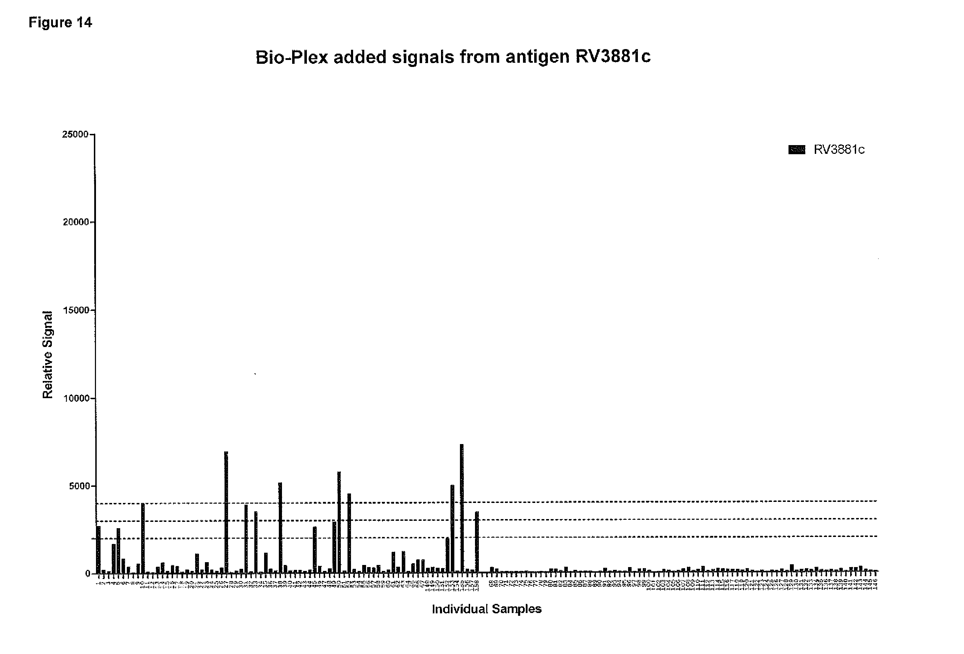

Figure 10 shows the number of patients testing positive for active TB using Rv1886 fusion protein. Figure 11 shows the number of patients testing positive for active TB using Rv3874 fusion protein. Figure 12 illustrates the number of patients testing positive for active TB using Rv1759 fusion protein. Figure 13 shows the number of patients testing positive for active TB using Rv0934 fusion protein. Figure 14 shows the number of patients testing positive for active TB using Rv3881 c fusion protein.

In Figure 8(b), 4 patients from the community control group also tested positive for active TB, suggesting that these non-symptomatic persons may be infected and developing TB disease.

Referring to Figure 8(c) the Bio-Plex test successfully distinguished (50-60%) between patients with active TB and the community controls or the "negative" controls. 4 patients with suspected TB also tested positive for active TB. This result may indicate that the Bio-Plex test detected active TB in a patient where conventional sputum smear microscopy and clinical diagnosis had failed.

### Validation

All patients tested were confirmed positive by smear tests and by growing cultures of TB bacteria from sputum samples taken from each patient.

### Example 2: Detection of active TB using the Rapid Test TB "prototype" ELISA Test Subjects

The Rapid Test TB "prototype" ELISA was carried out on the following test group:
(i) 78 Ethiopian patients with confirmed active TB and 79 healthy HIV-negative Ethiopian community controls. The community controls were people living in the same neighbourhood as the patients.

### Fusion Proteins

Five fusion proteins were used in the Rapid Test TB "prototype" ELISA. The antigenic regions of the five fusion proteins are Rv3881 c, Rv0934, Rev1886, Rv3875 and Rv1860, respectively. The anchoring region of each fusion protein is a murine IgG2a Fc fragment.

### Results

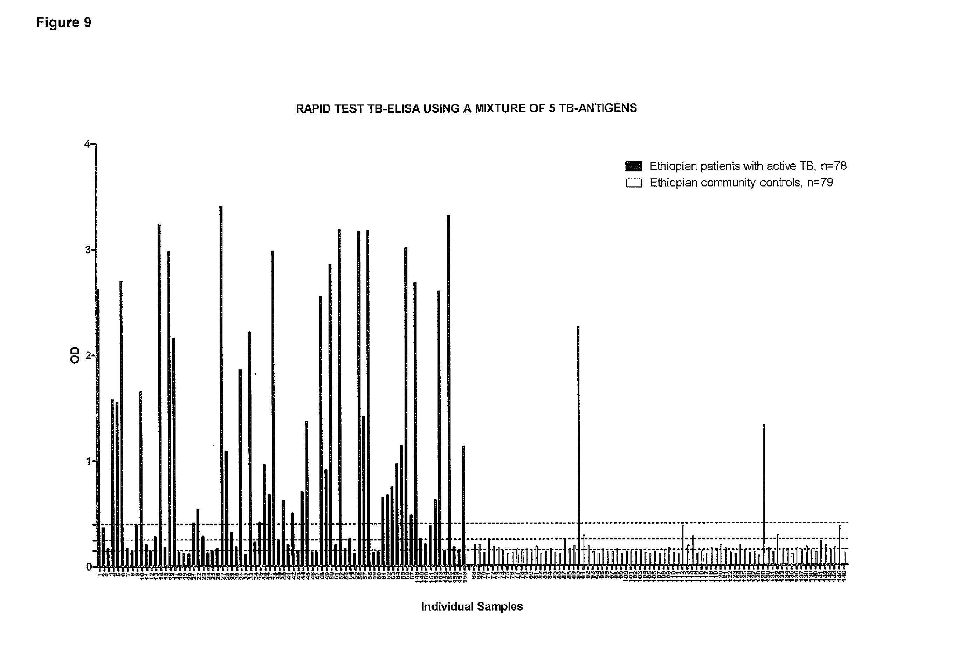

Referring to Figure 9, the ELISA test successfully distinguished (50-60%) between patients with an active TB and community control patients, i.e. those living in the same neighbourhood as the patients with active TB. 2 patients from the community control group also tested positive for active TB, suggesting that these patients are in fact developing a TB disease. A sample was defined as positive if the signal generated was at, or above, the predetermined cut off level, i.e. the lowest line in Figure 9.

### Validation

All patients tested were confirmed positive by smear tests and by growing cultures of TB bacteria from samples taken from each patient.

## Claims

1. A recombinant fusion protein comprising:
(a) at least one antigenic region comprising an amino acid sequence with at least 80% sequence identity to any of SEQ. ID NOS. 1 to 3, 8, 4, 7, 6 or 5, or an antigenic fragment thereof; and
(b) at least one anchoring region comprising a sequence of at least 25 amino acids.

2. A recombinant fusion protein according to claim 1, wherein the at least one antigenic region comprises a plurality of different antigenic regions having a different sequence selected from the following (a) to (h):
(a) a sequence with at least 80% sequence identity to SEQ. ID NO. 1 or an antigenic fragment thereof;
(b) a sequence with at least 80% sequence identity to SEQ. ID NO. 2 or an antigenic fragment thereof;
(c) a sequence with at least 80% sequence identity to SEQ. ID NO. 3 or an antigenic fragment thereof;
(d) a sequence with at least 80% sequence identity to SEQ. ID NO. 8 or an antigenic fragment thereof;
(e) a sequence with at least 80% sequence identity to SEQ. ID NO. 4 or an antigenic fragment thereof;
(f) a sequence with at least 80% sequence identity to SEQ. ID NO. 7 or an antigenic fragment thereof;
(g) a sequence with at least 80% sequence identity to SEQ. ID NO. 6 or an antigenic fragment thereof; and
(h) a sequence with at least 80% sequence identity to SEQ. ID NO. 5 or an antigenic fragment thereof.

3. A recombinant fusion protein according to claim 1 or 2 wherein at least one antigenic region is N-terminal to the at least one anchoring.

4. A recombinant fusion protein according to claim 1 or 2 wherein at least one antigenic region is C-terminal to the at least one anchoring region.

5. A recombinant fusion protein according to any one of the preceding claims wherein the fusion protein is glycosylated.

6. A recombinant fusion protein according to any one of the preceding claims wherein the at least one anchoring region at least constant domains 2 and 3 and a hinge region of an immunoglobulin Fc region, preferably a murine IgG2a Fc region or wherein the at least one anchoring region comprises an entire IgG antibody, preferably a murine IgG2a antibody.

7. An antigenic region comprising an amino acid sequence with at least 80% sequence identity to any of SEQ. ID NOS. 1 to 3, 8, 4, 7, 6 or 5, or an antigenic fragment thereof, wherein the antigenic region is glycosylated.

8. A nucleic acid molecule comprising a nucleotide sequence encoding a recombinant fusion protein according to any one of claims 1 to 6 or the antigenic region according to claim 7.

9. A vector comprising a nucleic acid molecule according to claim 8.

10. A host cell comprising a nucleic acid molecule according to claim 8 or a vector according to claim 9 and capable of expressing a recombinant fusion protein according to any one of claims 1 to 6, or the antigenic region according to claim 7.

11. A kit for the detection of active TB in a patient comprising at least two different peptides selected from the following (a) to (h):
(a) a sequence with at least 80% sequence identity to SEQ. ID NO. 1 or an antigenic fragment thereof;
(b) a sequence with at least 80% sequence identity to SEQ. ID NO. 2 or an antigenic fragment thereof;
(c) a sequence with at least 80% sequence identity to SEQ. ID NO. 3 or an antigenic fragment thereof;
(d) a sequence with at least 80% sequence identity to SEQ. ID NO. 8 or an antigenic fragment thereof;
(e) a sequence with at least 80% sequence identity to SEQ. ID NO. 4 or an antigenic fragment thereof;
(f) a sequence with at least 80% sequence identity to SEQ. ID NO. 7 or an antigenic fragment thereof;
(g) a sequence with at least 80% sequence identity to SEQ. ID NO. 6 or an antigenic fragment thereof; and
(h) a sequence with at least 80% sequence identity to SEQ, ID NO. 5 or an antigenic fragment thereof.

12. A kit for the detection of active TB in a patient comprising:
(a) a solid phase; and
(b) at least one peptide bound to the solid phase wherein the peptide comprises an amino acid sequence with at least 80% sequence identity to a sequence selected from any of SEQ. ID NOS. 1 to 3, 8, 4, 7, 6 or 5, or an antigenic fragment thereof.

13. A method of detecting active TB in a patient comprising the steps of:
(a) providing at least one peptide comprising an amino acid sequence with at least 80% sequence identity to a sequence selected from SEQ. ID NOS. 1 to 3, 8, 4, 7, 6 and 5, or an antigenic fragment thereof;
(b) contacting the at least one peptide with a biological sample obtained from the patient; and
(c) detecting the binding of the at least one peptide with an antibody in the biological sample wherein the presence of an antibody binding to the peptide is indicative of active TB in the patient.

14. A method according to claim 13, wherein the at least one peptide is a plurality of peptides, each comprising a different sequence selected from the following (a) to (e):
(a) a sequence with at least 80% sequence identity to SEQ. ID NO. 1 or an antigenic fragment thereof;
(b) a sequence with at least 80% sequence identity to SEQ. ID NO. 2 or an antigenic fragment thereof;
(c) a sequence with at least 80% sequence identity to SEQ. ID NO. 3 or an antigenic fragment thereof;
(d) a sequence with at least 80% sequence identity to SEQ. ID NO. 8 or an antigenic fragment thereof;
(e) a sequence with at least 80% sequence identity to SEQ. ID NO. 4 or an antigenic fragment thereof;
(f) a sequence with at least 80% sequence identity to SEQ. ID NO. 7 or an antigenic fragment thereof;
(g) a sequence with at least 80% sequence identity to SEQ. ID NO. 6 or an antigenic fragment thereof; and
(h) a sequence with at least 80% sequence identity to SEQ. ID NO. 5 or an antigenic fragment thereof.

15. Use of a peptide as a biological marker for the presence of active TB in a patient, wherein the peptide comprises an amino acid sequence with at least 80% sequence identity to a sequence selected from any of SEQ. ID NOS. 1 to 3, 8, 4, 7, 6 or 5, or an antigenic fragment thereof.


SEQUENCE LISTING

<110>  Norwegian Institute of Public Health

<120>  A Recombinant Fusion Protein

<130>  PN800628EP

<160>  28

<170>  PatentIn version 3.5

<210>  1
<211>  460
<212>  PRT
<213>  Mycobacterium tuberculosis

<400>  1

Met Thr Gln Ser Gln Thr Val Thr Val Asp Gln Gln Glu Ile Leu Asn
1               5                   10                  15

Arg Ala Asn Glu Val Glu Ala Pro Met Ala Asp Pro Pro Thr Asp Val
            20                  25                  30

Pro Ile Thr Pro Cys Glu Leu Thr Ala Ala Lys Asn Ala Ala Gln Gln
        35                  40                  45

Leu Val Leu Ser Ala Asp Asn Met Arg Glu Tyr Leu Ala Ala Gly Ala
    50                  55                  60

Lys Glu Arg Gln Arg Leu Ala Thr Ser Leu Arg Asn Ala Ala Lys Ala
65                  70                  75                  80

Tyr Gly Glu Val Asp Glu Glu Ala Ala Thr Ala Leu Asp Asn Asp Gly
                85                  90                  95

Glu Gly Thr Val Gln Ala Glu Ser Ala Gly Ala Val Gly Gly Asp Ser
            100                 105                 110

Ser Ala Glu Leu Thr Asp Thr Pro Arg Val Ala Thr Ala Gly Glu Pro
        115                 120                 125

Asn Phe Met Asp Leu Lys Glu Ala Ala Arg Lys Leu Glu Thr Gly Asp
    130                 135                 140

Gln Gly Ala Ser Leu Ala His Phe Ala Asp Gly Trp Asn Thr Phe Asn
145                 150                 155                 160

Leu Thr Leu Gln Gly Asp Val Lys Arg Phe Arg Gly Phe Asp Asn Trp

                165                 170                 175

Glu Gly Asp Ala Ala Thr Ala Cys Glu Ala Ser Leu Asp Gln Gln Arg
            180                 185                 190

Gln Trp Ile Leu His Met Ala Lys Leu Ser Ala Ala Met Ala Lys Gln
        195                 200                 205

Ala Gln Tyr Val Ala Gln Leu His Val Trp Ala Arg Arg Glu His Pro
    210                 215                 220

Thr Tyr Glu Asp Ile Val Gly Leu Glu Arg Leu Tyr Ala Glu Asn Pro
225                 230                 235                 240

Ser Ala Arg Asp Gln Ile Leu Pro Val Tyr Ala Glu Tyr Gln Gln Arg
                245                 250                 255

Ser Glu Lys Val Leu Thr Glu Tyr Asn Asn Lys Ala Ala Leu Glu Pro
            260                 265                 270

Val Asn Pro Pro Lys Pro Pro Pro Ala Ile Lys Ile Asp Pro Pro Pro
        275                 280                 285

Pro Pro Gln Glu Gln Gly Leu Ile Pro Gly Phe Leu Met Pro Pro Ser
    290                 295                 300

Asp Gly Ser Gly Val Thr Pro Gly Thr Gly Met Pro Ala Ala Pro Met
305                 310                 315                 320

Val Pro Pro Thr Gly Ser Pro Gly Gly Gly Leu Pro Ala Asp Thr Ala
                325                 330                 335

Ala Gln Leu Thr Ser Ala Gly Arg Glu Ala Ala Ala Leu Ser Gly Asp
            340                 345                 350

Val Ala Val Lys Ala Ala Ser Leu Gly Gly Gly Gly Gly Gly Gly Val
        355                 360                 365

Pro Ser Ala Pro Leu Gly Ser Ala Ile Gly Gly Ala Glu Ser Val Arg
    370                 375                 380

Pro Ala Gly Ala Gly Asp Ile Ala Gly Leu Gly Gln Gly Arg Ala Gly
385                 390                 395                 400

Gly Gly Ala Ala Leu Gly Gly Gly Gly Met Gly Met Pro Met Gly Ala

405                 410                 415

Ala His Gln Gly Gln Gly Gly Ala Lys Ser Lys Gly Ser Gln Gln Glu
            420                 425                 430

Asp Glu Ala Leu Tyr Thr Glu Asp Arg Ala Trp Thr Glu Ala Val Ile
        435                 440                 445

Gly Asn Arg Arg Arg Gln Asp Ser Lys Glu Ser Lys
    450                 455                 460

<210>  2
<211>  374
<212>  PRT
<213>  Mycobacterium tuberculosis

<400>  2

Met Lys Ile Arg Leu His Thr Leu Leu Ala Val Leu Thr Ala Ala Pro
1               5                   10                  15

Leu Leu Leu Ala Ala Ala Gly Cys Gly Ser Lys Pro Pro Ser Gly Ser
            20                  25                  30

Pro Glu Thr Gly Ala Gly Ala Gly Thr Val Ala Thr Thr Pro Ala Ser
        35                  40                  45

Ser Pro Val Thr Leu Ala Glu Thr Gly Ser Thr Leu Leu Tyr Pro Leu
    50                  55                  60

Phe Asn Leu Trp Gly Pro Ala Phe His Glu Arg Tyr Pro Asn Val Thr
65                  70                  75                  80

Ile Thr Ala Gln Gly Thr Gly Ser Gly Ala Gly Ile Ala Gln Ala Ala
                85                  90                  95

Ala Gly Thr Val Asn Ile Gly Ala Ser Asp Ala Tyr Leu Ser Glu Gly
            100                 105                 110

Asp Met Ala Ala His Lys Gly Leu Met Asn Ile Ala Leu Ala Ile Ser
        115                 120                 125

Ala Gln Gln Val Asn Tyr Asn Leu Pro Gly Val Ser Glu His Leu Lys
    130                 135                 140

Leu Asn Gly Lys Val Leu Ala Ala Met Tyr Gln Gly Thr Ile Lys Thr
145                 150                 155                 160

Trp Asp Asp Pro Gln Ile Ala Ala Leu Asn Pro Gly Val Asn Leu Pro
                165                 170                 175

Gly Thr Ala Val Val Pro Leu His Arg Ser Asp Gly Ser Gly Asp Thr
            180                 185                 190

Phe Leu Phe Thr Gln Tyr Leu Ser Lys Gln Asp Pro Glu Gly Trp Gly
        195                 200                 205

Lys Ser Pro Gly Phe Gly Thr Thr Val Asp Phe Pro Ala Val Pro Gly
    210                 215                 220

Ala Leu Gly Glu Asn Gly Asn Gly Gly Met Val Thr Gly Cys Ala Glu
225                 230                 235                 240

Thr Pro Gly Cys Val Ala Tyr Ile Gly Ile Ser Phe Leu Asp Gln Ala
                245                 250                 255

Ser Gln Arg Gly Leu Gly Glu Ala Gln Leu Gly Asn Ser Ser Gly Asn
            260                 265                 270

Phe Leu Leu Pro Asp Ala Gln Ser Ile Gln Ala Ala Ala Ala Gly Phe
        275                 280                 285

Ala Ser Lys Thr Pro Ala Asn Gln Ala Ile Ser Met Ile Asp Gly Pro
    290                 295                 300

Ala Pro Asp Gly Tyr Pro Ile Ile Asn Tyr Glu Tyr Ala Ile Val Asn
305                 310                 315                 320

Asn Arg Gln Lys Asp Ala Ala Thr Ala Gln Thr Leu Gln Ala Phe Leu
                325                 330                 335

His Trp Ala Ile Thr Asp Gly Asn Lys Ala Ser Phe Leu Asp Gln Val
            340                 345                 350

His Phe Gln Pro Leu Pro Pro Ala Val Val Lys Leu Ser Asp Ala Leu
        355                 360                 365

Ile Ala Thr Ile Ser Ser
    370

<210> 3
<211> 325

<212>  PRT
<213>  Mycobacterium tuberculosis

<400>  3

Met Thr Asp Val Ser Arg Lys Ile Arg Ala Trp Gly Arg Arg Leu Met
1               5                   10                  15

Ile Gly Thr Ala Ala Ala Val Val Leu Pro Gly Leu Val Gly Leu Ala
            20                  25                  30

Gly Gly Ala Ala Thr Ala Gly Ala Phe Ser Arg Pro Gly Leu Pro Val
        35                  40                  45

Glu Tyr Leu Gln Val Pro Ser Pro Ser Met Gly Arg Asp Ile Lys Val
    50                  55                  60

Gln Phe Gln Ser Gly Gly Asn Asn Ser Pro Ala Val Tyr Leu Leu Asp
65                  70                  75                  80

Gly Leu Arg Ala Gln Asp Asp Tyr Asn Gly Trp Asp Ile Asn Thr Pro
                85                  90                  95

Ala Phe Glu Trp Tyr Tyr Gln Ser Gly Leu Ser Ile Val Met Pro Val
            100                 105                 110

Gly Gly Gln Ser Ser Phe Tyr Ser Asp Trp Tyr Ser Pro Ala Cys Gly
        115                 120                 125

Lys Ala Gly Cys Gln Thr Tyr Lys Trp Glu Thr Phe Leu Thr Ser Glu
    130                 135                 140

Leu Pro Gln Trp Leu Ser Ala Asn Arg Ala Val Lys Pro Thr Gly Ser
145                 150                 155                 160

Ala Ala Ile Gly Leu Ser Met Ala Gly Ser Ser Ala Met Ile Leu Ala
                165                 170                 175

Ala Tyr His Pro Gln Gln Phe Ile Tyr Ala Gly Ser Leu Ser Ala Leu
            180                 185                 190

Leu Asp Pro Ser Gln Gly Met Gly Pro Ser Leu Ile Gly Leu Ala Met
        195                 200                 205

Gly Asp Ala Gly Gly Tyr Lys Ala Ala Asp Met Trp Gly Pro Ser Ser
    210                 215                 220

Asp Pro Ala Trp Glu Arg Asn Asp Pro Thr Gln Gln Ile Pro Lys Leu
225                 230                 235                 240

Val Ala Asn Asn Thr Arg Leu Trp Val Tyr Cys Gly Asn Gly Thr Pro
                245                 250                 255

Asn Glu Leu Gly Gly Ala Asn Ile Pro Ala Glu Phe Leu Glu Asn Phe
            260                 265                 270

Val Arg Ser Ser Asn Leu Lys Phe Gln Asp Ala Tyr Asn Ala Ala Gly
        275                 280                 285

Gly His Asn Ala Val Phe Asn Phe Pro Pro Asn Gly Thr His Ser Trp
    290                 295                 300

Glu Tyr Trp Gly Ala Gln Leu Asn Ala Met Lys Gly Asp Leu Gln Ser
305                 310                 315                 320

Ser Leu Gly Ala Gly
                325

<210>  4
<211>  884
<212>  PRT
<213>  Mycobacterium tuberculosis

<400>  4

Ala Ala Ala Ala Asn Thr Thr Ser Leu Leu Ala Ala Gly Ala Asp Glu
1               5                   10                  15

Ile Ser Ala Ala Ile Ala Ala Leu Phe Gly Ala His Gly Arg Ala Tyr
            20                  25                  30

Gln Ala Ala Ser Ala Glu Ala Ala Ala Phe His Gly Arg Phe Val Gln
        35                  40                  45

Ala Leu Thr Thr Gly Gly Gly Ala Tyr Ala Ala Ala Glu Ala Ala Ala
    50                  55                  60

Val Thr Pro Leu Leu Asn Ser Ile Asn Ala Pro Val Leu Ala Ala Thr
65                  70                  75                  80

Gly Arg Pro Leu Ile Gly Asn Gly Ala Asn Gly Ala Pro Gly Thr Gly
                85                  90                  95

Ala Asn Gly Gly Asp Ala Gly Trp Leu Ile Gly Asn Gly Gly Ala Gly
            100                 105                 110

Gly Ser Gly Ala Lys Gly Ala Asn Gly Gly Ala Gly Gly Pro Gly Gly
        115                 120                 125

Ala Ala Gly Leu Phe Gly Asn Gly Gly Ala Gly Gly Ala Gly Gly Thr
    130                 135                 140

Ala Thr Ala Asn Asn Gly Ile Gly Gly Ala Gly Gly Ala Gly Gly Ser
145                 150                 155                 160

Ala Met Leu Phe Gly Ala Gly Gly Ala Gly Gly Ala Gly Gly Ala Ala
                165                 170                 175

Thr Ser Leu Val Gly Gly Ile Gly Gly Thr Gly Gly Thr Gly Gly Asn
            180                 185                 190

Ala Gly Met Leu Ala Gly Ala Ala Gly Ala Gly Gly Ala Gly Gly Phe
        195                 200                 205

Ser Phe Ser Thr Ala Gly Gly Ala Gly Gly Ala Gly Gly Ala Gly Gly
    210                 215                 220

Leu Phe Thr Thr Gly Gly Val Gly Gly Ala Gly Gly Gln Gly His Thr
225                 230                 235                 240

Gly Gly Ala Gly Gly Ala Gly Gly Ala Gly Gly Leu Phe Gly Ala Gly
                245                 250                 255

Gly Met Gly Gly Ala Gly Gly Phe Gly Asp His Gly Thr Leu Gly Thr
            260                 265                 270

Gly Gly Ala Gly Gly Asp Gly Gly Gly Gly Gly Leu Phe Gly Ala Gly
        275                 280                 285

Gly Asp Gly Gly Ala Gly Gly Ser Gly Leu Thr Thr Gly Gly Ala Ala
    290                 295                 300

Gly Asn Gly Gly Asn Ala Gly Thr Leu Ser Leu Gly Ala Ala Gly Gly
305                 310                 315                 320

Ala Gly Gly Thr Gly Gly Ala Gly Gly Thr Val Phe Gly Gly Gly Lys
                325                 330                 335

Gly Gly Ala Gly Gly Ala Gly Gly Asn Ala Gly Met Leu Phe Gly Ser
            340                 345                 350

Gly Gly Gly Gly Gly Thr Gly Gly Phe Gly Phe Ala Ala Gly Gly Gln
        355                 360                 365

Gly Gly Val Gly Gly Ser Ala Gly Met Leu Ser Gly Ser Gly Gly Ser
    370                 375                 380

Gly Gly Ala Gly Gly Ser Gly Gly Pro Ala Gly Thr Ala Ala Gly Gly
385                 390                 395                 400

Ala Gly Gly Ala Gly Gly Ala Pro Gly Leu Ile Gly Asn Gly Gly Asn
                405                 410                 415

Gly Gly Asn Gly Gly Glu Ser Gly Gly Thr Gly Gly Val Gly Gly Ala
            420                 425                 430

Gly Gly Asn Ala Val Leu Ile Gly Asn Gly Gly Glu Gly Gly Ile Gly
        435                 440                 445

Ala Leu Ala Gly Lys Ser Gly Phe Gly Gly Phe Gly Gly Leu Leu Leu
    450                 455                 460

Gly Ala Asp Gly Tyr Asn Ala Pro Glu Ser Thr Ser Pro Trp His Asn
465                 470                 475                 480

Leu Gln Gln Asp Ile Leu Ser Phe Ile Asn Glu Pro Thr Glu Ala Leu
                485                 490                 495

Thr Gly Arg Pro Leu Ile Gly Asn Gly Asp Ser Gly Thr Pro Gly Thr
            500                 505                 510

Gly Asp Asp Gly Gly Ala Gly Gly Trp Leu Phe Gly Asn Gly Gly Asn
        515                 520                 525

Gly Gly Ala Gly Ala Ala Gly Thr Asn Gly Ser Ala Gly Gly Ala Gly
    530                 535                 540

Gly Ala Gly Gly Ile Leu Phe Gly Thr Gly Gly Ala Gly Gly Ala Gly
545                 550                 555                 560

Gly Val Gly Thr Ala Gly Ala Gly Gly Ala Gly Gly Ala Gly Gly Ser
                565                 570                 575

Ala Phe Leu Ile Gly Ser Gly Gly Thr Gly Gly Val Gly Gly Ala Ala
            580                 585                 590

Thr Thr Thr Gly Gly Val Gly Gly Ala Gly Gly Asn Ala Gly Leu Leu
        595                 600                 605

Ile Gly Ala Ala Gly Leu Gly Gly Cys Gly Gly Gly Ala Phe Thr Ala
    610                 615                 620

Gly Val Thr Thr Gly Gly Ala Gly Gly Thr Gly Gly Ala Ala Gly Leu
625                 630                 635                 640

Phe Ala Asn Gly Gly Ala Gly Gly Ala Gly Gly Thr Gly Ser Thr Ala
                645                 650                 655

Gly Gly Ala Gly Gly Ala Gly Gly Ala Gly Gly Leu Tyr Ala His Gly
            660                 665                 670

Gly Thr Gly Gly Pro Gly Gly Asn Gly Gly Ser Thr Gly Ala Gly Gly
        675                 680                 685

Thr Gly Gly Ala Gly Gly Pro Gly Gly Leu Tyr Gly Ala Gly Gly Ser
    690                 695                 700

Gly Gly Ala Gly Gly His Gly Gly Met Ala Gly Gly Gly Gly Gly Val
705                 710                 715                 720

Gly Gly Asn Ala Gly Ser Leu Thr Leu Asn Ala Ser Gly Gly Ala Gly
                725                 730                 735

Gly Ser Gly Gly Ser Ser Leu Ser Gly Lys Ala Gly Ala Gly Gly Ala
            740                 745                 750

Gly Gly Ser Ala Gly Leu Phe Tyr Gly Ser Gly Gly Ala Gly Gly Asn
        755                 760                 765

Gly Gly Tyr Ser Leu Asn Gly Thr Gly Gly Asp Gly Gly Thr Gly Gly
    770                 775                 780

Ala Gly Gln Ile Thr Gly Leu Arg Ser Gly Phe Gly Gly Ala Gly Gly
785                 790                 795                 800

Ala Gly Gly Ala Ser Asp Thr Gly Ala Gly Gly Asn Gly Gly Ala Gly
                805                 810                 815

Gly Lys Ala Gly Leu Tyr Gly Asn Gly Gly Asp Gly Gly Ala Gly Gly
            820                 825                 830

Asp Gly Ala Thr Ser Gly Lys Gly Gly Ala Gly Gly Asn Ala Val Val
        835                 840                 845

Ile Gly Asn Gly Gly Asn Gly Gly Asn Ala Gly Lys Ala Gly Gly Thr
    850                 855                 860

Ala Gly Ala Gly Gly Ala Gly Gly Leu Val Leu Gly Arg Asp Gly Gln
865                 870                 875                 880

His Gly Leu Thr

<210>  5
<211>  227
<212>  PRT
<213>  Mycobacterium tuberculosis

<400>  5

Met Arg Ile Lys Ile Phe Met Leu Val Thr Ala Val Val Leu Leu Cys
1               5                   10                  15

Cys Ser Gly Val Ala Thr Ala Ala Pro Lys Thr Tyr Cys Glu Glu Leu
            20                  25                  30

Lys Gly Thr Asp Thr Gly Gln Ala Cys Gln Ile Gln Met Ser Asp Pro
        35                  40                  45

Ala Tyr Asn Ile Asn Ile Ser Leu Pro Ser Tyr Tyr Pro Asp Gln Lys
    50                  55                  60

Ser Leu Glu Asn Tyr Ile Ala Gln Thr Arg Asp Lys Phe Leu Ser Ala
65                  70                  75                  80

Ala Thr Ser Ser Thr Pro Arg Glu Ala Pro Tyr Glu Tyr Ile Thr Ser
                85                  90                  95

Ala Thr Tyr Gln Ser Ala Ile Pro Pro Arg Gly Thr Gln Ala Val Val
            100                 105                 110

Leu Lys Val Tyr Gln Asn Ala Gly Gly Thr His Pro Thr Thr Thr Tyr
        115                 120                 125

Lys Ala Phe Asp Trp Asp Gln Ala Tyr Arg Lys Pro Ile Thr Tyr Asp

130                 135                 140

Thr Leu Trp Gln Ala Asp Thr Asp Pro Leu Pro Val Val Phe Pro Ile
145                 150                 155                 160

Val Gln Gly Glu Leu Ser Lys Gln Thr Gly Gln Gln Val Ser Ile Ala
                165                 170                 175

Pro Asn Ala Gly Leu Asp Pro Val Asn Tyr Gln Asn Phe Ala Val Thr
            180                 185                 190

Asn Asp Gly Val Ile Phe Phe Phe Asn Pro Gly Glu Leu Leu Pro Glu
        195                 200                 205

Ala Ala Gly Pro Thr Gln Val Leu Val Pro Arg Ser Ala Ile Asp Ser
    210                 215                 220

Met Leu Ala
225

<210>  6
<211>  325
<212>  PRT
<213>  Mycobacterium tuberculosis

<400>  6

Met His Gln Val Asp Pro Asn Leu Thr Arg Arg Lys Gly Arg Leu Ala
1               5                   10                  15

Ala Leu Ala Ile Ala Ala Met Ala Ser Ala Ser Leu Val Thr Val Ala
            20                  25                  30

Val Pro Ala Thr Ala Asn Ala Asp Pro Glu Pro Ala Pro Pro Val Pro
        35                  40                  45

Thr Thr Ala Ala Ser Pro Pro Ser Thr Ala Ala Ala Pro Pro Ala Pro
    50                  55                  60

Ala Thr Pro Val Ala Pro Pro Pro Pro Ala Ala Ala Asn Thr Pro Asn
65                  70                  75                  80

Ala Gln Pro Gly Asp Pro Asn Ala Ala Pro Pro Pro Ala Asp Pro Asn
                85                  90                  95

Ala Pro Pro Pro Pro Val Ile Ala Pro Asn Ala Pro Gln Pro Val Arg
            100                 105                 110

Ile Asp Asn Pro Val Gly Gly Phe Ser Phe Ala Leu Pro Ala Gly Trp
        115                 120                 125

Val Glu Ser Asp Ala Ala His Phe Asp Tyr Gly Ser Ala Leu Leu Ser
    130                 135                 140

Lys Thr Thr Gly Asp Pro Pro Phe Pro Gly Gln Pro Pro Pro Val Ala
145                 150                 155                 160

Asn Asp Thr Arg Ile Val Leu Gly Arg Leu Asp Gln Lys Leu Tyr Ala
                165                 170                 175

Ser Ala Glu Ala Thr Asp Ser Lys Ala Ala Ala Arg Leu Gly Ser Asp
            180                 185                 190

Met Gly Glu Phe Tyr Met Pro Tyr Pro Gly Thr Arg Ile Asn Gln Glu
        195                 200                 205

Thr Val Ser Leu Asp Ala Asn Gly Val Ser Gly Ser Ala Ser Tyr Tyr
    210                 215                 220

Glu Val Lys Phe Ser Asp Pro Ser Lys Pro Asn Gly Gln Ile Trp Thr
225                 230                 235                 240

Gly Val Ile Gly Ser Pro Ala Ala Asn Ala Pro Asp Ala Gly Pro Pro
                245                 250                 255

Gln Arg Trp Phe Val Val Trp Leu Gly Thr Ala Asn Asn Pro Val Asp
            260                 265                 270

Lys Gly Ala Ala Lys Ala Leu Ala Glu Ser Ile Arg Pro Leu Val Ala
        275                 280                 285

Pro Pro Pro Ala Pro Ala Pro Ala Pro Ala Glu Pro Ala Pro Ala Pro
    290                 295                 300

Ala Pro Ala Gly Glu Val Ala Pro Thr Pro Thr Thr Pro Thr Pro Gln
305                 310                 315                 320

Arg Thr Leu Pro Ala
                325

<210>  7
<211>  100

<212>  PRT
<213>  Mycobacterium tuberculosis

<400>  7

Met Ala Glu Met Lys Thr Asp Ala Ala Thr Leu Ala Gln Glu Ala Gly
1               5                   10                  15

Asn Phe Glu Arg Ile Ser Gly Asp Leu Lys Thr Gln Ile Asp Gln Val
            20                  25                  30

Glu Ser Thr Ala Gly Ser Leu Gln Gly Gln Trp Arg Gly Ala Ala Gly
        35                  40                  45

Thr Ala Ala Gln Ala Ala Val Val Arg Phe Gln Glu Ala Ala Asn Lys
    50                  55                  60

Gln Lys Gln Glu Leu Asp Glu Ile Ser Thr Asn Ile Arg Gln Ala Gly
65                  70                  75                  80

Val Gln Tyr Ser Arg Ala Asp Glu Glu Gln Gln Gln Ala Leu Ser Ser
                85                  90                  95

Gln Met Gly Phe
            100


<210>  8
<211>  96
<212>  PRT
<213>  Mycobacterium tuberculosis

<400>  8

Asn Met Thr Glu Gln Gln Trp Asn Phe Ala Gly Ile Glu Ala Ala Ala
1               5                   10                  15

Ser Ala Ile Gln Gly Asn Val Thr Ser Ile His Ser Leu Leu Asp Glu
            20                  25                  30

Gly Lys Gln Ser Leu Thr Lys Leu Ala Ala Ala Trp Gly Gly Ser Gly
        35                  40                  45

Ser Glu Ala Tyr Gln Gly Val Gln Gln Lys Trp Asp Ala Thr Ala Thr
    50                  55                  60

Glu Leu Asn Asn Ala Leu Gln Asn Leu Ala Arg Thr Ile Ser Glu Ala
65                  70                  75                  80

Gly Gln Ala Met Ala Ser Thr Glu Gly Asn Val Thr Gly Met Phe Ala
                85                  90                  95

<210>  9
<211>  1418
<212>  DNA
<213>  Artificial

<220>
<223>  Sequence from Mycobacterium tuberculosis artificially optimised
       for expression in human cells.

<220>
<221>  CDS
<222>  (20)..(1399)

<400>  9
atcgaccgtc tccacaggt atg aca cag agc cag acc gtg aca gtg gac cag      52
                     Met Thr Gln Ser Gln Thr Val Thr Val Asp Gln
                     1               5                   10

cag gag atc ctg aac cgg gcc aat gag gtg gaa gct ccc atg gcc gac      100
Gln Glu Ile Leu Asn Arg Ala Asn Glu Val Glu Ala Pro Met Ala Asp
            15                  20                  25

cct ccc act gat gtg cct att acc cca tgc gaa ctg aca gcc gct aag      148
Pro Pro Thr Asp Val Pro Ile Thr Pro Cys Glu Leu Thr Ala Ala Lys
        30                  35                  40

aac gcc gct cag cag ctg gtg ctg tct gcc gac aat atg aga gag tac      196
Asn Ala Ala Gln Gln Leu Val Leu Ser Ala Asp Asn Met Arg Glu Tyr
    45                  50                  55

ctg gct gct gga gct aag gaa aga cag agg ctg gct acc agt ctg cgg      244
Leu Ala Ala Gly Ala Lys Glu Arg Gln Arg Leu Ala Thr Ser Leu Arg
60                  65                  70                  75

aac gcc gct aaa gcc tat ggg gag gtg gat gag gaa gcc gct acc gcc      292
Asn Ala Ala Lys Ala Tyr Gly Glu Val Asp Glu Glu Ala Ala Thr Ala
                80                  85                  90

ctg gac aat gat gga gag ggc aca gtg cag gcc gaa agc gcc gga gct      340
Leu Asp Asn Asp Gly Glu Gly Thr Val Gln Ala Glu Ser Ala Gly Ala
            95                  100                 105

gtg ggc ggg gac agc tcc gcc gag ctg act gat acc ccc aga gtg gct      388
Val Gly Gly Asp Ser Ser Ala Glu Leu Thr Asp Thr Pro Arg Val Ala
        110                 115                 120

act gcc ggc gaa cct aac ttc atg gat ctg aag gag gcc gct agg aaa      436
Thr Ala Gly Glu Pro Asn Phe Met Asp Leu Lys Glu Ala Ala Arg Lys
    125                 130                 135

ctg gaa acc ggg gac cag gga gct agc ctg gct cac ttc gct gat ggg      484
Leu Glu Thr Gly Asp Gln Gly Ala Ser Leu Ala His Phe Ala Asp Gly
140                 145                 150                 155

tgg aac acc ttt aat ctg aca ctg cag ggc gac gtg aaa cgc ttc cgg      532

Trp Asn Thr Phe Asn Leu Thr Leu Gln Gly Asp Val Lys Arg Phe Arg
                160                 165                 170

ggg ttt gac aat tgg gag gga gat gcc gct acc gcc tgt gaa gct agt      580
Gly Phe Asp Asn Trp Glu Gly Asp Ala Ala Thr Ala Cys Glu Ala Ser
            175                 180                 185

ctg gat cag cag aga cag tgg atc ctg cat atg gcc aag ctg tca gcc      628
Leu Asp Gln Gln Arg Gln Trp Ile Leu His Met Ala Lys Leu Ser Ala
        190                 195                 200

gct atg gcc aaa cag gct cag tat gtg gct cag ctg cac gtg tgg gct      676
Ala Met Ala Lys Gln Ala Gln Tyr Val Ala Gln Leu His Val Trp Ala
    205                 210                 215

cgg aga gag cat cca aca tac gaa gac atc gtg ggc ctg gag aga ctg      724
Arg Arg Glu His Pro Thr Tyr Glu Asp Ile Val Gly Leu Glu Arg Leu
220                 225                 230                 235

tat gcc gaa aac cca agc gcc agg gat cag att ctg ccc gtg tac gcc      772
Tyr Ala Glu Asn Pro Ser Ala Arg Asp Gln Ile Leu Pro Val Tyr Ala
                240                 245                 250

gag tat cag cag agg tcc gag aag gtg ctg act gaa tat aac aac aag      820
Glu Tyr Gln Gln Arg Ser Glu Lys Val Leu Thr Glu Tyr Asn Asn Lys
            255                 260                 265

gcc gct ctg gaa cct gtg aat cca ccc aag cct cca ccc gcc atc aaa      868
Ala Ala Leu Glu Pro Val Asn Pro Pro Lys Pro Pro Pro Ala Ile Lys
        270                 275                 280

att gac cct cca ccc cct cca cag gag cag gga ctg att cct ggg ttt      916
Ile Asp Pro Pro Pro Pro Pro Gln Glu Gln Gly Leu Ile Pro Gly Phe
    285                 290                 295

ctg atg ccc cct tct gat ggg agt gga gtg acc cca gga act ggg atg      964
Leu Met Pro Pro Ser Asp Gly Ser Gly Val Thr Pro Gly Thr Gly Met
300                 305                 310                 315

cct gct gct cct atg gtg cca ccc aca gga tcc cct gga ggc ggg ctg      1012
Pro Ala Ala Pro Met Val Pro Pro Thr Gly Ser Pro Gly Gly Gly Leu
                320                 325                 330

cca gct gac act gcc gct cag ctg acc agc gcc gga aga gag gct gct      1060
Pro Ala Asp Thr Ala Ala Gln Leu Thr Ser Ala Gly Arg Glu Ala Ala
            335                 340                 345

gcc ctg tcc ggg gat gtg gct gtg aag gct gcc tct ctg gga ggc ggg      1108
Ala Leu Ser Gly Asp Val Ala Val Lys Ala Ala Ser Leu Gly Gly Gly
        350                 355                 360

gga ggc ggg gga gtg cca agc gcc cct ctg gga tca gcc att ggc ggg      1156
Gly Gly Gly Gly Val Pro Ser Ala Pro Leu Gly Ser Ala Ile Gly Gly
    365                 370                 375

gct gag tct gtg cgc cct gct gga gct ggc gac att gct ggg ctg gga      1204
Ala Glu Ser Val Arg Pro Ala Gly Ala Gly Asp Ile Ala Gly Leu Gly
380                 385                 390                 395

cag gga aga gct gga ggc ggg gct gcc ctg gga ggc ggg gga atg gga      1252

Gln Gly Arg Ala Gly Gly Gly Ala Ala Leu Gly Gly Gly Gly Met Gly
                400                 405                 410

atg cct atg gga gct gct cac cag gga cag ggc ggg gcc aag tca aaa      1300
Met Pro Met Gly Ala Ala His Gln Gly Gln Gly Gly Ala Lys Ser Lys
            415                 420                 425

ggc agc cag cag gag gac gaa gcc ctg tac aca gag gat cgg gcc tgg      1348
Gly Ser Gln Gln Glu Asp Glu Ala Leu Tyr Thr Glu Asp Arg Ala Trp
        430                 435                 440

act gaa gct gtg atc ggc aac agg cgc cgg cag gat tcc aag gag tct      1396
Thr Glu Ala Val Ile Gly Asn Arg Arg Arg Gln Asp Ser Lys Glu Ser
    445                 450                 455

aaa tgatgatgaa ttcttgggc                                             1418
Lys
460

<210>  10
<211>  460
<212>  PRT
<213>  Artificial

<220>
<223>  Synthetic Construct

<400>  10

Met Thr Gln Ser Gln Thr Val Thr Val Asp Gln Gln Glu Ile Leu Asn
1               5                   10                  15

Arg Ala Asn Glu Val Glu Ala Pro Met Ala Asp Pro Pro Thr Asp Val
            20                  25                  30

Pro Ile Thr Pro Cys Glu Leu Thr Ala Ala Lys Asn Ala Ala Gln Gln
        35                  40                  45

Leu Val Leu Ser Ala Asp Asn Met Arg Glu Tyr Leu Ala Ala Gly Ala
    50                  55                  60

Lys Glu Arg Gln Arg Leu Ala Thr Ser Leu Arg Asn Ala Ala Lys Ala
65                  70                  75                  80

Tyr Gly Glu Val Asp Glu Glu Ala Ala Thr Ala Leu Asp Asn Asp Gly
                85                  90                  95

Glu Gly Thr Val Gln Ala Glu Ser Ala Gly Ala Val Gly Gly Asp Ser
            100                 105                 110

Ser Ala Glu Leu Thr Asp Thr Pro Arg Val Ala Thr Ala Gly Glu Pro
        115                 120                 125

Asn Phe Met Asp Leu Lys Glu Ala Ala Arg Lys Leu Glu Thr Gly Asp
    130                 135                 140

Gln Gly Ala Ser Leu Ala His Phe Ala Asp Gly Trp Asn Thr Phe Asn
145                 150                 155                 160

Leu Thr Leu Gln Gly Asp Val Lys Arg Phe Arg Gly Phe Asp Asn Trp
                165                 170                 175

Glu Gly Asp Ala Ala Thr Ala Cys Glu Ala Ser Leu Asp Gln Gln Arg
            180                 185                 190

Gln Trp Ile Leu His Met Ala Lys Leu Ser Ala Ala Met Ala Lys Gln
        195                 200                 205

Ala Gln Tyr Val Ala Gln Leu His Val Trp Ala Arg Arg Glu His Pro
    210                 215                 220

Thr Tyr Glu Asp Ile Val Gly Leu Glu Arg Leu Tyr Ala Glu Asn Pro
225                 230                 235                 240

Ser Ala Arg Asp Gln Ile Leu Pro Val Tyr Ala Glu Tyr Gln Gln Arg
                245                 250                 255

Ser Glu Lys Val Leu Thr Glu Tyr Asn Asn Lys Ala Ala Leu Glu Pro
            260                 265                 270

Val Asn Pro Pro Lys Pro Pro Pro Ala Ile Lys Ile Asp Pro Pro Pro
        275                 280                 285

Pro Pro Gln Glu Gln Gly Leu Ile Pro Gly Phe Leu Met Pro Pro Ser
    290                 295                 300

Asp Gly Ser Gly Val Thr Pro Gly Thr Gly Met Pro Ala Ala Pro Met
305                 310                 315                 320

Val Pro Pro Thr Gly Ser Pro Gly Gly Gly Leu Pro Ala Asp Thr Ala
                325                 330                 335

Ala Gln Leu Thr Ser Ala Gly Arg Glu Ala Ala Ala Leu Ser Gly Asp
            340                 345                 350

Val Ala Val Lys Ala Ala Ser Leu Gly Gly Gly Gly Gly Gly Gly Val
        355                 360                 365

Pro Ser Ala Pro Leu Gly Ser Ala Ile Gly Gly Ala Glu Ser Val Arg
    370                 375                 380

Pro Ala Gly Ala Gly Asp Ile Ala Gly Leu Gly Gln Gly Arg Ala Gly
385                 390                 395                 400

Gly Gly Ala Ala Leu Gly Gly Gly Gly Met Gly Met Pro Met Gly Ala
                405                 410                 415

Ala His Gln Gly Gln Gly Gly Ala Lys Ser Lys Gly Ser Gln Gln Glu
            420                 425                 430

Asp Glu Ala Leu Tyr Thr Glu Asp Arg Ala Trp Thr Glu Ala Val Ile
        435                 440                 445

Gly Asn Arg Arg Arg Gln Asp Ser Lys Glu Ser Lys
    450                 455                 460

<210>  11
<211>  1160
<212>  DNA
<213>  artificial

<220>
<223>  Rv0934 sequence from Mycobacterium tuberculosis artificially
       optimised for expression in human cells

<220>
<221>  CDS
<222>  (20)..(1141)

<400>  11
atcgaccgtc tccacaggt atg aag atc agg ctg cac aca ctg ctg gcc gtg        52
                     Met Lys Ile Arg Leu His Thr Leu Leu Ala Val
                     1               5                   10

ctg aca gct gct cca ctg ctg ctg gct gct gct gga tgt gga agc aaa        100
Leu Thr Ala Ala Pro Leu Leu Leu Ala Ala Ala Gly Cys Gly Ser Lys
            15                  20                  25

ccc cct tct gga tct cca gag acc gga gct gga gct ggc aca gtg gcc        148
Pro Pro Ser Gly Ser Pro Glu Thr Gly Ala Gly Ala Gly Thr Val Ala
        30                  35                  40

acc aca cct gct agc tcc ccc gtg acc ctg gct gag acc gga tct aca        196
Thr Thr Pro Ala Ser Ser Pro Val Thr Leu Ala Glu Thr Gly Ser Thr
    45                  50                  55

ctg ctg tac ccc ctg ttc aac ctg tgg ggc cct gcc ttt cat gaa cgc        244
Leu Leu Tyr Pro Leu Phe Asn Leu Trp Gly Pro Ala Phe His Glu Arg
60                  65                  70                  75

tat cca aat gtg aca att aca gct cag gga act gga agt gga gct gga 292
Tyr Pro Asn Val Thr Ile Thr Ala Gln Gly Thr Gly Ser Gly Ala Gly
                80                  85                  90

att gct cag gct gct gct gga acc gtg aac att gga gct tct gat gct 340
Ile Ala Gln Ala Ala Ala Gly Thr Val Asn Ile Gly Ala Ser Asp Ala
            95                  100                 105

tac ctg tct gag ggc gat atg gcc gct cac aag ggg ctg atg aat atc 388
Tyr Leu Ser Glu Gly Asp Met Ala Ala His Lys Gly Leu Met Asn Ile
        110                 115                 120

gcc ctg gct att agt gcc cag cag gtg aac tac aat ctg ccc gga gtg 436
Ala Leu Ala Ile Ser Ala Gln Gln Val Asn Tyr Asn Leu Pro Gly Val
    125                 130                 135

tca gaa cat ctg aag ctg aac ggc aaa gtg ctg gcc gct atg tat cag 484
Ser Glu His Leu Lys Leu Asn Gly Lys Val Leu Ala Ala Met Tyr Gln
140                 145                 150                 155

ggg act atc aaa acc tgg gac gat cct cag att gcc gct ctg aac cct 532
Gly Thr Ile Lys Thr Trp Asp Asp Pro Gln Ile Ala Ala Leu Asn Pro
                160                 165                 170

gga gtg aat ctg cct gga aca gct gtg gtg cca ctg cac cgg agt gac 580
Gly Val Asn Leu Pro Gly Thr Ala Val Val Pro Leu His Arg Ser Asp
            175                 180                 185

gga tca ggc gat acc ttc ctg ttt aca cag tac ctg tcc aag cag gac 628
Gly Ser Gly Asp Thr Phe Leu Phe Thr Gln Tyr Leu Ser Lys Gln Asp
        190                 195                 200

ccc gaa ggg tgg gga aaa tct cct ggc ttc ggg act acc gtg gat ttt 676
Pro Glu Gly Trp Gly Lys Ser Pro Gly Phe Gly Thr Thr Val Asp Phe
    205                 210                 215

cct gct gtg cct ggg gct ctg gga gag aac ggc aat ggc gga atg gtg 724
Pro Ala Val Pro Gly Ala Leu Gly Glu Asn Gly Asn Gly Gly Met Val
220                 225                 230                 235

act gga tgt gct gaa acc cct gga tgc gtg gct tat atc gga att tct 772
Thr Gly Cys Ala Glu Thr Pro Gly Cys Val Ala Tyr Ile Gly Ile Ser
                240                 245                 250

ttc ctg gat cag gct agt cag aga gga ctg gga gag gct cag ctg ggg 820
Phe Leu Asp Gln Ala Ser Gln Arg Gly Leu Gly Glu Ala Gln Leu Gly
            255                 260                 265

aac tct agt gga aat ttc ctg ctg cct gat gct cag tcc atc cag gct 868
Asn Ser Ser Gly Asn Phe Leu Leu Pro Asp Ala Gln Ser Ile Gln Ala
        270                 275                 280

gct gct gct gga ttt gcc tca aag aca cca gcc aac cag gct atc agc 916
Ala Ala Ala Gly Phe Ala Ser Lys Thr Pro Ala Asn Gln Ala Ile Ser
    285                 290                 295

atg att gac ggg cct gcc cca gat gga tat ccc atc att aat tac gaa 964
Met Ile Asp Gly Pro Ala Pro Asp Gly Tyr Pro Ile Ile Asn Tyr Glu
300                 305                 310                 315

tat gcc atc gtg aac aat aga cag aaa gac gcc gct aca gcc cag act 1012
Tyr Ala Ile Val Asn Asn Arg Gln Lys Asp Ala Ala Thr Ala Gln Thr
                320                 325                 330

ctg cag gct ttt ctg cac tgg gcc att aca gac ggc aat aag gcc agc 1060
Leu Gln Ala Phe Leu His Trp Ala Ile Thr Asp Gly Asn Lys Ala Ser
            335                 340                 345

ttc ctg gat cag gtg cat ttt cag ccc ctg cca ccc gcc gtg gtg aaa 1108
Phe Leu Asp Gln Val His Phe Gln Pro Leu Pro Pro Ala Val Val Lys
        350                 355                 360

ctg tcc gat gcc ctg atc gct act att tca agc ggtgggggag acgatcagc 1160
Leu Ser Asp Ala Leu Ile Ala Thr Ile Ser Ser
    365                 370

<210> 12
<211> 374
<212> PRT
<213> artificial

<220>
<223> Synthetic Construct

<400> 12

Met Lys Ile Arg Leu His Thr Leu Leu Ala Val Leu Thr Ala Ala Pro
1               5                   10                  15

Leu Leu Leu Ala Ala Ala Gly Cys Gly Ser Lys Pro Pro Ser Gly Ser
            20                  25                  30

Pro Glu Thr Gly Ala Gly Ala Gly Thr Val Ala Thr Thr Pro Ala Ser
        35                  40                  45

Ser Pro Val Thr Leu Ala Glu Thr Gly Ser Thr Leu Leu Tyr Pro Leu
    50                  55                  60

Phe Asn Leu Trp Gly Pro Ala Phe His Glu Arg Tyr Pro Asn Val Thr
65                  70                  75                  80

Ile Thr Ala Gln Gly Thr Gly Ser Gly Ala Gly Ile Ala Gln Ala Ala
                85                  90                  95

Ala Gly Thr Val Asn Ile Gly Ala Ser Asp Ala Tyr Leu Ser Glu Gly
            100                 105                 110

Asp Met Ala Ala His Lys Gly Leu Met Asn Ile Ala Leu Ala Ile Ser
        115                 120                 125

Ala Gln Gln Val Asn Tyr Asn Leu Pro Gly Val Ser Glu His Leu Lys
    130                 135                 140

Leu Asn Gly Lys Val Leu Ala Ala Met Tyr Gln Gly Thr Ile Lys Thr
145                 150                 155                 160

Trp Asp Asp Pro Gln Ile Ala Ala Leu Asn Pro Gly Val Asn Leu Pro
                165                 170                 175

Gly Thr Ala Val Val Pro Leu His Arg Ser Asp Gly Ser Gly Asp Thr
            180                 185                 190

Phe Leu Phe Thr Gln Tyr Leu Ser Lys Gln Asp Pro Glu Gly Trp Gly
        195                 200                 205

Lys Ser Pro Gly Phe Gly Thr Thr Val Asp Phe Pro Ala Val Pro Gly
    210                 215                 220

Ala Leu Gly Glu Asn Gly Asn Gly Gly Met Val Thr Gly Cys Ala Glu
225                 230                 235                 240

Thr Pro Gly Cys Val Ala Tyr Ile Gly Ile Ser Phe Leu Asp Gln Ala
                245                 250                 255

Ser Gln Arg Gly Leu Gly Glu Ala Gln Leu Gly Asn Ser Ser Gly Asn
            260                 265                 270

Phe Leu Leu Pro Asp Ala Gln Ser Ile Gln Ala Ala Ala Ala Gly Phe
        275                 280                 285

Ala Ser Lys Thr Pro Ala Asn Gln Ala Ile Ser Met Ile Asp Gly Pro
    290                 295                 300

Ala Pro Asp Gly Tyr Pro Ile Ile Asn Tyr Glu Tyr Ala Ile Val Asn
305                 310                 315                 320

Asn Arg Gln Lys Asp Ala Ala Thr Ala Gln Thr Leu Gln Ala Phe Leu
                325                 330                 335

His Trp Ala Ile Thr Asp Gly Asn Lys Ala Ser Phe Leu Asp Gln Val
            340                 345                 350

His Phe Gln Pro Leu Pro Pro Ala Val Val Lys Leu Ser Asp Ala Leu
        355                 360                 365

Ile Ala Thr Ile Ser Ser
    370

<210>  13
<211>  1013
<212>  DNA
<213>  artificial

<220>
<223>  Rv1886c sequence from Mycobacterium tuberculosis artificially
       optimised for expression in human cells

<220>
<221>  CDS
<222>  (20)..(994)

<400>  13
   atcgaccgtc tccacaggt atg acc gat gtg agc cgt aaa att cgt gca tgg        52
                        Met Thr Asp Val Ser Arg Lys Ile Arg Ala Trp
                        1               5                   10
   ggt cgt cgc ctg atg atc ggt acg gca gca gca gtg gtt ctg ccg ggc        100
   Gly Arg Arg Leu Met Ile Gly Thr Ala Ala Ala Val Val Leu Pro Gly
               15                  20                  25
   ctg gtt ggt ctg gcg ggc ggt gca gca acc gca ggt gca ttt agc cgt        148
   Leu Val Gly Leu Ala Gly Gly Ala Ala Thr Ala Gly Ala Phe Ser Arg
           30                  35                  40
   ccg ggt ctg ccg gtg gaa tat ctg cag gtt ccg agc ccg agt atg ggc        196
   Pro Gly Leu Pro Val Glu Tyr Leu Gln Val Pro Ser Pro Ser Met Gly
       45                  50                  55
   cgc gat att aaa gtg cag ttc cag agc ggc ggt aac aat tct ccg gca        244
   Arg Asp Ile Lys Val Gln Phe Gln Ser Gly Gly Asn Asn Ser Pro Ala
   60                  65                  70                  75
   gtt tat ctg ctg gat ggc ctg cgt gcg cag gat gat tac aat ggt tgg        292
   Val Tyr Leu Leu Asp Gly Leu Arg Ala Gln Asp Asp Tyr Asn Gly Trp
                   80                  85                  90
   gat att aac acg ccg gcg ttt gaa tgg tat tac cag agt ggt ctg agc        340
   Asp Ile Asn Thr Pro Ala Phe Glu Trp Tyr Tyr Gln Ser Gly Leu Ser
               95                  100                 105
   atc gtg atg ccg gtt ggc ggt cag agc tct ttc tat tct gat tgg tac        388
   Ile Val Met Pro Val Gly Gly Gln Ser Ser Phe Tyr Ser Asp Trp Tyr
           110                 115                 120
   agt ccg gca tgc ggc aaa gcg ggt tgt cag acc tat aaa tgg gaa acc        436
   Ser Pro Ala Cys Gly Lys Ala Gly Cys Gln Thr Tyr Lys Trp Glu Thr
       125                 130                 135
   ttt ctg acg agc gaa ctg ccg cag tgg ctg tct gcg aat cgt gca gtg        484
   Phe Leu Thr Ser Glu Leu Pro Gln Trp Leu Ser Ala Asn Arg Ala Val
   140                 145                 150                 155
   aaa ccg acg ggt agt gca gca att ggt ctg agc atg gcc ggc agt agc        532
   Lys Pro Thr Gly Ser Ala Ala Ile Gly Leu Ser Met Ala Gly Ser Ser
                   160                 165                 170
   gca atg att ctg gcc gca tat cat ccg cag cag ttc atc tac gca ggt      580
   Ala Met Ile Leu Ala Ala Tyr His Pro Gln Gln Phe Ile Tyr Ala Gly
               175                 180                 185
   agc ctg tct gca ctg ctg gat ccg agc cag ggc atg ggt ccg agt ctg      628
   Ser Leu Ser Ala Leu Leu Asp Pro Ser Gln Gly Met Gly Pro Ser Leu
           190                 195                 200
   atc ggt ctg gcg atg ggt gat gcc ggc ggt tat aaa gcg gcc gat atg      676
   Ile Gly Leu Ala Met Gly Asp Ala Gly Gly Tyr Lys Ala Ala Asp Met
       205                 210                 215
   tgg ggt ccg tct agt gat ccg gcc tgg gaa cgt aac gat ccg acc cag      724
   Trp Gly Pro Ser Ser Asp Pro Ala Trp Glu Arg Asn Asp Pro Thr Gln
   220                 225                 230                 235
   cag att ccg aaa ctg gtg gcg aac aat acc cgc ctg tgg gtt tac tgc      772
   Gln Ile Pro Lys Leu Val Ala Asn Asn Thr Arg Leu Trp Val Tyr Cys
                   240                 245                 250
   ggc aac ggt acg ccg aat gaa ctg ggc ggt gca aat atc ccg gcg gaa      820
   Gly Asn Gly Thr Pro Asn Glu Leu Gly Gly Ala Asn Ile Pro Ala Glu
               255                 260                 265
   ttt ctg gaa aat ttc gtg cgt agc tct aac ctg aaa ttt cag gat gcc      868
   Phe Leu Glu Asn Phe Val Arg Ser Ser Asn Leu Lys Phe Gln Asp Ala
           270                 275                 280
   tat aac gca gcg ggc ggt cat aac gca gtt ttt aat ttc ccg ccg aac      916
   Tyr Asn Ala Ala Gly Gly His Asn Ala Val Phe Asn Phe Pro Pro Asn
       285                 290                 295
   ggt acc cac agc tgg gaa tac tgg ggc gcc cag ctg aac gca atg aaa      964
   Gly Thr His Ser Trp Glu Tyr Trp Gly Ala Gln Leu Asn Ala Met Lys
   300                 305                 310                 315
   ggt gat ctg cag agt agc ctg ggc gcg ggt ggtgggggag acgatcagc        1013
   Gly Asp Leu Gln Ser Ser Leu Gly Ala Gly
                   320                 325


<210>  14
<211>  325
<212>  PRT
<213>  artificial

<220>
<223>  Synthetic Construct

<400>  14

Met Thr Asp Val Ser Arg Lys Ile Arg Ala Trp Gly Arg Arg Leu Met
1               5                   10                  15


Ile Gly Thr Ala Ala Ala Val Val Leu Pro Gly Leu Val Gly Leu Ala
            20                  25                  30

Gly Gly Ala Ala Thr Ala Gly Ala Phe Ser Arg Pro Gly Leu Pro Val
        35                  40                  45

Glu Tyr Leu Gln Val Pro Ser Pro Ser Met Gly Arg Asp Ile Lys Val
    50                  55                  60

Gln Phe Gln Ser Gly Gly Asn Asn Ser Pro Ala Val Tyr Leu Leu Asp
65                  70                  75                  80

Gly Leu Arg Ala Gln Asp Asp Tyr Asn Gly Trp Asp Ile Asn Thr Pro
                85                  90                  95

Ala Phe Glu Trp Tyr Tyr Gln Ser Gly Leu Ser Ile Val Met Pro Val
            100                 105                 110

Gly Gly Gln Ser Ser Phe Tyr Ser Asp Trp Tyr Ser Pro Ala Cys Gly
        115                 120                 125

Lys Ala Gly Cys Gln Thr Tyr Lys Trp Glu Thr Phe Leu Thr Ser Glu
    130                 135                 140

Leu Pro Gln Trp Leu Ser Ala Asn Arg Ala Val Lys Pro Thr Gly Ser
145                 150                 155                 160

Ala Ala Ile Gly Leu Ser Met Ala Gly Ser Ser Ala Met Ile Leu Ala
                165                 170                 175

Ala Tyr His Pro Gln Gln Phe Ile Tyr Ala Gly Ser Leu Ser Ala Leu
            180                 185                 190

Leu Asp Pro Ser Gln Gly Met Gly Pro Ser Leu Ile Gly Leu Ala Met
        195                 200                 205

Gly Asp Ala Gly Gly Tyr Lys Ala Ala Asp Met Trp Gly Pro Ser Ser
    210                 215                 220

Asp Pro Ala Trp Glu Arg Asn Asp Pro Thr Gln Gln Ile Pro Lys Leu
225                 230                 235                 240

Val Ala Asn Asn Thr Arg Leu Trp Val Tyr Cys Gly Asn Gly Thr Pro
                245                 250                 255

Asn Glu Leu Gly Gly Ala Asn Ile Pro Ala Glu Phe Leu Glu Asn Phe
            260                 265                 270

Val Arg Ser Ser Asn Leu Lys Phe Gln Asp Ala Tyr Asn Ala Ala Gly
        275                 280                 285

Gly His Asn Ala Val Phe Asn Phe Pro Pro Asn Gly Thr His Ser Trp
    290                 295                 300

Glu Tyr Trp Gly Ala Gln Leu Asn Ala Met Lys Gly Asp Leu Gln Ser
305                 310                 315                 320

Ser Leu Gly Ala Gly
                325

<210>  15
<211>  2686
<212>  DNA
<213>  Artificial Sequence

<220>
<223>  Rv1759c sequence from Mycobacterium tuberculosis artificially
       optimised for expression in human cells

<220>
<221>  CDS
<222>  (17)..(2668)

<400>  15
   aaccgtctcc acaggt gcc gct gca gcc aac acc aca agt ctg ctg gct gca       52
                     Ala Ala Ala Ala Asn Thr Thr Ser Leu Leu Ala Ala
                     1               5                   10
   ggc gcc gat gag atc tca gcc gct att gca gca ctg ttc gga gct cac       100
   Gly Ala Asp Glu Ile Ser Ala Ala Ile Ala Ala Leu Phe Gly Ala His
           15                  20                  25
   gga cga gca tac cag gct gca tct gcc gaa gcc gct gca ttc cat ggc       148
   Gly Arg Ala Tyr Gln Ala Ala Ser Ala Glu Ala Ala Ala Phe His Gly
       30                  35                  40
   aga ttt gtg cag gcc ctg act acc gga gga gga gct tat gca gct gca       196
   Arg Phe Val Gln Ala Leu Thr Thr Gly Gly Gly Ala Tyr Ala Ala Ala
   45                  50                  55                  60
   gag gcc gct gca gtg act cca ctg ctg aac agc atc aat gca cca gtc       244
   Glu Ala Ala Ala Val Thr Pro Leu Leu Asn Ser Ile Asn Ala Pro Val
                   65                  70                  75
   ctg gcc gct acc gga cga cct ctg att ggc aac ggg gct aat gga gca       292
   Leu Ala Ala Thr Gly Arg Pro Leu Ile Gly Asn Gly Ala Asn Gly Ala
               80                  85                  90
   cca gga aca gga gca aac gga ggg gac gcc gga tgg ctg atc gga aat       340
   Pro Gly Thr Gly Ala Asn Gly Gly Asp Ala Gly Trp Leu Ile Gly Asn
           95                  100                 105
   gga gga gca gga gga tcc gga gca aaa gga gct aac ggc gga gca gga 388
   Gly Gly Ala Gly Gly Ser Gly Ala Lys Gly Ala Asn Gly Gly Ala Gly
   110 115 120
   gga cca gga gga gca gca ggc ctg ttc ggg aac ggc ggg gca gga ggc 436
   Gly Pro Gly Gly Ala Ala Gly Leu Phe Gly Asn Gly Gly Ala Gly Gly
   125 130 135 140
   gcc ggg gga aca gcc act gct aac aat gga atc gga gga gcc ggc gga 484
   Ala Gly Gly Thr Ala Thr Ala Asn Asn Gly Ile Gly Gly Ala Gly Gly
   145 150 155
   gca gga gga tct gcc atg ctg ttt gga gca gga gga gct gga gga gca 532
   Ala Gly Gly Ser Ala Met Leu Phe Gly Ala Gly Gly Ala Gly Gly Ala
   160 165 170
   gga gga gct gca acc agt ctg gtg gga gga att gga gga acc gga gga 580
   Gly Gly Ala Ala Thr Ser Leu Val Gly Gly Ile Gly Gly Thr Gly Gly
   175 180 185
   aca gga gga aat gca gga atg ctg gct gga gca gcc ggc gca gga gga 628
   Thr Gly Gly Asn Ala Gly Met Leu Ala Gly Ala Ala Gly Ala Gly Gly
   190 195 200
   gct ggg gga ttc tct ttt agt act gca ggc ggc gcc ggc ggc gcc gga 676
   Ala Gly Gly Phe Ser Phe Ser Thr Ala Gly Gly Ala Gly Gly Ala Gly
   205 210 215 220
   gga gca gga ggg ctg ttc aca act gga gga gtc gga gga gca gga gga 724
   Gly Ala Gly Gly Leu Phe Thr Thr Gly Gly Val Gly Gly Ala Gly Gly
   225 230 235
   cag gga cac acc gga ggc gcc ggg gga gct ggc ggg gca gga ggc ctg 772
   Gln Gly His Thr Gly Gly Ala Gly Gly Ala Gly Gly Ala Gly Gly Leu
   240 245 250
   ttc gga gca gga gga atg gga gga gct gga gga ttt ggg gac cat gga 820
   Phe Gly Ala Gly Gly Met Gly Gly Ala Gly Gly Phe Gly Asp His Gly
   255 260 265
   act ctg ggc acc ggg gga gcc ggc ggg gat gga ggc ggg gga ggc ctg 868
   Thr Leu Gly Thr Gly Gly Ala Gly Gly Asp Gly Gly Gly Gly Gly Leu
   270 275 280
   ttt gga gca gga ggc gac ggc gga gct gga gga tca gga ctg acc aca 916
   Phe Gly Ala Gly Gly Asp Gly Gly Ala Gly Gly Ser Gly Leu Thr Thr
   285 290 295 300
   gga gga gca gca gga aac gga gga aat gca gga acc ctg agc ctg ggc 964
   Gly Gly Ala Ala Gly Asn Gly Gly Asn Ala Gly Thr Leu Ser Leu Gly
   305 310 315
   gct gca gga gga gca gga gga acc gga gga gct gga gga aca gtg ttc 1012
   Ala Ala Gly Gly Ala Gly Gly Thr Gly Gly Ala Gly Gly Thr Val Phe
   320 325 330
   ggc ggc ggc aaa ggc ggc gcc ggc gga gct gga ggc aac gcc gga atg 1060
   Gly Gly Gly Lys Gly Gly Ala Gly Gly Ala Gly Gly Asn Ala Gly Met
   335 340 345
   ctg ttt gga agc gga gga gga gga gga aca gga gga ttc gga ttt gca 1108
   Leu Phe Gly Ser Gly Gly Gly Gly Gly Thr Gly Gly Phe Gly Phe Ala
   350 355 360
   gct gga gga cag gga gga gtc gga gga tcc gca gga atg ctg tca gga 1156
   Ala Gly Gly Gln Gly Gly Val Gly Gly Ser Ala Gly Met Leu Ser Gly
   365 370 375 380
   agc gga ggc agt ggg gga gcc ggc ggg tca gga ggc cct gct ggc aca 1204
   Ser Gly Gly Ser Gly Gly Ala Gly Gly Ser Gly Gly Pro Ala Gly Thr
   385 390 395
   gca gcc ggg gga gcc ggg gga gca gga gga gct cca ggg ctg atc gga 1252
   Ala Ala Gly Gly Ala Gly Gly Ala Gly Gly Ala Pro Gly Leu Ile Gly
   400 405 410
   aac ggg gga aat ggc ggg aac gga ggc gag tct gga gga act gga gga 1300
   Asn Gly Gly Asn Gly Gly Asn Gly Gly Glu Ser Gly Gly Thr Gly Gly
   415 420 425
   gtg gga gga gca gga gga aac gca gtc ctg att ggc aat gga gga gaa 1348
   Val Gly Gly Ala Gly Gly Asn Ala Val Leu Ile Gly Asn Gly Gly Glu
   430 435 440
   gga gga att gga gca ctg gcc ggc aag agt gga ttc gga gga ttt gga 1396
   Gly Gly Ile Gly Ala Leu Ala Gly Lys Ser Gly Phe Gly Gly Phe Gly
   445 450 455 460
   gga ctg ctg ctg gga gct gat ggc tac aac gca ccc gaa tcc acc tct 1444
   Gly Leu Leu Leu Gly Ala Asp Gly Tyr Asn Ala Pro Glu Ser Thr Ser
   465 470 475
   cct tgg cac aat ctg cag cag gac atc ctg tcc ttc att aac gag cca 1492
   Pro Trp His Asn Leu Gln Gln Asp Ile Leu Ser Phe Ile Asn Glu Pro
   480 485 490
   act gaa gcc ctg acc ggc aga ccc ctg atc ggg aat gga gat tct ggc 1540
   Thr Glu Ala Leu Thr Gly Arg Pro Leu Ile Gly Asn Gly Asp Ser Gly
   495 500 505
   aca cct gga act ggg gac gat ggc ggc gca gga gga tgg ctg ttt ggc 1588
   Thr Pro Gly Thr Gly Asp Asp Gly Gly Ala Gly Gly Trp Leu Phe Gly
   510 515 520
   aat gga gga aac gga gga gca gga gct gca gga act aac gga agc gct 1636
   Asn Gly Gly Asn Gly Gly Ala Gly Ala Ala Gly Thr Asn Gly Ser Ala
   525 530 535 540
   ggc ggc gcc ggg gga gct gga ggc att ctg ttc gga aca gga gga gcc 1684
   Gly Gly Ala Gly Gly Ala Gly Gly Ile Leu Phe Gly Thr Gly Gly Ala
   545 550 555
   gga ggc gca gga gga gtg gga act gct gga gca ggg ggc gca ggc ggc 1732
   Gly Gly Ala Gly Gly Val Gly Thr Ala Gly Ala Gly Gly Ala Gly Gly
   560 565 570
   gca gga gga agc gcc ttt ctg atc ggc tcc gga gga acc ggc ggc gtg 1780
   Ala Gly Gly Ser Ala Phe Leu Ile Gly Ser Gly Gly Thr Gly Gly Val
   575 580 585
   gga gga gca gct act acc aca gga gga gtc gga gga gct gga gga aat 1828
   Gly Gly Ala Ala Thr Thr Thr Gly Gly Val Gly Gly Ala Gly Gly Asn
   590 595 600
   gca gga ctg ctg att gga gca gca gga ctg gga gga tgc gga gga gga 1876
   Ala Gly Leu Leu Ile Gly Ala Ala Gly Leu Gly Gly Cys Gly Gly Gly
   605 610 615 620
   gcc ttc acc gct ggc gtg act acc gga gga gca gga gga aca gga gga 1924
   Ala Phe Thr Ala Gly Val Thr Thr Gly Gly Ala Gly Gly Thr Gly Gly
   625 630 635
   gct gca ggc ctg ttt gcc aac ggg ggc gcc ggc ggc gca gga gga acc 1972
   Ala Ala Gly Leu Phe Ala Asn Gly Gly Ala Gly Gly Ala Gly Gly Thr
   640 645 650
   gga agc acc gcc gga ggc gcc ggc gga gct ggg gga gct ggc ggg ctg 2020
   Gly Ser Thr Ala Gly Gly Ala Gly Gly Ala Gly Gly Ala Gly Gly Leu
   655 660 665
   tac gcc cac gga ggc aca ggg gga ccc ggc ggg aac gga ggc agt aca 2068
   Tyr Ala His Gly Gly Thr Gly Gly Pro Gly Gly Asn Gly Gly Ser Thr
   670 675 680
   gga gca gga gga act gga gga gct gga gga cct ggg gga ctg tat ggc 2116
   Gly Ala Gly Gly Thr Gly Gly Ala Gly Gly Pro Gly Gly Leu Tyr Gly
   685 690 695 700
   gct gga ggc tca gga ggc gct ggg gga cat gga gga atg gcc gga gga 2164
   Ala Gly Gly Ser Gly Gly Ala Gly Gly His Gly Gly Met Ala Gly Gly
   705 710 715
   gga gga gga gtg gga gga aac gca ggc tct ctg acc ctg aat gcc agt 2212
   Gly Gly Gly Val Gly Gly Asn Ala Gly Ser Leu Thr Leu Asn Ala Ser
   720 725 730
   gga ggc gcc gga gga tca gga gga agc tcc ctg agc gga aag gcc ggc 2260
   Gly Gly Ala Gly Gly Ser Gly Gly Ser Ser Leu Ser Gly Lys Ala Gly
   735 740 745
   gca ggc ggg gca gga gga tcc gca gga ctg ttc tac gga tct ggc ggc 2308
   Ala Gly Gly Ala Gly Gly Ser Ala Gly Leu Phe Tyr Gly Ser Gly Gly
   750 755 760
   gca gga gga aac gga gga tat agc ctg aat ggc act gga gga gat ggc 2356
   Ala Gly Gly Asn Gly Gly Tyr Ser Leu Asn Gly Thr Gly Gly Asp Gly
   765 770 775 780
   gga acc gga gga gca gga cag atc aca gga ctg agg agc ggc ttc ggc 2404
   Gly Thr Gly Gly Ala Gly Gln Ile Thr Gly Leu Arg Ser Gly Phe Gly
   785 790 795
   gga gcc gga ggg gca gga gga gct tcc gac act gga gca gga gga aat 2452
   Gly Ala Gly Gly Ala Gly Gly Ala Ser Asp Thr Gly Ala Gly Gly Asn
   800 805 810
   gga gga gct gga ggc aag gca ggg ctg tat gga aac gga ggc gac ggc 2500
   Gly Gly Ala Gly Gly Lys Ala Gly Leu Tyr Gly Asn Gly Gly Asp Gly
   815 820 825
   gga gca gga ggc gat ggc gct acc tcc ggc aaa ggc ggg gca gga gga          2548
   Gly Ala Gly Gly Asp Gly Ala Thr Ser Gly Lys Gly Gly Ala Gly Gly
       830                 835                 840
   aac gct gtg gtc att ggc aat gga ggc aac gga gga aat gca ggc aag          2596
   Asn Ala Val Val Ile Gly Asn Gly Gly Asn Gly Gly Asn Ala Gly Lys
   845                 850                 855                 860
   gca gga gga acc gca gga gca gga gga gcc ggg gga ctg gtg ctg gga          2644
   Ala Gly Gly Thr Ala Gly Ala Gly Gly Ala Gly Gly Leu Val Leu Gly
                   865                 870                 875
   cgc gac ggg cag cat gga ctg aca taatgaaagc ttgaattc                      2686
   Arg Asp Gly Gln His Gly Leu Thr
               880

<210>  16
<211>  884
<212>  PRT
<213>  Artificial Sequence

<220>
<223>  Synthetic Construct

<400>  16

Ala Ala Ala Ala Asn Thr Thr Ser Leu Leu Ala Ala Gly Ala Asp Glu
1               5                   10                  15

Ile Ser Ala Ala Ile Ala Ala Leu Phe Gly Ala His Gly Arg Ala Tyr
            20                  25                  30

Gln Ala Ala Ser Ala Glu Ala Ala Ala Phe His Gly Arg Phe Val Gln
        35                  40                  45

Ala Leu Thr Thr Gly Gly Gly Ala Tyr Ala Ala Ala Glu Ala Ala Ala
    50                  55                  60

Val Thr Pro Leu Leu Asn Ser Ile Asn Ala Pro Val Leu Ala Ala Thr
65                  70                  75                  80

Gly Arg Pro Leu Ile Gly Asn Gly Ala Asn Gly Ala Pro Gly Thr Gly
                85                  90                  95

Ala Asn Gly Gly Asp Ala Gly Trp Leu Ile Gly Asn Gly Gly Ala Gly
            100                 105                 110

Gly Ser Gly Ala Lys Gly Ala Asn Gly Gly Ala Gly Gly Pro Gly Gly
        115                 120                 125

Ala Ala Gly Leu Phe Gly Asn Gly Gly Ala Gly Gly Ala Gly Gly Thr
    130                 135                 140

Ala Thr Ala Asn Asn Gly Ile Gly Gly Ala Gly Gly Ala Gly Gly Ser
145                 150                 155                 160

Ala Met Leu Phe Gly Ala Gly Gly Ala Gly Gly Ala Gly Gly Ala Ala
                165                 170                 175

Thr Ser Leu Val Gly Gly Ile Gly Gly Thr Gly Gly Thr Gly Gly Asn
            180                 185                 190

Ala Gly Met Leu Ala Gly Ala Ala Gly Ala Gly Gly Ala Gly Gly Phe
        195                 200                 205

Ser Phe Ser Thr Ala Gly Gly Ala Gly Gly Ala Gly Gly Ala Gly Gly
    210                 215                 220

Leu Phe Thr Thr Gly Gly Val Gly Gly Ala Gly Gly Gln Gly His Thr
225                 230                 235                 240

Gly Gly Ala Gly Gly Ala Gly Gly Ala Gly Gly Leu Phe Gly Ala Gly
                245                 250                 255

Gly Met Gly Gly Ala Gly Gly Phe Gly Asp His Gly Thr Leu Gly Thr
            260                 265                 270

Gly Gly Ala Gly Gly Asp Gly Gly Gly Gly Gly Leu Phe Gly Ala Gly
        275                 280                 285

Gly Asp Gly Gly Ala Gly Gly Ser Gly Leu Thr Thr Gly Gly Ala Ala
    290                 295                 300

Gly Asn Gly Gly Asn Ala Gly Thr Leu Ser Leu Gly Ala Ala Gly Gly
305                 310                 315                 320

Ala Gly Gly Thr Gly Gly Ala Gly Gly Thr Val Phe Gly Gly Gly Lys
                325                 330                 335

Gly Gly Ala Gly Gly Ala Gly Gly Asn Ala Gly Met Leu Phe Gly Ser
            340                 345                 350

Gly Gly Gly Gly Gly Thr Gly Gly Phe Gly Phe Ala Ala Gly Gly Gln
        355                 360                 365

Gly Gly Val Gly Gly Ser Ala Gly Met Leu Ser Gly Ser Gly Gly Ser
    370                 375                 380

Gly Gly Ala Gly Gly Ser Gly Gly Pro Ala Gly Thr Ala Ala Gly Gly
385                 390                 395                 400

Ala Gly Gly Ala Gly Gly Ala Pro Gly Leu Ile Gly Asn Gly Gly Asn
                405                 410                 415

Gly Gly Asn Gly Gly Glu Ser Gly Gly Thr Gly Gly Val Gly Gly Ala
            420                 425                 430

Gly Gly Asn Ala Val Leu Ile Gly Asn Gly Gly Glu Gly Gly Ile Gly
        435                 440                 445

Ala Leu Ala Gly Lys Ser Gly Phe Gly Gly Phe Gly Gly Leu Leu Leu
    450                 455                 460

Gly Ala Asp Gly Tyr Asn Ala Pro Glu Ser Thr Ser Pro Trp His Asn
465                 470                 475                 480

Leu Gln Gln Asp Ile Leu Ser Phe Ile Asn Glu Pro Thr Glu Ala Leu
                485                 490                 495

Thr Gly Arg Pro Leu Ile Gly Asn Gly Asp Ser Gly Thr Pro Gly Thr
            500                 505                 510

Gly Asp Asp Gly Gly Ala Gly Gly Trp Leu Phe Gly Asn Gly Gly Asn
        515                 520                 525

Gly Gly Ala Gly Ala Ala Gly Thr Asn Gly Ser Ala Gly Gly Ala Gly
    530                 535                 540

Gly Ala Gly Gly Ile Leu Phe Gly Thr Gly Gly Ala Gly Gly Ala Gly
545                 550                 555                 560

Gly Val Gly Thr Ala Gly Ala Gly Gly Ala Gly Gly Ala Gly Gly Ser
                565                 570                 575

Ala Phe Leu Ile Gly Ser Gly Gly Thr Gly Gly Val Gly Gly Ala Ala
            580                 585                 590

Thr Thr Thr Gly Gly Val Gly Gly Ala Gly Gly Asn Ala Gly Leu Leu
        595                 600                 605

Ile Gly Ala Ala Gly Leu Gly Gly Cys Gly Gly Gly Ala Phe Thr Ala
    610                 615                 620

Gly Val Thr Thr Gly Gly Ala Gly Gly Thr Gly Gly Ala Ala Gly Leu
625                 630                 635                 640

Phe Ala Asn Gly Gly Ala Gly Gly Ala Gly Gly Thr Gly Ser Thr Ala
                645                 650                 655

Gly Gly Ala Gly Gly Ala Gly Gly Ala Gly Gly Leu Tyr Ala His Gly
            660                 665                 670

Gly Thr Gly Gly Pro Gly Gly Asn Gly Gly Ser Thr Gly Ala Gly Gly
        675                 680                 685

Thr Gly Gly Ala Gly Gly Pro Gly Gly Leu Tyr Gly Ala Gly Gly Ser
    690                 695                 700

Gly Gly Ala Gly Gly His Gly Gly Met Ala Gly Gly Gly Gly Gly Val
705                 710                 715                 720

Gly Gly Asn Ala Gly Ser Leu Thr Leu Asn Ala Ser Gly Gly Ala Gly
                725                 730                 735

Gly Ser Gly Gly Ser Ser Leu Ser Gly Lys Ala Gly Ala Gly Gly Ala
            740                 745                 750

Gly Gly Ser Ala Gly Leu Phe Tyr Gly Ser Gly Gly Ala Gly Gly Asn
        755                 760                 765

Gly Gly Tyr Ser Leu Asn Gly Thr Gly Gly Asp Gly Gly Thr Gly Gly
    770                 775                 780

Ala Gly Gln Ile Thr Gly Leu Arg Ser Gly Phe Gly Gly Ala Gly Gly
785                 790                 795                 800

Ala Gly Gly Ala Ser Asp Thr Gly Ala Gly Gly Asn Gly Gly Ala Gly
                805                 810                 815

Gly Lys Ala Gly Leu Tyr Gly Asn Gly Gly Asp Gly Gly Ala Gly Gly
            820                 825                 830

Asp Gly Ala Thr Ser Gly Lys Gly Gly Ala Gly Gly Asn Ala Val Val
        835                 840                 845

Ile Gly Asn Gly Gly Asn Gly Gly Asn Ala Gly Lys Ala Gly Gly Thr
    850                 855                 860

Ala Gly Ala Gly Gly Ala Gly Gly Leu Val Leu Gly Arg Asp Gly Gln
865                 870                 875                 880

His Gly Leu Thr

<210>  17
<211>  719
<212>  DNA
<213>  artificial

<220>
<223>  Rv1980c sequence from Mycobacterium tuberculosis artificially
       optimised for expression in human cells

<220>
<221>  CDS
<222>  (20)..(700)

<400>  17
atcgaccgtc tccacaggt atg cgg atc aag atc ttc atg ctg gtg aca gcc        52
                     Met Arg Ile Lys Ile Phe Met Leu Val Thr Ala
                     1               5                   10

gtg gtg ctg ctg tgc tgt tct ggc gtg gct act gcc gct ccc aag acc        100
Val Val Leu Leu Cys Cys Ser Gly Val Ala Thr Ala Ala Pro Lys Thr
            15                  20                  25

tac tgc gag gaa ctg aaa gga acc gac aca ggc cag gcc tgt cag atc        148
Tyr Cys Glu Glu Leu Lys Gly Thr Asp Thr Gly Gln Ala Cys Gln Ile
        30                  35                  40

cag atg tcc gat cct gct tac aac atc aat att tct ctg cct agt tac        196
Gln Met Ser Asp Pro Ala Tyr Asn Ile Asn Ile Ser Leu Pro Ser Tyr
    45                  50                  55

tat cca gac cag aag agc ctg gag aac tat att gcc cag acc cgg gat        244
Tyr Pro Asp Gln Lys Ser Leu Glu Asn Tyr Ile Ala Gln Thr Arg Asp
60                  65                  70                  75

aaa ttc ctg tcc gcc gct act agc tcc acc ccc aga gag gcc cct tac        292
Lys Phe Leu Ser Ala Ala Thr Ser Ser Thr Pro Arg Glu Ala Pro Tyr
                80                  85                  90

gaa tat atc aca tcc gcc act tat cag tct gct att ccc cct agg gga        340
Glu Tyr Ile Thr Ser Ala Thr Tyr Gln Ser Ala Ile Pro Pro Arg Gly
            95                  100                 105

aca cag gct gtg gtg ctg aag gtg tac cag aat gct ggc ggg act cac        388
Thr Gln Ala Val Val Leu Lys Val Tyr Gln Asn Ala Gly Gly Thr His
        110                 115                 120

cct acc aca act tat aag gcc ttt gac tgg gat cag gct tac cgc aaa        436

Pro Thr Thr Thr Tyr Lys Ala Phe Asp Trp Asp Gln Ala Tyr Arg Lys
    125                 130                 135

cca atc acc tat gac aca ctg tgg cag gct gac aca gat cca ctg cct        484
Pro Ile Thr Tyr Asp Thr Leu Trp Gln Ala Asp Thr Asp Pro Leu Pro
140                 145                 150                 155

gtg gtg ttc cca atc gtg cag ggg gaa ctg agt aaa cag aca gga cag        532
Val Val Phe Pro Ile Val Gln Gly Glu Leu Ser Lys Gln Thr Gly Gln
                160                 165                 170

cag gtg tca att gcc cca aac gct ggg ctg gac ccc gtg aac tac cag        580
Gln Val Ser Ile Ala Pro Asn Ala Gly Leu Asp Pro Val Asn Tyr Gln
            175                 180                 185

aat ttc gcc gtg act aac gat ggc gtg atc ttc ttt ttc aat cct gga        628
Asn Phe Ala Val Thr Asn Asp Gly Val Ile Phe Phe Phe Asn Pro Gly
        190                 195                 200

gag ctg ctg cct gaa gct gct gga cct acc cag gtg ctg gtg cca agg        676
Glu Leu Leu Pro Glu Ala Ala Gly Pro Thr Gln Val Leu Val Pro Arg
    205                 210                 215

tca gcc att gat agc atg ctg gct ggtgggggag acgatcagc                   719
Ser Ala Ile Asp Ser Met Leu Ala
220                 225


<210>  18
<211>  227
<212>  PRT
<213>  artificial

<220>
<223>  Synthetic Construct

<400>  18

Met Arg Ile Lys Ile Phe Met Leu Val Thr Ala Val Val Leu Leu Cys
1               5                   10                  15


Cys Ser Gly Val Ala Thr Ala Ala Pro Lys Thr Tyr Cys Glu Glu Leu
            20                  25                  30


Lys Gly Thr Asp Thr Gly Gln Ala Cys Gln Ile Gln Met Ser Asp Pro
        35                  40                  45


Ala Tyr Asn Ile Asn Ile Ser Leu Pro Ser Tyr Tyr Pro Asp Gln Lys
    50                  55                  60


Ser Leu Glu Asn Tyr Ile Ala Gln Thr Arg Asp Lys Phe Leu Ser Ala
65                  70                  75                  80


Ala Thr Ser Ser Thr Pro Arg Glu Ala Pro Tyr Glu Tyr Ile Thr Ser
                85                  90                  95

Ala Thr Tyr Gln Ser Ala Ile Pro Pro Arg Gly Thr Gln Ala Val Val
            100                 105                 110

Leu Lys Val Tyr Gln Asn Ala Gly Gly Thr His Pro Thr Thr Thr Tyr
        115                 120                 125

Lys Ala Phe Asp Trp Asp Gln Ala Tyr Arg Lys Pro Ile Thr Tyr Asp
    130                 135                 140

Thr Leu Trp Gln Ala Asp Thr Asp Pro Leu Pro Val Val Phe Pro Ile
145                 150                 155                 160

Val Gln Gly Glu Leu Ser Lys Gln Thr Gly Gln Gln Val Ser Ile Ala
                165                 170                 175

Pro Asn Ala Gly Leu Asp Pro Val Asn Tyr Gln Asn Phe Ala Val Thr
            180                 185                 190

Asn Asp Gly Val Ile Phe Phe Phe Asn Pro Gly Glu Leu Leu Pro Glu
        195                 200                 205

Ala Ala Gly Pro Thr Gln Val Leu Val Pro Arg Ser Ala Ile Asp Ser
    210                 215                 220

Met Leu Ala
225

<210> 19
<211> 1013
<212> DNA
<213> artificial

<220>
<223> Rv1860 sequence from Mycobacterium tuberculosis artificially
optimised for expression in human cells

<220>
<221> CDS
<222> (20)..(994)

<400> 19
   atcgaccgtc tccacaggt atg cac cag gtg gac ccc aac ctg acc cgg aga 52
   Met His Gln Val Asp Pro Asn Leu Thr Arg Arg
   1 5 10
   aaa ggc cgg ctg gcc gct ctg gct att gct gct atg gct agc gcc tcc 100
   Lys Gly Arg Leu Ala Ala Leu Ala Ile Ala Ala Met Ala Ser Ala Ser
   15 20 25
   ctg gtg act gtg gct gtg cca gct acc gcc aat gct gat ccc gaa cca 148
   Leu Val Thr Val Ala Val Pro Ala Thr Ala Asn Ala Asp Pro Glu Pro
   30 35 40
   gcc cct ccc gtg cca acc aca gct gct agc cca ccc tcc acc gcc gct 196
   Ala Pro Pro Val Pro Thr Thr Ala Ala Ser Pro Pro Ser Thr Ala Ala
   45 50 55
   gcc cct cca gcc cca gct aca cct gtg gcc cct ccc cca ccc gct gct 244
   Ala Pro Pro Ala Pro Ala Thr Pro Val Ala Pro Pro Pro Pro Ala Ala
   60 65 70 75
   gct aac aca cct aat gct cag cca ggg gac cca aat gct gct cct cca 292
   Ala Asn Thr Pro Asn Ala Gln Pro Gly Asp Pro Asn Ala Ala Pro Pro
   80 85 90
   ccc gct gat ccc aat gcc cct cca ccc cct gtg atc gct cct aac gcc 340
   Pro Ala Asp Pro Asn Ala Pro Pro Pro Pro Val Ile Ala Pro Asn Ala
   95 100 105
   cca cag ccc gtg cgg att gac aat cct gtg ggc ggg ttc agt ttt gct 388
   Pro Gln Pro Val Arg Ile Asp Asn Pro Val Gly Gly Phe Ser Phe Ala
   110 115 120
   ctg cct gct gga tgg gtg gaa tca gat gct gct cat ttc gat tac ggg 436
   Leu Pro Ala Gly Trp Val Glu Ser Asp Ala Ala His Phe Asp Tyr Gly
   125 130 135
   tca gcc ctg ctg agc aaa act acc gga gat cca ccc ttc cct gga cag 484
   Ser Ala Leu Leu Ser Lys Thr Thr Gly Asp Pro Pro Phe Pro Gly Gln
   140 145 150 155
   cct cca ccc gtg gct aat gac act agg atc gtg ctg ggg cgc ctg gat 532
   Pro Pro Pro Val Ala Asn Asp Thr Arg Ile Val Leu Gly Arg Leu Asp
   160 165 170
   cag aag ctg tat gcc tcc gct gag gcc acc gac tct aaa gct gct gct 580
   Gln Lys Leu Tyr Ala Ser Ala Glu Ala Thr Asp Ser Lys Ala Ala Ala
   175 180 185
   cgg ctg gga agt gat atg gga gag ttc tac atg cct tat cca gga aca 628
   Arg Leu Gly Ser Asp Met Gly Glu Phe Tyr Met Pro Tyr Pro Gly Thr
   190 195 200
   aga att aac cag gag acc gtg agc ctg gac gct aat gga gtg tct ggc 676
   Arg Ile Asn Gln Glu Thr Val Ser Leu Asp Ala Asn Gly Val Ser Gly
   205 210 215
   agt gcc tca tac tat gaa gtg aag ttt tct gat ccc agt aaa cct aac 724
   Ser Ala Ser Tyr Tyr Glu Val Lys Phe Ser Asp Pro Ser Lys Pro Asn
   220 225 230 235
   gga cag atc tgg aca ggc gtg atc gga tct cct gct gct aac gct cct 772
   Gly Gln Ile Trp Thr Gly Val Ile Gly Ser Pro Ala Ala Asn Ala Pro
   240 245 250
   gat gct ggc cct cca cag agg tgg ttc gtg gtg tgg ctg ggg act gcc 820
   Asp Ala Gly Pro Pro Gln Arg Trp Phe Val Val Trp Leu Gly Thr Ala
   255 260 265
   aac aat cct gtg gat aag gga gct gcc aaa gct ctg gct gaa tcc atc          868
   Asn Asn Pro Val Asp Lys Gly Ala Ala Lys Ala Leu Ala Glu Ser Ile
           270                 275                 280
   aga cca ctg gtg gcc cct ccc cca gct cct gct cct gct cca gcc gag          916
   Arg Pro Leu Val Ala Pro Pro Pro Ala Pro Ala Pro Ala Pro Ala Glu
       285                 290                 295
   ccc gct cct gcc cca gct ccc gcc ggc gaa gtg gcc cct acc cca aca          964
   Pro Ala Pro Ala Pro Ala Pro Ala Gly Glu Val Ala Pro Thr Pro Thr
   300                 305                 310                 315
   act ccc acc cct cag cgg aca ctg ccc gcc ggtgggggag acgatcagc          1013
   Thr Pro Thr Pro Gln Arg Thr Leu Pro Ala
                   320                 325


<210>  20
<211>  325
<212>  PRT
<213>  artificial

<220>
<223>  Synthetic Construct

<400>  20

Met His Gln Val Asp Pro Asn Leu Thr Arg Arg Lys Gly Arg Leu Ala
1               5                   10                  15


Ala Leu Ala Ile Ala Ala Met Ala Ser Ala Ser Leu Val Thr Val Ala
            20                  25                  30


Val Pro Ala Thr Ala Asn Ala Asp Pro Glu Pro Ala Pro Pro Val Pro
        35                  40                  45


Thr Thr Ala Ala Ser Pro Pro Ser Thr Ala Ala Ala Pro Pro Ala Pro
    50                  55                  60


Ala Thr Pro Val Ala Pro Pro Pro Pro Ala Ala Ala Asn Thr Pro Asn
65                  70                  75                  80


Ala Gln Pro Gly Asp Pro Asn Ala Ala Pro Pro Pro Ala Asp Pro Asn
                85                  90                  95


Ala Pro Pro Pro Pro Val Ile Ala Pro Asn Ala Pro Gln Pro Val Arg
            100                 105                 110


Ile Asp Asn Pro Val Gly Gly Phe Ser Phe Ala Leu Pro Ala Gly Trp
        115                 120                 125

Val Glu Ser Asp Ala Ala His Phe Asp Tyr Gly Ser Ala Leu Leu Ser
    130                 135                 140

Lys Thr Thr Gly Asp Pro Pro Phe Pro Gly Gln Pro Pro Pro Val Ala
145                 150                 155                 160

Asn Asp Thr Arg Ile Val Leu Gly Arg Leu Asp Gln Lys Leu Tyr Ala
                165                 170                 175

Ser Ala Glu Ala Thr Asp Ser Lys Ala Ala Ala Arg Leu Gly Ser Asp
            180                 185                 190

Met Gly Glu Phe Tyr Met Pro Tyr Pro Gly Thr Arg Ile Asn Gln Glu
        195                 200                 205

Thr Val Ser Leu Asp Ala Asn Gly Val Ser Gly Ser Ala Ser Tyr Tyr
    210                 215                 220

Glu Val Lys Phe Ser Asp Pro Ser Lys Pro Asn Gly Gln Ile Trp Thr
225                 230                 235                 240

Gly Val Ile Gly Ser Pro Ala Ala Asn Ala Pro Asp Ala Gly Pro Pro
                245                 250                 255

Gln Arg Trp Phe Val Val Trp Leu Gly Thr Ala Asn Asn Pro Val Asp
            260                 265                 270

Lys Gly Ala Ala Lys Ala Leu Ala Glu Ser Ile Arg Pro Leu Val Ala
        275                 280                 285

Pro Pro Pro Ala Pro Ala Pro Ala Pro Ala Glu Pro Ala Pro Ala Pro
    290                 295                 300

Ala Pro Ala Gly Glu Val Ala Pro Thr Pro Thr Thr Pro Thr Pro Gln
305                 310                 315                 320

Arg Thr Leu Pro Ala
                325

<210>  21
<211>  337
<212>  DNA
<213>  artificial

<220>
<223>  Rv3874 sequence from Mycobacterium tuberculosis artificially
       optimised for expression in human cells

<220>
<221>  CDS
<222>  (20)..(319)

<400>  21
atcgaccgtc tccacaggt atg gca gaa atg aaa aca gat gca gca aca ctg        52
                     Met Ala Glu Met Lys Thr Asp Ala Ala Thr Leu
                     1               5                   10

gca cag gaa gcc gga aac ttt gaa cga att agc ggg gac ctg aaa acc        100
Ala Gln Glu Ala Gly Asn Phe Glu Arg Ile Ser Gly Asp Leu Lys Thr
            15                  20                  25

cag att gac cag gtg gag agc acc gca gga tcc ctg cag gga cag tgg        148
Gln Ile Asp Gln Val Glu Ser Thr Ala Gly Ser Leu Gln Gly Gln Trp
        30                  35                  40

aga ggc gcc gct ggg aca gca gcc cag gct gca gtg gtc aga ttc cag        196
Arg Gly Ala Ala Gly Thr Ala Ala Gln Ala Ala Val Val Arg Phe Gln
    45                  50                  55

gaa gcc gct aac aag cag aaa cag gag ctg gat gaa atc tct acc aat        244
Glu Ala Ala Asn Lys Gln Lys Gln Glu Leu Asp Glu Ile Ser Thr Asn
60                  65                  70                  75

att agg cag gcc ggg gtc cag tac agc aga gca gat gag gag cag cag        292
Ile Arg Gln Ala Gly Val Gln Tyr Ser Arg Ala Asp Glu Glu Gln Gln
                80                  85                  90

cag gca ctg agc agc cag atg ggg ttc taatgagaat tcaagctt                337
Gln Ala Leu Ser Ser Gln Met Gly Phe
            95                  100


<210>  22
<211>  100
<212>  PRT
<213>  artificial

<220>
<223>  Synthetic Construct

<400>  22

Met Ala Glu Met Lys Thr Asp Ala Ala Thr Leu Ala Gln Glu Ala Gly
1               5                   10                  15


Asn Phe Glu Arg Ile Ser Gly Asp Leu Lys Thr Gln Ile Asp Gln Val
            20                  25                  30


Glu Ser Thr Ala Gly Ser Leu Gln Gly Gln Trp Arg Gly Ala Ala Gly
        35                  40                  45


Thr Ala Ala Gln Ala Ala Val Val Arg Phe Gln Glu Ala Ala Asn Lys
    50                  55                  60

Gln Lys Gln Glu Leu Asp Glu Ile Ser Thr Asn Ile Arg Gln Ala Gly
65                  70                  75                  80

Val Gln Tyr Ser Arg Ala Asp Glu Glu Gln Gln Gln Ala Leu Ser Ser
                85                  90                  95

Gln Met Gly Phe
            100

<210>  23
<211>  321
<212>  DNA
<213>  artificial

<220>
<223>  Rv3875 sequence from Mycobacterium tuberculosis artificially
       optimised for expression in human cells

<220>
<221>  CDS
<222>  (20)..(304)

<400>  23
atcgaccgtc tccacaggt atg acc gaa cag cag tgg aac ttt gcg ggc att      52
                     Met Thr Glu Gln Gln Trp Asn Phe Ala Gly Ile
                     1               5                   10

gaa gcg gcc gca agc gcc atc cag ggt aat gtg acc agt att cat agc      100
Glu Ala Ala Ala Ser Ala Ile Gln Gly Asn Val Thr Ser Ile His Ser
            15                  20                  25

ctg ctg gat gaa ggc aaa cag tct ctg acg aaa ctg gca gca gca tgg      148
Leu Leu Asp Glu Gly Lys Gln Ser Leu Thr Lys Leu Ala Ala Ala Trp
        30                  35                  40

ggt ggt agc ggt tct gaa gcg tat cag ggt gtt cag cag aaa tgg gat      196
Gly Gly Ser Gly Ser Glu Ala Tyr Gln Gly Val Gln Gln Lys Trp Asp
    45                  50                  55

gcg acc gcc acg gaa ctg aac aat gcc ctg cag aac ctg gca cgt acg      244
Ala Thr Ala Thr Glu Leu Asn Asn Ala Leu Gln Asn Leu Ala Arg Thr
60                  65                  70                  75

att tct gaa gcc ggt cag gca atg gcg agt acc gaa ggc aat gtg acg      292
Ile Ser Glu Ala Gly Gln Ala Met Ala Ser Thr Glu Gly Asn Val Thr
                80                  85                  90

ggc atg ttc gcc ggtgggggag acgatca                                   321
Gly Met Phe Ala
            95

<210>  24
<211>  95

<212>  PRT
<213>  artificial

<220>
<223>  Synthetic Construct

<400>  24

Met Thr Glu Gln Gln Trp Asn Phe Ala Gly Ile Glu Ala Ala Ala Ser
1               5                   10                  15

Ala Ile Gln Gly Asn Val Thr Ser Ile His Ser Leu Leu Asp Glu Gly
            20                  25                  30

Lys Gln Ser Leu Thr Lys Leu Ala Ala Ala Trp Gly Gly Ser Gly Ser
        35                  40                  45

Glu Ala Tyr Gln Gly Val Gln Gln Lys Trp Asp Ala Thr Ala Thr Glu
    50                  55                  60

Leu Asn Asn Ala Leu Gln Asn Leu Ala Arg Thr Ile Ser Glu Ala Gly
65                  70                  75                  80

Gln Ala Met Ala Ser Thr Glu Gly Asn Val Thr Gly Met Phe Ala
                85                  90                  95

<210>  25
<211>  24
<212>  PRT
<213>  Homo sapiens

<400>  25

Met Ala Val Met Ala Pro Arg Thr Leu Val Leu Leu Leu Ser Gly Ala
1               5                   10                  15

Leu Ala Leu Thr Gln Thr Trp Ala
            20

<210>  26
<211>  17
<212>  PRT
<213>  Mus musculus

<400>  26

Thr Met Gly Trp Ser Cys Ile Ile Leu Phe Leu Val Ala Thr Ala Thr
1               5                   10                  15

Gly

<210> 27
<211> 9234
<212> DNA
<213> Artificial Sequence

<220>
<223> pFRIDA-mIgG2a-N N-terminal cloning vector

<400> 27
gacggatcgg gagatccagg aaaaggacaa gcagcgaaaa ttcacgcccc cttgggaggt 60

ggcggcatat gcaaaggata gcactcccac tctactactg ggtatcatat gctgactgta 120

tatgcatgag gatagcatat gctacccgga tacagattag gatagcatat actacccaga 180

tatagattag gatagcatat gctacccaga tatagattag gatagcctat gctacccaga 240

tataaattag gatagcatat actacccaga tatagattag gatagcatat gctacccaga 300

tatagattag gatagcctat gctacccaga tatagattag gatagcatat gctacccaga 360

tatagattag gatagcatat gctatccaga tatttgggta gtatatgcta cccagatata 420

aattaggata gcatatacta ccctaatctc tattaggata gcatatgcta cccggataca 480

gattaggata gcatatacta cccagatata gattaggata gcatatgcta cccagatata 540

gattaggata gcctatgcta cccagatata aattaggata gcatatacta cccagatata 600

gattaggata gcatatgcta cccagatata gattaggata gcctatgcta cccagatata 660

gattaggata gcatatgcta tccagatatt tgggtagtat atgctaccca tggcaacatt 720

agcccaccgt gctctcagcg acctcgtgaa tatgaggacc aacaaccctg tgcttggcgc 780

tcaggcgcaa gtgtgtgtaa tttgtcctcc agatcgcagc aatcgcgccc ctatcttggc 840

ccgcccacct acttatgcag gtattccccg gggtgccatt agtggttttg tgggcaagtg 900

gtttgaccgc agtggttagc ggggttacaa tcagccaagt tattacaccc ttattttaca 960

gtccaaaacc gcagggcggc gtgtgggggc tgacgcgtgc ccccactcca caatttcaaa 1020

aaaaagagtg gccacttgtc tttgtttatg ggccccattg gcgtggagcc ccgtttaatt 1080

ttcgggggtg ttagagacaa ccagtggagt ccgctgctgt cggcgtccac tctctttccc 1140

cttgttacaa atagagtgta acaacatggt tcacctgtct tggtccctgc ctgggacaca 1200

tcttaataac cccagtatca tattgcacta ggattatgtg ttgcccatag ccataaattc 1260

gtgtgagatg gacatccagt ctttacggct tgtccccacc ccatggattt ctattgttaa 1320

agatattcag aatgtttcat tcctacacta gtatttattg cccaaggggt ttgtgagggt 1380

tatattggtg tcatagcaca atgccaccac tgaacccccc gtccaaattt tattctgggg 1440

gcgtcacctg aaaccttgtt ttcgagcacc tcacatacac cttactgttc acaactcagc 1500

agttattcta ttagctaaac gaaggagaat gaagaagcag gcgaagattc aggagagttc 1560

actgcccgct ccttgatctt cagccactgc ccttgtgact aaaatggttc actaccctcg 1620

tggaatcctg accccatgta aataaaaccg tgacagctca tggggtggga gatatcgctg 1680

ttccttagga cccttttact aaccctaatt cgatagcata tgcttcccgt tgggtaacat 1740

atgctattga attagggtta gtctggatag tatatactac tacccgggaa gcatatgcta 1800

cccgtttagg gttaacaagg gggccttata aacactattg ctaatgccct cttgagggtc 1860

cgcttatcgg tagctacaca ggcccctctg attgacgttg gtgtagcctc ccgtagtctt 1920

cctgggcccc tgggaggtac atgtccccca gcatagatct cccgatcccc tatggtcgac 1980

tctcagtaca atctgctctg atgccgcata gttaagccag tatctgctcc ctgcttgtgt 2040

gttggaggtc gctgagtagt gcgcgagcaa aatttaagct acaacaaggc aaggcttgac 2100

cgacaattaa ttgcatgaag aatctgctta gggttaggcg ttttgcgctg cttcgcgatg 2160

tacgggccag atatacgcgt tgacattgat tattgactag ttattaatag taatcaatta 2220

cggggtcatt agttcatagc ccatatatgg agttccgcgt tacataactt acggtaaatg 2280

gcccgcctgg ctgaccgccc aacgaccccc gcccattgac gtcaataatg acgtatgttc 2340

ccatagtaac gccaataggg actttccatt gacgtcaatg ggtggactat ttacggtaaa 2400

ctgcccactt ggcagtacat caagtgtatc atatgccaag tacgccccct attgacgtca 2460

atgacggtaa atggcccgcc tggcattatg cccagtacat gaccttatgg gactttccta 2520

cttggcagta catctacgta ttagtcatcg ctattaccat ggtgatgcgg ttttggcagt 2580

acatcaatgg gcgtggatag cggtttgact cacggggatt tccaagtctc caccccattg 2640

acgtcaatgg gagtttgttt tggcaccaaa atcaacggga ctttccaaaa tgtcgtaaca 2700

actccgcccc attgacgcaa atgggcggta ggcgtgtacg gtgggaggtc tatataagca 2760

gagctctctg gctaactaga gaacccactg cttactggct tatcgaaatt aatacgactc 2820

actataggga gacccaagct agcttggacc tcaccatggg atggagctgt atcatcctct 2880

tcttggtagc aacagctaca gggagacggg cgcgccggat cccgtctcct ggggagccca 2940

gagggcccac aatcaagccc tgtcctccat gcaaatgccc agcacctaac ctcttgggtg 3000

gaccatccgt cttcatcttc cctccaaaga tcaaggatgt actcatgatc tccctgagcc 3060

ccatagtcac atgtgtggtg gtggatgtga gcgaggatga cccagatgtc cagatcagct 3120

ggtttgtgaa caacgtggaa gtacacacag ctcagacaca aacccataga gaggattaca 3180

acagtactct ccgggtggtc agtgccctcc ccatccagca ccaggactgg atgagtggca 3240

aggagttcaa atgcaaggtc aacaacaaag acctcccagc gcccatcgag agaaccatct 3300

caaaacccaa agggtcagta agagctccac aggtatatgt cttgcctcca ccagaagaag 3360

agatgactaa gaaacaggtc actctgacct gcatggtcac agacttcatg cctgaagaca 3420

tttacgtgga gtggaccaac aacgggaaaa cagagctaaa ctacaagaac actgaaccag 3480

tcctggactc tgatggttct tacttcatgt acagcaagct gagagtggaa aagaagaact 3540

gggtggaaag aaatagctac tcctgttcag tggtccacga gggtctgcac aatcaccaca 3600

cgactaagag cttctcccgg actccgggta aatgatgaga attcgatatc aagcttgtcg 3660

ataatcaacc tctggattac aaaatttgtg aaagattgac tggtattctt aactatgttg 3720

ctccttttac gctatgtgga tacgctgctt taatgccttt gtatcatgct attgcttccc 3780

gtatggcttt cattttctcc tccttgtata aatcctggtt gctgtctctt tatgaggagt 3840

tgtggcccgt tgtcaggcaa cgtggcgtgg tgtgcactgt gtttgctgac gcaaccccca 3900

ctggttgggg cattgccacc acctgtcagc tcctttccgg gactttcgct ttccccctcc 3960

ctattgccac ggcggaactc atcgccgcct gccttgcccg ctgctggaca ggggctcggc 4020

tgttgggcac tgacaattcc gtggtgttgt cggggaaatc atcgtccttt ccttggctgc 4080

tcgcctgtgt tgccacctgg attctgcgcg ggacgtcctt ctgctacgtc ccttcggccc 4140

tcaatccagc ggaccttcct tcccgcggcc tgctgccggc tctgcggcct cttccgcgtc 4200

ttcgccttcg ccctcagacg agtcggatct ccctttgggc cgcctccccg catcgatacc 4260

gtcgacctcg agacctagaa aaacatggag caatcacaag tagcaataca gcagctacca 4320

atgctgattg tgcctggcta gaagcacaag aggaggagga ggtgggtttt ccagtcacac 4380

ctcaggtacc tttaagacca atgacttaca aggcagctgt agatcttagc cactttttaa 4440

aagaaaaggg gggactggaa gggctaattc actcccaacg aagacaagat atccttgatc 4500

tgtggatcta ccacacacaa ggctacttcc ctgattggca gaactacaca ccagggccag 4560

ggatcagata tccactgacc tttggatggt gctacaagct agtaccagtt gagcaagaga 4620

aggtagaaga agccaatgaa ggagagaaca cccgcttgtt acaccctgtg agcctgcatg 4680

ggatggatga cccggagaga gaagtattag agtggaggtt tgacagccgc ctagcatttc 4740

atcacatggc ccgagagctg catccggatc tgaggggccc tattctatag tgtcacctaa 4800

atgctagagc tcgctgatca gcctcgactg tgccttctag ttgccagcca tctgttgttt 4860

gcccctcccc cgtgccttcc ttgaccctgg aaggtgccac tcccactgtc ctttcctaat 4920

aaaatgagga aattgcatcg cattgtctga gtaggtgtca ttctattctg gggggtgggg 4980

tggggcagga cagcaagggg gaggattggg aagacaatag caggcatgct ggggatgcgg 5040

tgggctctat ggcttctgag gcggaaagaa ccagctgggg ctctaggggg tatccccacg 5100

cgccctgtag cggcgcatta agcgcggcgg gtgtggtggt tacgcgcagc gtgaccgcta 5160

cacttgccag cgccctagcg cccgctcctt tcgctttctt cccttccttt ctcgccacgt 5220

tcgccggctt tccccgtcaa gctctaaatc ggggcatccc tttagggttc cgatttagtg 5280

ctttacggca cctcgacccc aaaaaacttg attagggtga tggttcacgt agtgggccat 5340

cgccctgata gacggttttt cgccctttga cgttggagtc cacgttcttt aatagtggac 5400

tcttgttcca aactggaaca acactcaacc ctatctcggt ctattctttt gatttataag 5460

ggattttggg gatttcggcc tattggttaa aaaatgagct gatttaacaa aaatttaacg 5520

cgaattaatt ctgtggaatg tgtgtcagtt agggtgtgga aagtccccag gctccccagg 5580

caggcagaag tatgcaaagc atgcatctca attagtcagc aaccaggtgt ggaaagtccc 5640

caggctcccc agcaggcaga agtatgcaaa gcatgcatct caattagtca gcaaccatag 5700

tcccgcccct aactccgccc atcccgcccc taactccgcc cagttccgcc cattctccgc 5760

cccatggctg actaattttt tttatttatg cagaggccga ggccgcctct gcctctgagc 5820

tattccagaa gtagtgagga ggcttttttg gaggcctagg cttttgcaaa aagctcccgg 5880

gagcttgtat atccattttc ggatctgatc aagagacagg atgaggatcg tttcgcatga 5940

ttgaacaaga tggattgcac gcaggttctc cggccgcttg ggtggagagg ctattcggct 6000

atgactgggc acaacagaca atcggctgct ctgatgccgc cgtgttccgg ctgtcagcgc 6060

aggggcgccc ggttcttttt gtcaagaccg acctgtccgg tgccctgaat gaactgcagg 6120

acgaggcagc gcggctatcg tggctggcca cgacgggcgt tccttgcgca gctgtgctcg 6180

acgttgtcac tgaagcggga agggactggc tgctattggg cgaagtgccg gggcaggatc 6240

tcctgtcatc tcaccttgct cctgccgaga aagtatccat catggctgat gcaatgcggc 6300

ggctgcatac gcttgatccg gctacctgcc cattcgacca ccaagcgaaa catcgcatcg 6360

agcgagcacg tactcggatg gaagccggtc ttgtcgatca ggatgatctg gacgaagagc 6420

atcaggggct cgcgccagcc gaactgttcg ccaggctcaa ggcgcgcatg cccgacggcg 6480

aggatctcgt cgtgacccat ggcgatgcct gcttgccgaa tatcatggtg gaaaatggcc 6540

gcttttctgg attcatcgac tgtggccggc tgggtgtggc ggaccgctat caggacatag 6600

cgttggctac ccgtgatatt gctgaagagc ttggcggcga atgggctgac cgcttcctcg 6660

tgctttacgg tatcgccgct cccgattcgc agcgcatcgc cttctatcgc cttcttgacg 6720

agttcttctg agcgggactc tggggttcga aatgaccgac caagcgacgc ccaacctgcc 6780

atcacgagat ttcgattcca ccgccgcctt ctatgaaagg ttgggcttcg gaatcgtttt 6840

ccgggacgcc ggctggatga tcctccagcg cggggatctc atgctggagt tcttcgccca 6900

ccccaacttg tttattgcag cttataatgg ttacaaataa agcaatagca tcacaaattt 6960

cacaaataaa gcattttttt cactgcaaat tctagttgtg gtttgtccaa actcatcaat 7020

gtatcttatc atgtctgtat accgtcgacc tctagctaga gcttggcgta atcatggtca 7080

tagctgtttc ctgtgtgaaa ttgttatccg ctcacaattc cacacaacat acgagccgga 7140

agcataaagt gtaaagcctg gggtgcctaa tgagtgagct aactcacatt aattgcgttg 7200

cgctcactgc ccgctttcca gtcgggaaac ctgtcgtgcc agctgcatta atgaatcggc 7260

caacgcgcgg ggagaggcgg tttgcgtatt gggcgctctt ccgcttcctc gctcactgac 7320

tcgctgcgct cggtcgttcg gctgcggcga gcggtatcag ctcactcaaa ggcggtaata 7380

cggttatcca cagaatcagg ggataacgca ggaaagaaca tgtgagcaaa aggccagcaa 7440

aaggccagga accgtaaaaa ggccgcgttg ctggcgtttt tccataggct ccgcccccct 7500

gacgagcatc acaaaaatcg acgctcaagt cagaggtggc gaaacccgac aggactataa 7560

agataccagg cgtttccccc tggaagctcc ctcgtgcgct ctcctgttcc gaccctgccg 7620

cttaccggat acctgtccgc ctttctccct tcgggaagcg tggcgctttc tcaatgctca 7680

cgctgtaggt atctcagttc ggtgtaggtc gttcgctcca agctgggctg tgtgcacgaa 7740

ccccccgttc agcccgaccg ctgcgcctta tccggtaact atcgtcttga gtccaacccg 7800

gtaagacacg acttatcgcc actggcagca gccactggta acaggattag cagagcgagg 7860

tatgtaggcg gtgctacaga gttcttgaag tggtggccta actacggcta cactagaagg 7920

acagtatttg gtatctgcgc tctgctgaag ccagttacct tcggaaaaag agttggtagc 7980

tcttgatccg gcaaacaaac caccgctggt agcggtggtt tttttgtttg caagcagcag 8040

attacgcgca gaaaaaaagg atctcaagaa gatcctttga tcttttctac ggggtctgac 8100

gctcagtgga acgaaaactc acgttaaggg attttggtca tgagattatc aaaaaggatc 8160

ttcacctaga tccttttaaa ttaaaaatga agttttaaat caatctaaag tatatatgag 8220

taaacttggt ctgacagtta ccaatgctta atcagtgagg cacctatctc agcgatctgt 8280

ctatttcgtt catccatagt tgcctgactc cccgtcgtgt agataactac gatacgggag 8340

ggcttaccat ctggccccag tgctgcaatg ataccgcgag acccacgctc accggctcca 8400

gatttatcag caataaacca gccagccgga agggccgagc gcagaagtgg tcctgcaact 8460

ttatccgcct ccatccagtc tattaattgt tgccgggaag ctagagtaag tagttcgcca 8520

gttaatagtt tgcgcaacgt tgttgccatt gctacaggca tcgtggtgtc acgctcgtcg 8580

tttggtatgg cttcattcag ctccggttcc caacgatcaa ggcgagttac atgatccccc 8640

atgttgtgca aaaaagcggt tagctccttc ggtcctccga tcgttgtcag aagtaagttg 8700

gccgcagtgt tatcactcat ggttatggca gcactgcata attctcttac tgtcatgcca 8760

tccgtaagat gcttttctgt gactggtgag tactcaacca agtcattctg agaatagtgt 8820

atgcggcgac cgagttgctc ttgcccggcg tcaatacggg ataataccgc gccacatagc 8880

agaactttaa aagtgctcat cattggaaaa cgttcttcgg ggcgaaaact ctcaaggatc 8940

ttaccgctgt tgagatccag ttcgatgtaa cccactcgtg cacccaactg atcttcagca 9000

tcttttactt tcaccagcgt ttctgggtga gcaaaaacag gaaggcaaaa tgccgcaaaa 9060

aagggaataa gggcgacacg gaaatgttga atactcatac tcttcctttt tcaatattat 9120

tgaagcattt atcagggtta ttgtctcatg agcggataca tatttgaatg tatttagaaa 9180

aataaacaaa taggggttcc gcgcacattt ccccgaaaag tgccacctga cgtc 9234

<210> 28
<211> 9273
<212> DNA
<213> Artificial Sequence

<220>
<223> pFRIDA-mIg2a-C C-terminal cloning vector

<400> 28
   gacggatcgg gagatccagg aaaaggacaa gcagcgaaaa ttcacgcccc cttgggaggt 60
   ggcggcatat gcaaaggata gcactcccac tctactactg ggtatcatat gctgactgta 120
   tatgcatgag gatagcatat gctacccgga tacagattag gatagcatat actacccaga 180
   tatagattag gatagcatat gctacccaga tatagattag gatagcctat gctacccaga 240
   tataaattag gatagcatat actacccaga tatagattag gatagcatat gctacccaga 300
   tatagattag gatagcctat gctacccaga tatagattag gatagcatat gctacccaga 360
   tatagattag gatagcatat gctatccaga tatttgggta gtatatgcta cccagatata 420
   aattaggata gcatatacta ccctaatctc tattaggata gcatatgcta cccggataca 480
   gattaggata gcatatacta cccagatata gattaggata gcatatgcta cccagatata 540
   gattaggata gcctatgcta cccagatata aattaggata gcatatacta cccagatata 600
   gattaggata gcatatgcta cccagatata gattaggata gcctatgcta cccagatata 660
   gattaggata gcatatgcta tccagatatt tgggtagtat atgctaccca tggcaacatt 720
   agcccaccgt gctctcagcg acctcgtgaa tatgaggacc aacaaccctg tgcttggcgc 780
   tcaggcgcaa gtgtgtgtaa tttgtcctcc agatcgcagc aatcgcgccc ctatcttggc 840
   ccgcccacct acttatgcag gtattccccg gggtgccatt agtggttttg tgggcaagtg 900
   gtttgaccgc agtggttagc ggggttacaa tcagccaagt tattacaccc ttattttaca 960
   gtccaaaacc gcagggcggc gtgtgggggc tgacgcgtgc ccccactcca caatttcaaa 1020
   aaaaagagtg gccacttgtc tttgtttatg ggccccattg gcgtggagcc ccgtttaatt 1080
   ttcgggggtg ttagagacaa ccagtggagt ccgctgctgt cggcgtccac tctctttccc 1140
   cttgttacaa atagagtgta acaacatggt tcacctgtct tggtccctgc ctgggacaca 1200
   tcttaataac cccagtatca tattgcacta ggattatgtg ttgcccatag ccataaattc 1260
   gtgtgagatg gacatccagt ctttacggct tgtccccacc ccatggattt ctattgttaa 1320
   agatattcag aatgtttcat tcctacacta gtatttattg cccaaggggt ttgtgagggt 1380
   tatattggtg tcatagcaca atgccaccac tgaacccccc gtccaaattt tattctgggg 1440
   gcgtcacctg aaaccttgtt ttcgagcacc tcacatacac cttactgttc acaactcagc 1500
   agttattcta ttagctaaac gaaggagaat gaagaagcag gcgaagattc aggagagttc 1560
   actgcccgct ccttgatctt cagccactgc ccttgtgact aaaatggttc actaccctcg 1620
   tggaatcctg accccatgta aataaaaccg tgacagctca tggggtggga gatatcgctg 1680
   ttccttagga cccttttact aaccctaatt cgatagcata tgcttcccgt tgggtaacat 1740
   atgctattga attagggtta gtctggatag tatatactac tacccgggaa gcatatgcta 1800
   cccgtttagg gttaacaagg gggccttata aacactattg ctaatgccct cttgagggtc 1860
   cgcttatcgg tagctacaca ggcccctctg attgacgttg gtgtagcctc ccgtagtctt 1920
   cctgggcccc tgggaggtac atgtccccca gcatagatct cccgatcccc tatggtcgac 1980
   tctcagtaca atctgctctg atgccgcata gttaagccag tatctgctcc ctgcttgtgt 2040
   gttggaggtc gctgagtagt gcgcgagcaa aatttaagct acaacaaggc aaggcttgac 2100
   cgacaattaa ttgcatgaag aatctgctta gggttaggcg ttttgcgctg cttcgcgatg 2160
   tacgggccag atatacgcgt tgacattgat tattgactag ttattaatag taatcaatta 2220
   cggggtcatt agttcatagc ccatatatgg agttccgcgt tacataactt acggtaaatg 2280
   gcccgcctgg ctgaccgccc aacgaccccc gcccattgac gtcaataatg acgtatgttc 2340
   ccatagtaac gccaataggg actttccatt gacgtcaatg ggtggactat ttacggtaaa 2400
   ctgcccactt ggcagtacat caagtgtatc atatgccaag tacgccccct attgacgtca 2460
   atgacggtaa atggcccgcc tggcattatg cccagtacat gaccttatgg gactttccta 2520
   cttggcagta catctacgta ttagtcatcg ctattaccat ggtgatgcgg ttttggcagt 2580
   acatcaatgg gcgtggatag ctgtttgact cacggggatt tccaagtctc caccccattg 2640
   acgtcaatgg gagtttgttt tggcaccaaa atcaacggga ctttccaaaa tgtcgtaaca 2700
   actccgcccc attgacgcaa atgggcggta ggcgtgtacg gtgggaggtc tatataagca 2760
   gagctctctg gctaactaga gaacccactg cttactggct tatcgaaatt aatacgactc 2820
   actataggga gacccaagct agcttggacc tcaccatggg atggagctgt atcatcctct 2880
   tcttggtagc aacagctaca ggtgagccca gagggcccac aatcaagccc tgtcctccat 2940
   gcaaatgccc agcacctaac ctcttgggtg gaccatccgt cttcatcttc cctccaaaga 3000
   tcaaggatgt actcatgatc tccctgagcc ccatagtcac atgtgtggtg gtggatgtga 3060
   gcgaggatga cccagatgtc cagatcagct ggtttgtgaa caacgtggaa gtacacacag 3120
   ctcagacaca aacccataga gaggattaca acagtactct ccgggtggtc agtgccctcc 3180
   ccatccagca ccaggactgg atgagtggca aggagttcaa atgcaaggtc aacaacaaag 3240
   acctcccagc gcccatcgag agaaccatct caaaacccaa agggtcagta agagctccac 3300
   aggtatatgt cttgcctcca ccagaagaag agatgactaa gaaacaggtc actctgacct 3360
   gcatggtcac agacttcatg cctgaagaca tttacgtgga gtggaccaac aacgggaaaa 3420
   cagagctaaa ctacaagaac actgaaccag tcctggactc tgatggttct tacttcatgt 3480
   acagcaagct gagagtggaa aagaagaact gggtggaaag aaatagctac tcctgttcag 3540
   tggtccacga gggtctgcac aatcaccaca cgactaagag cttctcccgg actccgggta 3600
   agacagcgag acgtttaaac cgagggcgct agtgtttaaa cggccgccgt ctccccccac 3660
   tagtaacggc cgccaccgaa ttcgatatca agcttgtcga taatcaacct ctggattaca 3720
   aaatttgtga aagattgact ggtattctta actatgttgc tccttttacg ctatgtggat 3780
   acgctgcttt aatgcctttg tatcatgcta ttgcttcccg tatggctttc attttctcct 3840
   ccttgtataa aacctggttg ctgtctcttt atgaggagtt gtggcccgtt gtcaggcaac 3900
   gtggcgtggt gtgcactgtg tttgctgacg caacccccac tggttggggc attgccacca 3960
   cctgtcagct cctttccggg actttcgctt tccccctccc tattgccacg gcggaactca 4020
   tcgccgcctg ccttgcccgc tgctggacag gggctcggct gttgggcact gacaattccg 4080
   tggtgttgtc ggggaaatca tcgtcctttc cttggctgct cgcctgtgtt gccacctgga 4140
   ttctgcgcgg gacgtccttc tgctacgtcc cttcggccct caatccagcg gaccttcctt 4200
   cccgcggcct gctgccggct ctgcggcctc ttccgcgtct tcgccttcgc cctcagacga 4260
   gtcggatctc cctttgggcc gcctccccgc atcgataccg tcgacctcga gacctagaaa 4320
   aacatggagc aatcacaagt agcaatacag cagctaccaa tgctgattgt gcctggctag 4380
   aagcacaaga ggaggaggag gtgggttttc cagtcacacc tcaggtacct ttaagaccaa 4440
   tgacttacaa ggcagctgta gatcttagcc actttttaaa agaaaagggg ggactggaag 4500
   ggctaattca ctcccaacga agacaagata tccttgatct gtggatctac cacacacaag 4560
   gctacttccc tgattggcag aactacacac cagggccagg gatcagatat ccactgacct 4620
   ttggatggtg ctacaagcta gtaccagttg agcaagagaa ggtagaagaa gccaatgaag 4680
   gagagaacac ccgcttgtta caccctgtga gcctgcatgg gatggatgac ccggagagag 4740
   aagtattaga gtggaggttt gacagccgcc tagcatttca tcacatggcc cgagagctgc 4800
   atccggatct gaggggccct attctatagt gtcacctaaa tgctagagct cgctgatcag 4860
   cctcgactgt gccttctagt tgccagccat ctgttgtttg cccctccccc gtgccttcct 4920
   tgaccctgga aggtgccact cccactgtcc tttcctaata aaatgaggaa attgcatcgc 4980
   attgtctgag taggtgtcat tctattctgg ggggtggggt ggggcaggac agcaaggggg 5040
   aggattggga agacaatagc aggcatgctg gggatgcggt gggctctatg gcttctgagg 5100
   cggaaagaac cagctggggc tctagggggt atccccacgc gccctgtagc ggcgcattaa 5160
   gcgcggcggg tgtggtggtt acgcgcagcg tgaccgctac acttgccagc gccctagcgc 5220
   ccgctccttt cgctttcttc ccttcctttc tcgccacgtt cgccggcttt ccccgtcaag 5280
   ctctaaatcg gggcatccct ttagggttcc gatttagtgc tttacggcac ctcgacccca 5340
   aaaaacttga ttagggtgat ggttcacgta gtgggccatc gccctgatag acggtttttc 5400
   gccctttgac gttggagtcc acgttcttta atagtggact cttgttccaa actggaacaa 5460
   cactcaaccc tatctcggtc tattcttttg atttataagg gattttgggg atttcggcct 5520
   attggttaaa aaatgagctg atttaacaaa aatttaacgc gaattaattc tgtggaatgt 5580
   gtgtcagtta gggtgtggaa agtccccagg ctccccaggc aggcagaagt atgcaaagca 5640
   tgcatctcaa ttagtcagca accaggtgtg gaaagtcccc aggctcccca gcaggcagaa 5700
   gtatgcaaag catgcatctc aattagtcag caaccatagt cccgccccta actccgccca 5760
   tcccgcccct aactccgccc agttccgccc attctccgcc ccatggctga ctaatttttt 5820
   ttatttatgc agaggccgag gccgcctctg cctctgagct attccagaag tagtgaggag 5880
   gcttttttgg aggcctaggc ttttgcaaaa agctcccggg agcttgtata tccattttcg 5940
   gatctgatca agagacagga tgaggatcgt ttcgcatgat tgaacaagat ggattgcacg 6000
   caggttctcc ggccgcttgg gtggagaggc tattcggcta tgactgggca caacagacaa 6060
   tcggctgctc tgatgccgcc gtgttccggc tgtcagcgca ggggcgcccg gttctttttg 6120
   tcaagaccga cctgtccggt gccctgaatg aactgcagga cgaggcagcg cggctatcgt 6180
   ggctggccac gacgggcgtt ccttgcgcag ctgtgctcga cgttgtcact gaagcgggaa 6240
   gggactggct gctattgggc gaagtgccgg ggcaggatct cctgtcatct caccttgctc 6300
   ctgccgagaa agtatccatc atggctgatg caatgcggcg gctgcatacg cttgatccgg 6360
   ctacctgccc attcgaccac caagcgaaac atcgcatcga gcgagcacgt actcggatgg 6420
   aagccggtct tgtcgatcag gatgatctgg acgaagagca tcaggggctc gcgccagccg 6480
   aactgttcgc caggctcaag gcgcgcatgc ccgacggcga ggatctcgtc gtgacccatg 6540
   gcgatgcctg cttgccgaat atcatggtgg aaaatggccg cttttctgga ttcatcgact 6600
   gtggccggct gggtgtggcg gaccgctatc aggacatagc gttggctacc cgtgatattg 6660
   ctgaagagct tggcggcgaa tgggctgacc gcttcctcgt gctttacggt atcgccgctc 6720
   ccgattcgca gcgcatcgcc ttctatcgcc ttcttgacga gttcttctga gcgggactct 6780
   ggggttcgaa atgaccgacc aagcgacgcc caacctgcca tcacgagatt tcgattccac 6840
   cgccgccttc tatgaaaggt tgggcttcgg aatcgttttc cgggacgccg gctggatgat 6900
   cctccagcgc ggggatctca tgctggagtt cttcgcccac cccaacttgt ttattgcagc 6960
   ttataatggt tacaaataaa gcaatagcat cacaaatttc acaaataaag catttttttc 7020
   actgcaattc tagttgtggt ttgtccaaac tcatcaatgt atcttatcat gtctgtatac 7080
   cgtcgacctc tagctagagc ttggcgtaat catggtcata gctgtttcct gtgtgaaatt 7140
   gttatccgct cacaattcca cacaacatac gagccggaag cataaagtgt aaagcctggg 7200
   gtgcctaatg agtgagctaa ctcacattaa ttgcgttgcg ctcactgccc gctttccagt 7260
   cgggaaacct gtcgtgccag ctgcattaat gaatcggcca acgcgcgggg agaggcggtt 7320
   tgcgtattgg gcgctcttcc gcttcctcgc tcactgactc gctgcgctcg gtcgttcggc 7380
   tgcggcgagc ggtatcagct cactcaaagg cggtaatacg gttatccaca gaatcagggg 7440
   ataacgcagg aaagaacatg tgagcaaaag gccagcaaaa ggccaggaac cgtaaaaagg 7500
   ccgcgttgct ggcgtttttc cataggctcc gcccccctga cgagcatcac aaaaatcgac 7560
   gctcaagtca gaggtggcga aacccgacag gactataaag ataccaggcg tttccccctg 7620
   gaagctccct cgtgcgctct cctgttccga ccctgccgct taccggatac ctgtccgcct 7680
   ttctcccttc gggaagcgtg gcgctttctc aatgctcacg ctgtaggtat ctcagttcgg 7740
   tgtaggtcgt tcgctccaag ctgggctgtg tgcacgaacc ccccgttcag cccgaccgct 7800
   gcgccttatc cggtaactat cgtcttgagt ccaacccggt aagacacgac ttatcgccac 7860
   tggcagcagc cactggtaac aggattagca gagcgaggta tgtaggcggt gctacagagt 7920
   tcttgaagtg gtggcctaac tacggctaca ctagaaggac agtatttggt atctgcgctc 7980
   tgctgaagcc agttaccttc ggaaaaagag ttggtagctc ttgatccggc aaacaaacca 8040
   ccgctggtag cggtggtttt tttgtttgca agcagcagat tacgcgcaga aaaaaaggat 8100
   ctcaagaaga tcctttgatc ttttctacgg ggtctgacgc tcagtggaac gaaaactcac 8160
   cgttaaggga ttttggtcat gagattatca aaaaggatct tcacctagat ccttttaaat 8220
   taaaaatgaa gttttaaatc aatctaaagt atatatgagt aaacttggtc tgacagttac 8280
   caatgcttaa tcagtgaggc acctatctca gcgatctgtc tatttcgttc atccatagtt 8340
   gcctgactcc ccgtcgtgta gataactacg atacgggagg gcttaccatc tggccccagt 8400
   gctgcaatga taccgcgaga cccacgctca ccggctccag atttatcagc aataaaccag 8460
   ccagccggaa gggccgagcg cagaagtggt cctgcaactt tatccgcctc catccagtct 8520
   attaattgtt gccgggaagc tagagtaagt agttcgccag ttaatagttt gcgcaacgtt 8580
   gttgccattg ctacaggcat cgtggtgtca cgctcgtcgt ttggtatggc ttcattcagc 8640
   tccggttccc aacgatcaag gcgagttaca tgatccccca tgttgtgcaa aaaagcggtt 8700
   agctccttcg gtcctccgat cgttgtcaga agtaagttgg ccgcagtgtt atcactcatg 8760
   gttatggcag cactgcataa ttctcttact gtcatgccat ccgtaagatg cttttctgtg 8820
   actggtgagt actcaaccaa gtcattctga gaatagtgta tgcggcgacc gagttgctct 8880
   tgcccggcgt caatacggga taataccgcg ccacatagca gaactttaaa agtgctcatc 8940
   attggaaaac gttcttcggg gcgaaaactc tcaaggatct taccgctgtt gagatccagt 9000
   tcgatgtaac ccactcgtgc acccaactga tcttcagcat cttttacttt caccagcgtt 9060
   tctgggtgag caaaaacagg aaggcaaaat gccgcaaaaa agggaataag ggcgacacgg 9120
   aaatgttgaa tactcatact cttccttttt caatattatt gaagcattta tcagggttat 9180
   tgtctcatga gcggatacat atttgaatgt atttagaaaa ataaacaaat aggggttccg 9240
   cgcacatttc cccgaaaagt gccacctgac gtc 9273